# EUROPEAN PATENT APPLICATION

(11) **EP 3 842 802 A1**
(43) Date of publication of application: **30.06.2021**
(21) Application number: 20214482.0
(22) Date of filing: 16.12.2020
(51) Int. Cl.: G01N 33/564

(54) **METHOD FOR THE DETECTION OF AN AUTOANTIBODY TO ACETYLCHOLINE RECEPTOR**

(30) Priority: 20.12.2019 EP 19000592
(71) Applicant: Euroimmun Medizinische Labordiagnostika AG, 23560 Lübeck (DE)
(72) Inventor: Dettmann, Inga-Madeleine, 23623 Ahrensbök (DE); Stegelmann, Christin, 23684 Gronenberg (DE); Probst, Christian, 23909 Ratzeburg (DE); Radzimski, Christiane, 23858 Reinfeld (DE); Mindorf, Swantje, 23911 Ziethen (DE)
(74) Representative: Richter, Carsten

(57) **Abstract**

The present invention relates to a diagnostically useful carrier comprising a eukaryotic cell overexpressing an adult ACh receptor or a variant thereof and a eukaryotic cell overexpressing a fetal ACh receptor or a variant thereof, wherein the cell is fixed for immunofluorescence and a method comprising the step of contacting said carrier with a patient sample and a use of a use of the combination of a eukaryotic cell overexpressing an adult ACh receptor or a variant thereof and a eukaryotic cell overexpressing a fetal ACh receptor or a variant thereof for increasing the sensitivity of an assay, preferably immunofluorescence assay, for the detection of an autoantibody to ACh receptor.

## Description

The present invention relates to a diagnostically useful carrier comprising a eukaryotic cell overexpressing an adult ACh receptor or a variant thereof and a eukaryotic cell overexpressing a fetal ACh receptor or a variant thereof, wherein the cell is fixed for immunofluorescence and a method comprising the step of contacting said carrier with a patient sample and a use of the combination of a eukaryotic cell overexpressing an adult ACh receptor or a variant thereof and a eukaryotic cell overexpressing a fetal ACh receptor or a variant thereof for increasing the sensitivity of an assay, preferably immunofluorescence assay, for the detection of an autoantibody to ACh receptor.

In myasthenia gravis (MG), autoantibodies against the acetylcholine (ACh) receptor (AChR) of the motor end plates cause a dysfunction of neuromuscular data transmission. This leads to weakness of vital muscles, which may be fatal.

MG is divided into different forms. These encompass acquired forms of MG, either generalized MG or MG with exclusively ocular syndromes and genetic forms. The prevalence of MG is estimated to be 40 to 125 cases per 1,000,000 people in Europe and 50 to 200 in America (USA). MG can occur from early childhood to old age. Women, particularly young women of 20 to 30 years of age, and men older than 50 years of age are more likely than others to be afflicted with the disease.

If diagnosed at an early stage, life-saving treatment is possible. Therefore, the earlier an accurate diagnosis, the higher the chance that the patient can be successfully treated.

Various serological tests are available for MG diagnostics, in particular for detecting autoantibodies against AChR, for example based on ELISA, radioimmunoassay (RIA) and immunofluorescence.

While the RIA assay is the most sensitive, the use of radioactive isotopes prevents many laboratories from implementing it. Others hope that RIA tests can be replaced by sufficiently sensitive non-radioactive assays for safety reasons.

Immunofluorescence-based assays are a convenient and generally sensitive method. It has been hoped that such assays could replace the RIA, but previous studies have shown that the sensitivity of such assays leaves room for improvement.

For example George *et al.* found that autoantibodies could not be detected in one out of 13 patients (George, S., Noack, M., Vanek, M., Rentzsch, J., Röber, N., Conrad, K., Roggenbuck, D., and Küpper, J. H. (2015) Expression of nicotinic acetylcholine receptor subunits in HEp-2 cells for immunodetection of autoantibodies specificities in sera from Myasthenia gravis patients, Clin. Hamorheology and Microcirculation DOI 10.3233/CH-15199). Live cells expressing either adult or fetal ACh receptor were used for the assay and fixed after the incubation with the samples. Typical fluorescence patterns indicating the presence of an autoantibody to ACh receptor are also disclosed.

Yang *et al.,* Shi *et al.* and Leite *et al.* used an acetylcholine receptor labeled with EGFP, a 30 kD fluorescent protein, which was fused to rapsyn, a component of the complex used. ACh receptor autoantibodies were detected by detecting whether this EGFP and a fluorescent label binding to an ACh receptor autoantibody emitted colocalized fluorescence signals. (Yang, L., Maxwell, S., Leite, M. I., Waters, P., Clover, L., Fan, X., Zhang, D., Yang, C., Beeson, D., and Vincent, A. (2011) J. Neurol. Sc. 301, 71-76; Shi, Q. G., Wang, H. H., Ma, X. W., Zhang, D. Q., Yang, C. S., Shi, F. D., Yang, L. (2012) Clinical significance of detection of antibodies to fetal and adult acetylcholine receptors in myasthenia gravis, Neurosc. Bull. 28(5), 469-474; Leite, M. I, Jacob, S., Viegas, S., Cossins, J., Clover, L., Morgan, B. P., Beeson, D., Willcox, N., Vincent, A. (2008) IgGI antibodies to acetylcholine receptors in seronegative myasthenia gravis (2008) Brain 131, 1940-1952). They used live cells fixed after incubation with a patient sample.

The problem underlying the present invention was to provide an immunofluorescence assay based on the simple detection of a fluorescent label with an increased sensitivity with the potential to replace the RIA. The assay should be in a format that allows the manufacturer to ship reagents that are ready for incubation with the samples and detection reagents, without the need for fixing directly prior to the incubation, a step that may introduce deviations that make it impossible to standardize and compare results from various laboratories.

Another problem underlying the present invention is to increase the diagnostic reliability, in particular sensitivity and/or specificity, of immunofluorescence tests.

The problem underlying the present invention is solved by the subject matter of the attached independent and dependent claims.

In a first aspect, the problem is solved by a diagnostically useful carrier comprising a eukaryotic cell overexpressing an adult ACh receptor or a variant thereof and a eukaryotic cell overexpressing a fetal ACh receptor or a variant thereof, wherein the cell is fixed for immunofluorescence.

In a preferred embodiment, any eukaryotic cell overexpressing an autoantigen, preferably from the group comprising an adult ACh receptor, a fetal ACh receptor and Musk, is pre-fixed.

In a preferred embodiment, the carrier further comprises a eukaryotic cell overexpressing Musk or a variant thereof.

In a preferred embodiment, the cell overexpressing an adult ACh receptor or a variant thereof and the cell overexpressing a fetal ACh receptor or a variant thereof permanently express adult and fetal ACh receptor, respectively.

In a preferred embodiment, the carrier further comprises a control, preferably a mock-transfected cell.

In a preferred embodiment, the cell is selected from the group comprising HEK293, HeLa, CHO and Jurkat cells and derivatives thereof.

In a preferred embodiment of the carrier, at least one eukaryotic cell, preferably all eukaryotic cells overexpressing an autoantigen from the group comprising adult ACh receptor, fetal ACh receptor and Musk or a variant thereof comprises or comprise one or more vectors, wherein the nucleic acid encoding the autoantigen is under the control of an promotor inducible by addition of an inducer, and wherein for each cell an additional, spatially separate eukaryotic cell is present which comprises the same vector lacking said nucleic acid or comprises the same vector, but does not express the autoantigen, preferably because of the absence of the inducer.

In a second aspect, the problem is solved by a method, preferably for the diagnosis of myasthenia gravis, LEMS and OMD, comprising the step of detecting in a sample an autoantibody to ACh receptor, optionally in addition to detecting an autoantibody to Musk, comprising the steps:
a) contacting the sample with the carrier according to the present invention under conditions allowing formation of a complex comprising an autoantibody from the group comprising an autoantibody to ACh receptor, preferably adult and/or fetal ACh receptor, and Musk and an autoantigen from the group comprising ACh receptor and Musk,
b) contacting any complex formed in step b) with a fluorescent ligand to the autoantibody,
c) removing any fluorescent ligand not bound to an autoantibody, and
d) detecting the presence or absence of the fluorescent ligand.

In a third aspect, the problem is solved by a kit comprising the carrier according to the present invention and a ligand to an autoantibody to ACh receptor, optionally also to Musk, wherein the ligand is a fluorescent ligand, wherein any eukaryotic cell overexpressing an autoantigen is preferably pre-fixed.

In a fourth aspect, the problem is solved by a use of the combination of a eukaryotic cell overexpressing an adult ACh receptor or a variant thereof and a eukaryotic cell overexpressing a fetal ACh receptor or a variant thereof for increasing the sensitivity of an assay, preferably immunofluorescence assay, for the detection of an autoantibody to ACh receptor.

In a preferred embodiment, the presence or absence of a fluorescent ligand binding to the autoantibody indicates whether or not the autoantibody is present in the sample.

In a preferred embodiment, the use is for diagnosing an autoimmune disease, preferably myasthenia gravis, Lambert-Eaton-Myasthenia syndrome (LEMS) and other muscular disorders OMD.

In a preferred embodiment, the eukaryotic cell expressing an adult ACh receptor or a variant thereof and the eukaryotic cell overexpressing a fetal ACh receptor or a variant thereof and optionally the eukaryotic cell expressing Musk or a variant thereof are spatially separated.

In a preferred embodiment, the eukaryotic cell overexpressing an adult ACh receptor or a variant thereof and a eukaryotic cell overexpressing a fetal ACh receptor or a variant thereof and optionally the eukaryotic cell expressing Musk or a variant thereof are colocalized.

In a preferred embodiment, the sample comprises antibodies from a patient, preferably blood, more preferably selected from the group comprising serum, liquor, plasma and whole blood. In a more preferred embodiment, the patient is a human patient.

The present invention is based on the inventors' surprising finding that a combination of adult and fetal AChR on the same carrier may be used to increase the sensitivity. Cells may be pre-fixed in a mass production process prior to shipping the product and incubating the carrier according to the present invention with a patient sample.

In a preferred embodiment, an ACh receptor comprises two alpha subunits and one beta and one delta subunit. In addition, the fetal ACh receptor contains the gamma subunit, whereas the adult ACh receptor contains the epsilon subunit. The complex assembles spontaneously if all the subunits required are present in a cell or *in vitro.* In a preferred embodiment, the alpha subunit is represented by SEQ ID NO1 or a variant thereof. In a preferred embodiment, the beta subunit is represented by SEQ ID NO2 or a variant thereof. In a preferred embodiment, the delta subunit is represented by SEQ ID NO3 or a variant thereof. In a preferred embodiment, the epsilon subunit is represented by SEQ ID NO4 or a variant thereof. In a preferred embodiment, the gamma subunit is represented by SEQ ID NO5 or a variant thereof.

In a preferred embodiment, the adult ACh receptor or a variant thereof and/or the fetal ACh receptor or a variant thereof are associated with rapsyn or a variant thereof. Rapsyn has been reported to aid in the assembly of ACh receptor subunits used for immunodiagnostics (Leite, M. I, Jacob, S., Viegas, S., Cossins, J., Clover, L., Morgan, B. P., Beeson, D., Willcox, N., Vincent, A. (2008) IgGI antibodies to acetylcholine receptors in seronegative myasthenia gravis (2008) Brain 131, 1940-1952). In a preferred embodiment, rapsyn is represented by SEQ ID NO6 or a variant thereof.

In a preferred embodiment, the carrier may comprise a eukaryotic cell overexpressing Musk. In a preferred embodiment, Musk is represented by SEQ ID NO 13 or a variant thereof.

In a preferred embodiment, the diagnostically useful carrier is intended for microscopic immunofluorescence analyses. Such a carrier may be a glass slide. The cell on the glass slide may be covered with a glycerol-containing mounting buffer, and a cover glass may be placed on top. Slides with cover glasses (for example FD 1435-92, FD 1435-93, FD 1435-94 and FD 1434-90) are available from EUROIMMUN Medizinische Labordiagnostika, AG. However, any carrier compatible with microscopic analysis of the fluorescence pattern may be used. The carrier may comprise a mock-transfected cell, which has been transfected with the same vector as the cell overexpressing the autoantigen of interest, but without the nucleic acid encoding for the latter. Such mock-transfected cell may serve as a negative control. The carrier is configured for analysis using an immunofluorescence microscope.

In a preferred embodiment, a fixed eukaryotic, preferably mammalian, more preferably immortalized human cell is used. In a preferred embodiment, the term "fixed" cell, as used herein, refers to a cell that has been treated with a reactive chemical compound to the effect that the cell is no longer metabolically active, but still presents its epitopes for immunostaining with antibodies and their subsequent detection, for example by fluorescence. More preferably, the reactive chemical compound is selected from the group comprising acetone, formalin, methanol and ethanol or mixtures thereof, preferably all of them. The person skilled in the art is familiar with protocols that may be used to prepare fixed cells. Essentially, the cell which is attached to a solid support is washed by using washing buffer, followed by contacting with the reactive compound, for example immersion. Formalin or pure acetone or aqueous dilutions of the reactive chemical compound may be used.

In a preferred embodiment, the cell according to the present invention overexpresses adult ACh receptor or a variant thereof and fetal ACh receptor or a variant thereof, optionally further autoantigens such as Musk or a variant thereof. In a preferred embodiment, the term "overexpressing", as used herein, means that the cell has been transfected with a nucleic acid, either transiently or stably, the latter in the sense that the nucleic acid has been incorporated in the genome of the cell, that comprises a nucleic acid sequence encoding a polypeptide comprising the human ACh receptor or Musk or a variant thereof under the control of a promotor, preferably a strong promotor. Consequently, the transfected cell expresses more polypeptide recognized by the autoantibody than the non-transfected, same type of cell normally would, probably at least 10, 20, 30, 50, 100, 200 or 500 % more as judged by quantitative Western Blot. Preferably the non-transfected cell expresses no detectable amount of the autoantigen.

Examples of such vectors include SEQ ID NO7, SEQ ID NO8, SEQID NO9, SEQ ID NO10, SEQ ID NO11, SEQ ID NO12 and SEQ ID NO14.

In a preferred embodiment, the cell comprises one or more vectors comprising a nucleic acid encoding for the ACh receptor subunits under the functional control of a promotor.

In a preferred embodiment, the cell comprises the vectors represented by SEQ ID NO7 (ACh receptor alpha subunit), SEQ ID NO8 (ACh receptor beta subunit), SEQ ID NO9 (ACh receptor delta subunit) and one or both of SEQ ID NO10 (ACh receptor epsilon subunit) and SEQ ID NO11 (ACh receptor gamma subunit). In addition the cell may comprises the vector represented by SEQ ID NO12 (rapsyn) and/or the vector represented by SEQ ID NO14 (Musk).

The promotor may be an inducible promotor, which allows for the induction of expression by addition of an inducer. The person skilled in the art is familiar with protocols and vectors for transiently overexpressing a polypeptide in a eukaryotic cell, for example the pTriEx system from Novagen and with protocols and vectors for stably transfecting a eukaryotic cell, for example the pcDNA™4/TO vector system from Invitrogen.

In a preferred embodiment, the carrier may comprise a panel comprising the cell according to the invention. In addition the carrier may comprise additional panels. The panels are preferably surrounded by a hydrophobic surface.

In a preferred embodiment, the cell is bound to one or more autoantibodies each binding to an autoantigen from the group comprising fetal or adult ACh receptor or Musk, and a secondary antibody is bound to the antibody. In a more preferred embodiment, the secondary antibody recognizes IgA, IgG and IgM class antibodies or all of them, preferably class IgG antibodies. A fragment or other variant of a secondary antibody or an aptamer or other molecule with binding properties that can be engineered such that the molecule binds specifically and/or recognizes the autoantibody or IgA, IgG or IgM class antibodies may be used. For immunofluorescence analysis, the secondary antibody may comprise a detectable fluorescent ligand. In a preferred embodiment, the fluorescent ligand is a fluorescent molecule suitable for immunofluorescence staining of a eukaryotic cell, preferably selected from the group comprising fluorescein and derivatives thereof, preferably the isothiocyanate mixed isomer (FITC), tetramethylrhodamine isothiocyanate (TRITC) and derivatives thereof, rhodamine and derivatives thereof, 4,6'-Diamidino-2-phenylindole dihydrochloride (DAPI) and derivatives thereof, 2'-(4-Ethoxyphenyl)-5-(4-methyl-1-piperazinyl)-2,5'-bi-1H-benzimidazole trihydrochloride trihydrat and derivatives thereof (Hoechst 33342), and is more preferably fluorescein or a derivative thereof. Suitable fluorescent molecules are described in Martynov, V. I., Pakhomov, A. A., Popva, N. V., Dexev, I. E. and Petrenko, A. G. (2016) Synthetic Fluorophores for Visualizing Biomolecules in Living Systems, Acta Naturae, 4 (31), 33 and in Thermo Scientific Pierce fluorescent Products Guide, Thermo Scientific, 2012.

The cell according to the invention is preferably spatially separated from any of the mock-transfected cell or a cell expressing another autoantigen such that a fluorescence signal can be assigned to a specific autoantigen, for example the fetal or adult ACh receptor or Musk.

In a preferred embodiment, the adult and/or fetal ACh receptor is non-fluorescent in the sense that it is unable to emit a fluorescence signal that would be detectable in a normal fluorescence microscope used for diagnostic immunofluorescence. In other words, the ACh receptor is undetectable by immunofluorescence analysis.

In a preferred embodiment, two or more, preferably all cells on the diagnostically useful carrier, including both one or more cells overexpressing an autoantigen, preferably from the group comprising adult ACh receptor, fetal ACh receptor and Musk, as well as any cell serving as a control, are spatially separate on the carrier but close enough such that all cells can be incubated with the same sample in a one-pot reaction, ideally with a single drop of sample. For example, the Mosaik system by EUROIMMUN Medizinische Labordiagnostika AG is based on miniature panels arranged such that a number of panels, for example four or six panels, are contacted by the same drop of liquid sample.

In another preferred embodiment, a cell overexpressing adult ACh receptor and a cell overexpressing fetal ACh receptor, optionally a cell overexpressing Musk, which cells are colocalized, are used. This may be a mixture of a cell expressing an adult ACh receptor and a cell expressing a fetal ACh receptor or it may be a cell expressing both adult and fetal ACh receptor and optionally Musk.

The teachings of the present invention may not only be carried out using the polypeptides, in particular a polypeptide comprising the native sequence of a polypeptide such as the fetal or adult ACh receptor or nucleic acids having the exact sequences referred to in this application explicitly, for example by function, name, sequence or accession number, or implicitly, but also using variants of such polypeptides or nucleic acids.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 6, 7, 8, 10, 12, 15, 20, 25, 50, 75, 100, 150, 200, 250, 300, 350 or 400 successive amino acids of the original sequence or a variant thereof. The total length of the variant may be at least 6, 7, 8, 9, 10, 11, 12, 20, 25, 30, 40, 50, 60, 70, 80, 90, 100, 200, 300, 400, 500, 750, 1000 or more amino acids.

The term "variant" relates not only to at least one fragment, but also to a polypeptide or a fragment thereof comprising amino acid sequences that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability of an antigen to bind to an (auto)antibody, or the fold or structure of the polypeptide are deleted or substituted and/or one or more such essential amino acids are replaced in a conservative manner and/or amino acids are added such that the biological activity of the polypeptide is preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default settings.

In a preferred embodiment, the polypeptide and variants thereof may, in addition, comprise chemical modifications, for example isotopic labels or covalent modifications such as glycosylation, phosphorylation, acetylation, decarboxylation, citrullination, methylation, hydroxylation and the like. The person skilled in the art is familiar with methods to modify polypeptides. Any modification is designed such that it does not abolish the biological activity of the variant.

Moreover, variants may also be generated by N- or/and C-terminal fusion of polypeptides, fragments or variants thereof with other known polypeptides or variants thereof and comprise active portions or domains, preferably having a sequence identity of at least 70, 75, 80, 85, 90, 92, 94, 95, 96, 97, 98 or 99 % when aligned with the active portion of the reference sequence, wherein the term "active portion", as used herein, refers to an amino acid sequence, which is less than the full length amino acid sequence or, in the case of a nucleic acid sequence, codes for less than the full length amino acid sequence, respectively, and/or is a variant of the natural sequence, but retains at least some of the biological activity. Any fusion parts are chosen such that they do not interfere with the binding reaction between autoantigen and autoantibody to be detected and do not bind to other autoantibodies and do not give a false positive or any other unwanted signal.

In a preferred embodiment, the term "variant" of a nucleic acid comprises nucleic acids the complementary strand of which hybridizes, preferably under stringent conditions, to the reference or wild type nucleic acid. Stringency of hybridization reactions is readily determinable by one of ordinary skilled in the art, and in general is an empirical calculation dependent on probe length, washing temperature and salt concentration. In general longer probes require higher temperatures for proper annealing, while shorter probes less so. Hybridization generally depends on the ability of denatured DNA to reanneal to complementary strands present in an environment below their melting temperature: The higher the degree of desired homology between the probe and hybridizable sequence, the higher the relative temperature which may be used. As a result, higher relative temperatures would tend to make the reaction conditions more stringent, while lower temperature less so. For additional details and explanation of stringency of hybridization reactions, see Ausubel, F. M. (1995), Current Protocols in Molecular Biology. John Wiley & Sons, Inc. Moreover, the person skilled in the art may follow the instructions given in the manual Boehringer Mannheim GmbH (1993) The DIG System Users Guide for Filter Hybridization, Boehringer Mannheim GmbH, Mannheim, Germany and in Liebl, W., Ehrmann, M., Ludwig, W., and Schleifer, K. H. (1991) International Journal of Systematic Bacteriology 41: 255-260 on how to identify DNA sequences by means of hybridization. In a preferred embodiment, stringent conditions are applied for any hybridization, i.e. hybridization occurs only if the probe is 70 % or more identical to the target sequence. Probes having a lower degree of identity with respect to the target sequence may hybridize, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C - 68°C, approximately 56°C - 68°C, approximately 58°C - 68°C, approximately 60°C - 68°C, approximately 62°C - 68°C, approximately 64°C - 68°C, approximately 66°C - 68°C. In a particularly preferred embodiment, the temperature is approximately 64°C - 68°C or approximately 66°C - 68°C. It is possible to adjust the concentration of salt to 0.2 x SSC or even 0.1 x SSC. Nucleic acid sequences having a degree of identity with respect to the reference or wild type sequence of at least 70, 80, 90, 91, 92, 93, 94, 95, 96, 97, 98, 99 % may be isolated. In a preferred embodiment, the term variant of a nucleic acid sequence, as used herein, refers to any nucleic acid sequence that encodes the same amino acid sequence and variants thereof as the reference nucleic acid sequence, in line with the degeneracy of the genetic code.

The variant of the polypeptide has biological activity. In a preferred embodiment, such biological activity is the ability to bind specifically to an autoantibody binding to a mammalian ACh receptor, preferably human ACh receptor, as found in a patient suffering from an autoimmune disease associated with such autoantibody, preferably myasthenia gravis, LEMS and OMG, more preferably myasthenia gravis. For example, whether or not a variant of the polypeptide has such biological activity may be checked by determining whether or not the variant of interest binds to an autoantibody from a sample of a patient which autoantibody binds to wild type human ACh receptor, preferably as determined by indirect immunofluorescence as described in the experimental section of this application.

Various publications may aid the person skilled in the art in designing immunoreactive variants and in recognizing which variation will not abolish detection by the autoantibody. B cell epitopes have been identified on all five AChR subunits of fetal and adult forms, and the main immunogenic region is located on the acetylcholine-binding α1 subunit (Tzartos, S. J., Barkas, T., Cung, M.T., Mamalaki, A., Marraud, M., Orlewski, P., Papanastasiou, D., Sakarellos, C., Sakarellos-Daitsiotis, M., Tsantili, P., Tsikaris ,V. Anatomy of the antigenic structure of a large membrane autoantigen, the muscle-type nicotinic acetylcholine receptor. Immunol Rev. 1998;163:89-120). Early studies mapping the binding sites of anti-AChR antibodies looked at binding between synthetic peptides and rat monoclonal antibodies which recognized AChR from various species, including humans. The main immunogenic region of AChR was localized to aa67-76 on the extracellular loop of the α1 subunit using a solid-phase radioimmunoassay where 14-20mers were incubated with the different monoclonal antibodies (Tzartos S.J., Kokla A., Walgrave S.L., Conti-Tronconi, B.M. Localization of the main immunogenic region of human muscle acetylcholine receptor to residues 67-76 of the alpha subunit. Proc Natl Acad Sci USA. 1988;85:2899-2903). The importance of the α1 subunit was then confirmed by competition binding radioimmunoassay examining the adsorption of these monoclonal antibodies by the chosen peptide. Results of a later study indicated that interaction of the main immunogenic loop with aa1-14 on the N-terminal α-helix produces a conformational epitope on AChR that significantly augments the degree of antibody binding (Luo J, Taylor P, Losen M, de Baets MH, Shelton GD, Lindstrom J. Main immunogenic region structure promotes binding of conformation-dependent myasthenia gravis autoantibodies, nicotinic acetylcholine receptor conformation maturation, and agonist sensitivity. J Neurosci. 2009;29:13898-13908). The amino acids at positions 68 and 71 appeared to be crucial contact sites, as substitutions of these residues with L-alanine nearly abolished monoclonal antibody binding (Papadouli I, Potamianos S, Hadjidakis I, Bairaktari E, Tsikaris V, Sakarellos C, Cung MT, Marraud M, Tzartos SJ. Antigenic role of single residues within the main immunogenic region of the nicotinic acetylcholine receptor. Biochem J. 1990;269:239-245; Wahlsten JL, Lindstrom JM, Ostlie N, Wu XD, Conti-Tronconi BM. Myasthenia gravis: effect on antibody binding of conservative substitutions of amino acid residues forming the main immunogenic region of the nicotinic acetylcholine receptor. J Recept Res. 1993). Autoantibodies against intracellular portions of AChRs have also been detected in MG patients, targeting a variety of subunits. Cytoplasmic epitopes reported thus far include aa373-380 of the α1 subunit and aa354-359 of the β1 subunit (Tzartos, S.J., Remoundos, M. Detection of antibodies directed against the cytoplasmic region of the human acetylcholine receptor in sera from myasthenia gravis patients. Clin Exp Immunol. 1999; 116: 146-152).

In a more preferred embodiment, the cell is a pre-fixed cell, which term means, as used herein, that the cell has been treated with a fixing reagent such as formalin prior to incubation with the sample. In other words, the sample is absent from the cell or the carrier comprising such a cell or cells when the cell is fixed. Such a cell is metabolically inactive, but presents autoantigen epitopes such that they are reactive with autoantibodies. It is conserved and can be shipped in a kit.

In a preferred embodiment, the cell is transiently transfected, which preferably means that the cell is transfected with a vector comprising a nucleic acid encoding for an autoantigen such as fetal or adult ACh receptor or Musk, but this nucleic acid is not integrated into the genome of the cell. Consequently, the cell may lose the vector when cultivated for a prolonged time to the effect that it no longer expresses said polypeptide.

In a preferred embodiment, the cell permanently expresses an autoantigen such as fetal or adult ACh receptor or Musk, more preferably because a nucleic acid encoding for a polypeptide said autoantigen has been integrated into the genome. Consequently, the cell is likely to express the polypeptide permanently. This may also be referred to as a stable transfection or a stable expression of the polypeptide.

In a preferred embodiment, the problem is solved by a method comprising the steps of coating a diagnostically useful carrier with a eukaryotic cell overexpressing an adult ACh receptor or a variant thereof and a eukaryotic cell overexpressing a fetal ACh receptor or a variant thereof, optionally a cell overexpressing Musk or a variant thereof, and fixing the cells for immunofluorescence.

According to the invention method, preferably for the diagnosis of myasthenia gravis, LEMS and OMD, comprising the step of detecting in a sample an autoantibody to ACh receptor, optionally in addition to an autoantibody to Musk, is provided comprising the step contacting the sample with the carrier according to the present invention under conditions allowing formation of a complex comprising an autoantibody and an autoantigen from the group comprising ACh receptor and Musk. This may be done by placing in contact with the cell a drop of the adequately diluted sample to be examined and incubating, for example for 30 minutes. PBS Tween may be used for diluting. A drop may preferably comprise 20 to 100 µl, preferably 25 to 50 µl. The sample may be diluted 1:1 to 1:100, preferably 1:5 to 1:20. The sample may then be removed and the cell on the carrier may be washed. PBS Tween may be used as washing buffer. If the carrier comprises more than one cell, it is preferred that the same sample is contacted with the two or more cells at the same time, more preferably in a one-pot reaction wherein the same portion of sample is in contact with two or more, preferably all cells on the carrier.

The cell and any complex formed in the previous step may then be contacted with a fluorescent ligand to the autoantibody. This can be done by placing in contact with the cell, preferably two or more or all cells on the carrier, a drop or portion of a solution comprising a ligand comprising one or more fluorescent labels. In a particularly preferred embodiment, the fluorescent label is a fluorescein derivative, preferably FITC.

Subsequently, any fluorescent ligand not bound to an autoantibody may be removed, for example by washing the carrier in an excess of washing buffer. Finally, the absence or presence of the autoantibody may be detected, for example by examining whether the carrier comprises a fluorescent pattern using an immunofluorescence microscope, for example the EUROStar Bluelight system.

In a preferred embodiment, the autoantibody, more specifically its absence or presence, is detected by detecting the presence or absence of a fluorescent ligand. There is no need to detect the colocalization of the fluorescent ligand and a fluorescent label associated with the ACh receptor complex. Preferably the ACh receptor complex is not fluorescent or has fluorescent properties that differ from the ligand's fluorescent properties such that any fluorescent signal emitted by the AChR cannot be confused with a fluorescent signal by the fluorescent ligand.

According to the invention, a method for the detection of an autoantibody to ACh receptor, optionally in addition an autoantibody to Musk, a sample is contacted with the carrier according to the present invention under conditions allowing formation of a complex comprising an autoantibody and an autoantigen from the group comprising ACh receptor and Musk. Suitable buffers and conditions are described in the state of the art, for example George et al.. Subsequently, any excess sample is removed and the carrier may be washed. The carrier is then contacted with a fluorescent ligand to the autoantibody in the complex formed in the first step. Such a ligand may be a ligand binding to the autoantibody and carrying a fluorescent label, linked via a covalent or non-covalent bond, optionally via a linker molecule. For example, the ligand may be a secondary antibody carrying a biotin label bound to streptavidin labeled with a fluorescent label. If the fluorescent ligand comprises more than one molecule, more specifically a secondary antibody binding to the autoantibody and, via a non-covalent bond, a fluorescent molecule binding to the secondary antibody, the autoantibody may optionally first be contacted with the secondary antibody only followed, by incubation with the secondary antibody. Any excess ligand may then be separated from the carrier, for example by washing. The presence of the autoantibody is then detected by examining whether the fluorescent ligand is present or not. In a more preferred embodiment, there is no need to check the colocalization of the fluorescent ligand with another fluorescent ligand. Only one type of fluorescent label is used for the detection.

In a preferred embodiment, the fluorescent ligand binds specifically to the autoantibody, preferably in the sense that it recognizes the Ig class of the autoantibody, preferably IgG, but essentially does not bind to any other Ig classes. In a preferred embodiment, the term "binding specifically", as used herein, means that the binding is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7. The fluorescent ligand may be a monoclonal secondary or a polyclonal secondary antibody. The fluorescent ligand may be an isolated and/or purified secondary antibody. The fluorescent ligand may carry a single type of fluorescent label.

The carrier according to the present invention may be manufactured by coating a carrier such as a glass carrier with the cell or cells expressing one or more autoantigens. The cells may then be fixed. In a preferred embodiment, a fixed mammalian cell may be used. In a preferred embodiment, the term "fixed" cell, as used herein, refers to a cell that has been treated with a reactive chemical compound to the effect that the cell is no longer metabolically active, but still presents its epitopes for immunostaining with antibodies and their subsequent detection, for example by fluorescence. More preferably, the reactive chemical compound is selected from the group comprising acetone, formalin, methanol and ethanol or mixtures thereof, preferably formalin. The person skilled in the art is familiar with protocols that may be used to prepare fixed cells. Essentially, the cell which is attached to a solid support is washed by using washing buffer, followed by contacting with the reactive compound, for example immersion.

In a preferred embodiment, the term "autoantibody", as used herein, refers to an antibody binding specifically to an endogenous molecule of the animal, preferably mammal, more preferably human, which produces said autoantibody, wherein the level of such antibody is more preferably elevated compared to the average healthy subject. In a most preferred embodiment, the autoantibody is an autoantibody binding to mammalian, preferably human ACh receptor. The autoantibody may have the sequence of an antibody's constant regions from the animal, preferably human, making it, but the variable region is able to bind specifically to the endogenous molecule of the animal, more specifically ACh receptor. In a preferred embodiment, the autoantibody is isolated and/or purified from a sample, preferably tissue, serum, plasma, blood or CSF from the animal, preferably human. The autoantibody is a polyclonal, native antibody from the animal rather than a synthetic or recombinant antibody. Preferably it is a human autoantibody.

The method according to the present invention is preferably an *in vitro* method.

According to the present invention, a kit is provided, comprising the cell or the carrier and further comprising one or more, preferably all reagents from the group comprising a ligand to an autoantibody to the ACh receptor, preferably a secondary antibody, preferably labeled with a fluorescent label, a washing buffer, a positive control, a negative control, a detergent, a cover glass, a mounting medium and a physiological salt solution, preferably PBS, or salt required to prepare it. In a preferred embodiment, the positive control is a diluted sample, preferably serum from a patient suffering from a disease associated with ACh receptor autoantibodies or a monoclonal antibody to human ACh receptor. The negative control may be a diluted sample from a healthy subject, for example a blood donor. The kit may comprise instructions how to carry out the assay.

In a preferred embodiment, the term "immunofluorescence assay", as used herein, means that an autoantibody is detected by fluorescence, more preferably by detection of the presence or absence of a fluorescence ligand bound to said autoantibody, while the autoantibody is bound to the autoantigen to which it binds. The autoantigen may be overexpressed by a cell and the presence or absence of the ligand may be determined by detecting, using a fluorescence microscope, whether a fluorescence signal, preferably fluorescence pattern determined by the location of the autoantigen on the cell, is present or absent.

In a preferred embodiment, any cell, method, kit, polypeptide use or carrier according to the present invention is used for the diagnosis of an autoimmune disease, preferably from the group comprising myasthenia gravis, LEMS and OMD.

In a preferred embodiment, the term "diagnosis", as used herein, is to be used in its broadest possible sense and may refer to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness a patient or patients in general with regard to a certain treatment, for example the administration of immunosuppressive drugs or an immunotherapeutic treatment, or to find out whether a sample is from such a patient. Such information may be used for a clinical diagnosis, but may also be obtained by an experimental and/or research laboratory for the purpose of general research, for example to determine the proportion of subjects suffering from the disease in a patient cohort or in a population. In other words, the term "diagnosis" comprises not only diagnosing, but also prognosticating and/or monitoring the course of a disease or disorder, including monitoring the response of one or more patients to the administration of a drug or candidate drug, for example to determine its efficacy. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to an sample from an anonymized patient.ln a preferred embodiment, the methods and products according to the present invention may be used for interaction studies, including determining whether a drug candidate or other compound may interfere with the binding of an autoantibody to a human ACh receptor or may affect any downstream process or the strength of their binding to their target. In preferred embodiment, they may be used for monitoring the immune response, more preferably the emergence and/or titer of autoantibodies to a human autoantibody to a human ACh receptor, following the administration of an drug such as an immunosuppressive drug.

In many cases the mere detection of the autoantibody, preferably determining whether or not detectable levels of the antibody are present in the sample, is sufficient for the diagnosis. If the autoantibody can be detected, this will be information instrumental for the clinician's diagnosis and indicates an increased likelihood that the patient suffers from a disease.

The person skilled in the art will appreciate that a clinician does usually not conclude whether or not the patient suffers or is likely to suffer from a disease, condition or disorders solely on the basis of a single diagnostic parameter, but needs to take into account other aspects, for example the presence of other autoantibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred embodiment, the meaning of any symptoms or diseases referred to throughout this application is in line with the person skilled in the art's understanding as of the filing date or, preferably, earliest priority date of this application as evidenced by text books and scientific publications. It should be mentioned that the inventive methods or uses or products, taken alone, cannot be used to arrive at a definite, final diagnosis.

In a preferred embodiment, the term "diagnosis" may also refer to a method or agent used to choose the most promising treatment regime for a patient. In other words, the method or agent may relate to selecting a treatment regimen for a subject. For example, the detection of autoantibodies may indicate that an immunosuppressive therapy is to be selected, which may include administrating to the patient one or more immunosuppressive drugs.

In a preferred embodiment, any information or data demonstrating the presence of absence of the autoantibody may be communicated to the patient or a medical doctor treating the patient, preferably by telephone, in a written form or via the internet, for example as an email or text message.

In a preferred embodiment, any method or use according to the present invention may be intended for a non-diagnostic use, *i.e.* determining the presence of an autoantibody to binding to human ACh receptor for a use other than diagnosing a patient. For example, the method or use may be for testing *in vitro* the efficiency of a medical device designed to remove an autoantibody from a patient's blood, wherein the testing is performed on a liquid other than patient's blood. After the use of the medical device with a patient, its capacity to remove autoantibody may be checked by running a solution comprising antibody to human ACh receptor through the device, followed by use of the method according to the present invention to confirm that less or no antibody is in the solution that has been passed through the device, i.e. showing that the device has still the capacity to remove antibody from the solution.

In a preferred embodiment, the present invention provides an apparatus for analyzing a sample from a patient to detect an autoantibody against human ACh receptor, indicating an increased likelihood of a disease or of developing a disease, comprising:
a) a carrier, which contains a means for capturing the autoantibody from the sample when the sample is contacted with the carrier, wherein the means is the cell and the carrier is the carrier according to the present invention,
b) a detectable means capable of binding to the antibody captured by the carrier when the detectable means is contacted with the carrier, wherein the detectable means is preferably a fluorescent ligand capable of binding to the autoantibody captured on the carrier,
c) optionally a means for removing any sample from the carrier and the detectable means, preferably by washing;
d) a detecting device for detecting the presence or absence of the detectable means and converting the results into an electrical signal, for example a fluorescence reader connected with a software capable of recognizing a pattern characteristic of a stained cell on the carrier according to the present invention in an image of the cell taken by the fluorescence reader or camera, and
optionally a means for receiving the electronical signal from the detecting device and determining if the level of the signal is indicative of an increased likelihood of having or developing a disease, by comparing with the patterns characteristic of wild type or non-stained cells, preferably by a mock-transfected cell or cells not positively stained by an autoantibody binding to human ACh receptor on the same carrier, or an input reference value obtained with samples from healthy subjects or by comparing the level of signal obtained with one sample with the level of signal obtained with a second sample obtained at a later time point, preferably at least one month later.

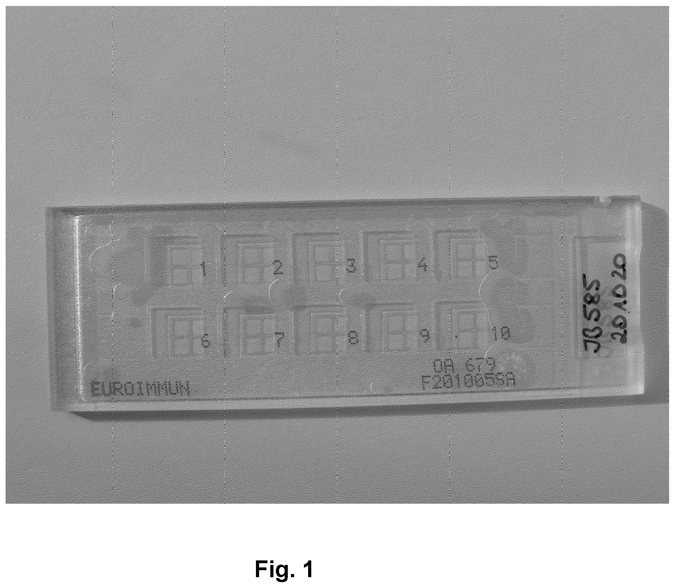

**Fig. 1** shows a representative Biochip mosaik comprising cells in four separate panels in each of the ten fields, more specifically fixed HEK293 cells expressing the ACh receptor comprising the epsilon subunit (upper left panel) and fixed HEK293 cells expressing the ACh receptor comprising the gamma subunit (bottom left panel). Fixed HEK293 cells transfected with the ACh receptor comprising the epsilon subunit, but without induction of expression (upper right panel) and fixed HEK293 cells transfected with the ACh receptor comprising the gamma subunit, but without induction of expression (bottom left panel) serve as negative controls.

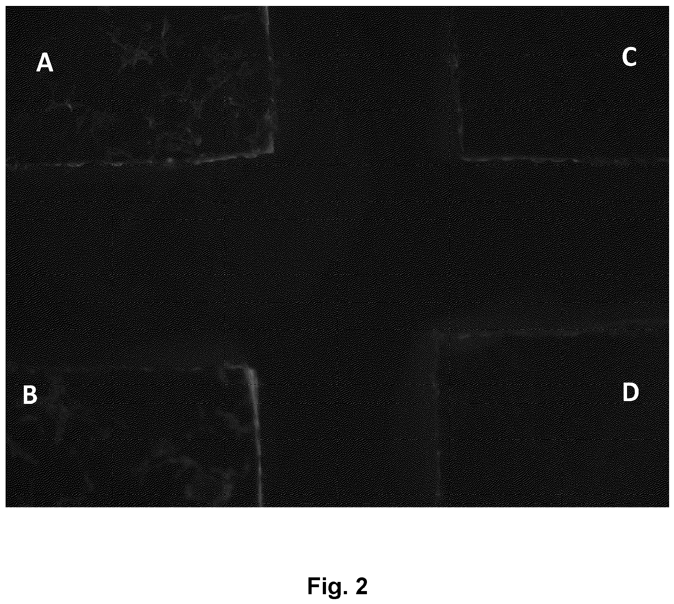

**Fig. 2** shows a fluorescence image comprising corners of the four panels following incubation with a sample comprising autoantibodies to both the ACh receptor comprising the epsilon subunit and the ACh receptor comprising the gamma subunit. The panels comprise (**A**) a fixed HEK293 cell expressing the ACh receptor comprising the epsilon subunit (upper left panel) and (**B**) a fixed HEK293 cells expressing the ACh receptor comprising the gamma subunit (bottom left panel). (**C**) A fixed HEK293 cell transfected with the ACh receptor comprising the epsilon subunit, but without induction of expression (upper right panel) and (**D**) a fixed HEK293 cells transfected with the ACh receptor comprising the gamma subunit, but without induction of expression (bottom left panel) serve as negative controls.

### Sequences:

The present invention comprises a range of novel nucleic acid and polypeptide sequences, more specifically

SEQ ID NO1 CHRNA1 (Isoform 1, UniProt: P02708-2)










SEQ ID NO2 CHRNB1 (Isoform 1, UniProt: P11230-1)
Mtpgallmllgalgaplapgvrgseaegrlreklfsgydssvrparevgdrvrvsvglilaqlislnekdeemst
kvyldlewtdyrlswdpaehdgidslritaesvwlpdvvllnnndgnfdvaldisvvvssdgsvrwqppgiyrss
csiqvtyfpfdwqnctmvfssysydssevslqtglgpdgqghqeihihegtfiengqweiihkpsrliqppgdpr
ggregqrqevifyliirrkplfylvnviapcilitllaifvfylppdagekmglsifalltltvflllladkvpe
tslsvpiiikylmftmvlvtfsvilsvvvlnlhhrsphthqmplwvrqifihklplylrlkrpkperdlmpepph
csspgsgwgrgtdeyfirkppsdflfpkpnrfqpelsapdlrrfidgpnravallpelrevvssisyiarqlqeq
edhdalkedwqfvamvvdrlflwtfiiftsvgtlviflldatyhlpppdpfp

SEQ ID NO3 CHRND (Isoform 1, UniProt: Q07001-1)
Megpvltlgllaalavcgswglneeerlirhlfqekgynkelrpvahkeesvdvalaltlsnlislkeveetltt
nvwiehgwtdnrlkwnaeefgnisvlrlppdmvwlpeivlennndgsfqisyscnvlvyhygfvywlppaifrss
cpisvtyfpfdwqncslkfsslkytakeitlslkqdakenrtypvewiiidpegftengeweivhrparvnvdpr
apldspsrqditfyliirrkplfyiinilvpcvlisfmvnlvfylpadsgektsvaisvllaqsvflllisrklp
atsmaipligkfllfgmvlvtmvvvicvivlnihfrtpsthvlsegvkklfletlpellhmsrpaedgpspgalv
rrssslgyiskaeeyfllksrsdlmfekqserhglarrlttarrppasseqaqqelfnelkpavdganfivnhmr
dqnnyneekdswnrvartvdrlclfvvtpvmvvgtawiflqgvynqpppqpfpgdpysynvqdkrfi

SEQ ID NO4 CHRNE (Isoform 1, UniProt: Q04844-1)
Maraplgvllllgllgrgvgkneelrlyhhlfnnydpgsrpvrepedtvtislkvtltnlislnekeetlttsvw
igidwqdyrlnyskddfggietlrvpselvwlpeivlennidgqfgvaydanvlvyeggsvtwlppaiyrsvcav
evtyfpfdwqncslifrsqtynaeevefftavdndgktinkididteaytengewaidfcpgvirrhhggatdgp
getdviysliirrkplfyviniivpcvlisglvllayflpaqaggqkctvsinvllaqtvflfliaqkipetsls
vpllgrflifvmvvatlivmncvivlnvsqrtptthamsprlrhvllellprllgsppppeapraasspprrassv
glllraeelilkkprselvfegqrhrqgtwtaafcqslgaaapevrccvdavnfvaestrdqeatgeevsdwvrm
gnaldnicfwaalvlfsvgssliflgayfnrvpdlpyapciqp

SEQ ID NO5 CHRNG (Isoform 1, UniProt: P07510-1)
mhggqgpllllllllavclgaqgrnqeerlladlmqnydpnlrpaerdsdvvnvslkltltnlislnereealttn
vwiemqwcdyrlrwdprdyeglwvlrvpstmvwrpdivlennvdgvfevalycnvlvspdgciywlppaifrsac
sisvtyfpfdwqncslifqsqtystneidlqlsqedgqtiewifidpeaftengewaiqhrpakmlldpaapaqe
aghqkvvfyllliqrkplfyviniiapcvlissvailihflpakaggqkctvainvllaqtvflflvakkvpetsq
avpliskyltfllvvtilivvnavvvlnvslrsphthsmargvrkvflrllpqllrmhvrplapaavqdtqsrlq
ngssgwsittgeevalclprsellfqqwqrqglvaaaleklekgpelglsqfcgslkqaapaiqacveacnliac
arhqqshfdngneewflvgrvldrvcflamlslficgtagiflmahynrvpalpfpgdprpylpspd

SEQ ID NO6 Rapsyn (Isoform 1, Uni-Prot: Q13702-1)







SEQ ID NO7 pTriEx-1-CHRNA1
ggggaattgtgagcggataacaattccccggagttaatccgggacctttaattcaacccaacacaatatattata
gttaaataagaattattatcaaatcatttgtatattaattaaaatactatactgtaaattacattttatttacaa
tcaaaggagatataccatggagccctggcctctcctcctgctctttagcctttgctcagctggcctcgtcctggg
ctccgaacatgagacccgtctggtggcaaagctatttaaagactacagcagcgtggtgcggccagtggaagacca
ccgccaggtcgtggaggtcaccgtgggcctgcagctgatacagctcatcaatgtggatgaagtaaatcagatcgt
gacaaccaatgtgcgtctgaaacagcaatgggtggattacaacctaaaatggaatccagatgactatggcggtgt
gaaaaaaattcacattccttcagaaaagatctggcgcccagaccttgttctctataacaatgcagatggtgactt
tgctattgtcaagttcaccaaagtgctcctgcagtacactggccacatcacgtggacacctccagccatctttaa

aagctactgtgagatcatcgtcacccactttccctttgatgaacagaactgcagcatgaagctgggcacctggac
ctacgacggctctgtcgtggccatcaacccggaaagcgaccagccagacctgagcaacttcatggagagcgggga
gtgggtgatcaaggagtcccgggctggaagcactccgtgacctattcctgctgccccgacacccctacctgga
catcacctaccacttcgtcatgcagcgcctgcccctctacttcatcgtcaacgtcatcatccctgcctgctctt
ctccttcttaactggcctggtattctacctgcccacagactcaggggagaagatgactctgagcatctctgtctt
actgtctttgactgtgttccttctggtcatcgtggagctgatccctccacgtccagtgctgtgcccttgattgg
aaaatacatgctgttcaccatggtgttcgtcattgcctccatcatcatcactgtcatcgtcatcaacacacacca
ccgctcacccagcacccatgtcatgcccaactgggtgcggaaggttttatcgacactatcccaaatatcatgtt
tttctccacaatgaaaagaccatccagagaaaagcaagacaaaaagattttacagaagacattgatatctctga
catttctggaaagccagggcctccacccatgggcttccactctcccctgatcaaacaccccgaggtgaaaagtgc
catcgagggcatcaagtacatcgcagagaccatgaagtcagaccaggagtctaacaatgcggcggcagagtggaa
gtacgttgcaatggtgatggaccacatactcctcggagtcttcatgcttgtttgcatcatcggaaccctagccgt
gtttgcaggtcgactcattgaattaaatcagcaaggatgactcgagcaccaccatcaccatcaccatcactaagt
gattaacctcaggtgcaggctgcctatcagaaggtggtggctggtgtggccaatgccctggctcacaaataccac
tgagatcgatctttttccctctgccaaaaattatggggacatcatgaagccccttgagcatctgacttctggcta
ataaaggaaatttattttcattgcaatagtgtgttggaattttttgtgtctctcactcggaaggacatatggag
ggcaaatcatttaaaacatcagaatgagtatttggtttagagtttggcaacatatgcccatatgtaactagcata
accccttggggcctctaaacgggtcttgaggggttttttgctgaaagcatgcggaggaaattctccttgaagttt
ccctggtgttcaaagtaaaggagtttgcaccagacgcacctctgttcactggtccggcgtattaaaacacgatac
attgttattagtacatttattaagcgctagattctgtgcgttgttgatttacagacaattgttgtacgtatttta
ataattcattaaatttataatctttagggtggtatgttagagcgaaaatcaaatgatttcagcgtctttatatc
tgaatttaaatattaaatcctcaatagatttgtaaaataggtttcgattagtttcaaacaagggttgtttttccg
aaccgatggctggactatctaatggatttcgctcaacgccacaaaacttgccaaatcttgtagcagcaatctag
ctttgtcgatattcgtttgtgttttgttttgtaataaaggttcgacgtcgttcaaaatattatgcgcttttgtat
ttctttcatcactgtcgttagtgtacaattgactcgacgtaaacacgttaaatagagcttggacatatttaacat
cgggcgtgttagctttattaggccgattatcgtcgtcgtcccaaccctcgtcgttagaagttgcttccgaagacg
attttgccatagccacacgacgcctattaattgtgtcggctaacacgtccgcgatcaaatttgtagttgagcttt
ttggaattatttctgattgcgggcgtttttgggcgggtttcaatctaactgtgcccgattttaattcagacaaca
cgttagaaagcgatggtgcaggcggtggtaacatttcagacggcaaatctactaatggcggcggtggtggagctg
atgataaatctaccatcggtggaggcgcaggcggggctggcggcggaggcggaggcggaggtggtggcggtgatg
cagacggcggtttaggctcaaatgtctctttaggcaacacagtcggcacctcaactattgtactggtttcgggcg
ccgtttttggtttgaccggtctgagacgagtgcgatttttttcgtttctaatagcttccaacaattgttgtctgt
cgtctaaaggtgcagcgggttgaggttccgtcggcattggtggagcgggcggcaattcagacatcgatggtggtg
gtggtggtggaggcgctggaatgttaggcacgggagaaggtggtggcggcggtgccgccggtataatttgttctg
gtttagtttgttcgcgcacgattgtgggcaccggcgcaggcgccgctggctgcacaacggaaggtcgtctgcttc
gaggcagcgcttggggtggtggcaattcaatattataattggaatacaaatcgtaaaaatctgctataagcattg
taatttcgctatcgtttaccgtgccgatatttaacaaccgctcaatgtaagcaattgtattgtaaagagattgtc
tcaagctcggaacgctgcgctcggtcgttcggctgcggcgagcggtatcagctcactcaaaggcggtaatacggt
tatccacagaatcaggggataacgcaggaaagaacatgtgagcaaaaggccagcaaaaggccaggaaccgtaaaa
aggccgcgttgctggcgtttttccataggctccgcccccctgacgagcatcacaaaaatcgacgctcaagtcaga
ggtggcgaaacccgacaggactataaagataccaggcgtttccccctggaagctccctcgtgcgctctcctgttc
cgaccctgccgcttaccggatacctgtccgcctttctcccttcgggaagcgtggcgctttctcaatgctcacgct
gtaggtatctcagttcggtgtaggtcgttcgctccaagctgggctgtgtgcacgaaccccccgttcagcccgacc
gctgcgccttatccggtaactatcgtcttgagtccaacccggtaagacacgacttatcgccactggcagcagcca
ctggtaacaggattagcagagcgaggtatgtaggcggtgctacagagttcttgaagtggtggcctaactacggct
acactagaaggacagtatttggtatctgcgctctgctgaagccagttaccttcggaaaaagagttggtagctctt
gatccggcaaacaaaccaccgctggtagcggtggtttttttgtttgcaagcagcagattacgcgcagaaaaaaag
gatctcaagaagatcctttgttaccaatgcttaatcagtgaggcacctatctcagcgatctgtctatttcgttca
tccatagttgcctgactccccgtcgtgtagataactacgatacgggagggcttaccatctggccccagtgctgca
atgataccgcgagacccacgctcaccggctccagatttatcagcaataaaccagccagccggaagggccgagcgc
agaagtggtcctgcaactttatccgcctccatccagtctattaattgttgccgggaagctagagtaagtagttcg
ccagttaatagtttgcgcaacgttgttgccattgctacaggcatcgtggtgtcacgctcgtcgtttggtatggct
tcattcagctccggttcccaacgatcaaggcgagttacatgatcccccatgttgtgcaaaaaagcggttagctcc
ttcggtcctccgatcgttgtcagaagtaagttggccgcagtgttatcactcatggttatggcagcactgcataat
tctcttactgtcatgccatccgtaagatgcttttctgtgactggtgagtactcaaccaagtcattctgagaatag
tgtatgcggcgaccgagttgctcttgcccggcgtcaatacgggataataccgcgccacatagcagaactttaaaa
gtgctcatcattggaaaacgttcttcggggcgaaaactctcaaggatcttaccgctgttgagatccagttcgatg
taacccactcgtgcacccaactgatcttcagcatcttttactttcaccagcgtttctgggtgagcaaaaacagga
aggcaaaatgccgcaaaaaagggaataagggcgacacggaaatgttgaatactcatactcttcctttttcaatat

tattgaagcatttatcagggttattgtctcatgtccgcgtttcctgcatcttttaatcaaatcccaagatgtg
tataaaccaccaaactgccaaaaaatgaaaactgtcgacaagctctgtccgtttgctggcaactgcaaggtctc
aatcctatttgtaattattgaataataaaacaattataaatgtcaaatttgtttttattaacgatacaaaccaa
acgcaacaagaacatttgtagtattatctataattgaaaacgcgtagttataatcgctgaggtaatatttaaaat
cattttcaaatgattcacagttaatttgcgacaatataatttattttcacataaactagacgccttgtcgtctt
cttcttcgtattccttctcttttttcatttttctcttcataaaaattaacatagttattatcgtatccatatatgt
atctatcgtatagagtaaattttttgttgtcataaatatatatgtctttttttaatggggtgtatagtaccgctgc
gcatagtttttctgtaatttacaacagtgctattttctggtagttcttcggagtgtgttgctttaattattaaat
ttatataatcaatgaatttgggatcgtcggttttgtacaatatgttgccggcatagtacgcagcttcttctagtt
caattacaccatttttttagcagcaccggattaacataactttccaaaatgttgtacgaaccgttaaacaaaaaca
gttcacctcccttttctatactattgtctgcgagcagttgtttgttgttaaaaataacagccattgtaatgagac
gcacaaactaatatcacaaactggaaatgtctatcaatatatagttgctctagttattaatagtaatcaattacg
gggtcattagttcatagcccatatatggagttccgcgttacataacttacggtaaatggcccgcctggctgaccg
cccaacgacccccgcccattgacgtcaataatgacgtatgttcccatagtaacgccaatagggactttccattga
cgtcaatgggtggactatttacggtaaactgcccacttggcagtacatcaagtgtatcatatgccaagtacgccc
cctattgacgtcaatgacggtaaatggcccgcctggcattatgcccagtacatgaccttatgggactttcctact
tggcagtacatctacgtattagtcatcgctattaccatgcatggtcgaggtgagccccacgttctgcttcactct
ccccatctcccccccctccccacccccaattttgtatttatttattttttaattattttgtgcagcgatgggggc
ggggggggggggggggcgcgcgccaggcggggcggggcggggcgaggggcggggcggggcgaggcggagaggtgc
ggcggcagccaatcagagcggcgcgctccgaaagtttccttttatggcgaggcggcggcggcggcggccctataa
aaagcgaagcgcgcggcgggcgggagtcgctgcgacgctgccttcgccccgtgccccgctccgccgccgcctcgc
gccgcccgccccggctctgactgaccgcgttactcccacaggtgagcgggcgggacggcccttctccttcgggct
gtaattagcgcttggtttaatgacggcttgtttcttttctgtggctgcgtgaaagccttgaggggctccgggagg
gccctttgtgcggggggagcggctcggggctgtccgcggggggacggctgccttcgggggggacggggcagggcg
gggttcggcttctggcgtgtgaccggcggctctagagcctctgctaaccatgttcatgccttcttctttttccta
cagctcctgggcaacgtgctggttattgtgctgtctcatcattttggcaaagaattggatcggaccgaaattaat
acgactcactata

SEQ ID NO8 pTriEx-1-CHRNB1
ggggaattgtgagcggataacaattccccggagttaatccgggacctttaattcaacccaacacaatatattata
gttaaataagaattattatcaaatcatttgtatattaattaaaatactatactgtaaattacattttatttacaa
tcaaaggagatataccatgaccccaggggctctgctgatgctgctgggggcgctgggggcgccgctcgccccagg
cgtccgcggctcggaggcggagggtcgactccgggagaaacttttctctggctatgatagctccgtgcggccagc
gcgggaggtgggagaccgtgtcagggtcagcgttggtctcatcctggcgcaactcatcagcctgaacgagaagga
tgaagagatgagcacaaaggtgtacttagacctggagtggactgactacaggctgagctgggaccctgcggagca
cgacggcatcgattcgctccgcatcacggcggaatccgtgtggctccctgacgtggtgctactgaacaacaatga
tgggaattttgacgtggctctggacattagcgtcgtggtgtcctccgacggctccgtgcgttggcaaccccccggg
catctatcgcagcagctgcagcatccaggtcacctacttccccttcgactggcagaattgcactatggtgttcag
ctcctacagctacgacagctcggaggtcagcctgcagacaggcctgggtcctgacgggcaagggcatcaggaaat
ccacattcatgaaggactttcattgagaatggccagtgggagattatccacaagccctctcggctaatccagcc
tccaggcgatcctaggggagggagggaaggacagcgccaggaagtcatcttctacctcatcatccgccgcaagcc
tctcttctacctggtcaacgtcattgccccatgcatcctcatcactcttctggccatcttcgtcttctacctgcc
accagatgcaggagagaagatggggctctcaatctttgccctgctgacccttactgtgttcctgctgctgctggc
tgacaaagtacctgagacctcactatcagtacccattattatcaagtacctcatgtttaccatggtcctcgtcac
cttctcagtcatccttagtgtcgtggttctcaacctgcaccaccgctcacccacacccaccaaatgcccctttg
ggtccgtcagatcttcattcacaaacttccgctgtacctgcgtctaaaaaggcccaaacccgagagagacctgat
gccggagcccctcactgttcttctccaggaagtggctggggtcggggaacagatgaatatttcatccggaagcc
gccaagtgattttctcttccccaaacccaataggttccagcctgaactgtctgcccctgatctgcggcgatttat
cgatggtccaaaccgggctgtggccctgcttccggagctacgggaggtcgtctcctctatcagctacatcgctcg
acagctgcaggaacaggaggaccacgatgcgctgaaggaggactggcagtttgtggccatggtagtggaccgcct
cttcctgtggactttcatcatcttcaccagcgttgggaccctagtcatcttcctggacgccacgtaccacttgcc
ccctccagacccctttccttaactcgagcaccaccatcaccatcaccatcactaagtgattaacctcaggtgcag
gctgcctatcagaaggtggtggctggtgtggccaatgccctggctcacaaataccactgagatcgatcttttcc
ctctgccaaaaattatggggacatcatgaagccccttgagcatctgacttctggctaataaaggaaatttatttt
cattgcaatagtgtgttggaattttttgtgtctctcactcggaaggacatatgggagggcaaatcatttaaaaca
tcagaatgagtatttggtttagagtttggcaacatatgcccatatgtaactagcataaccccttggggcctctaa
acgggtcttgaggggttttttgctgaaagcatgcggaggaaattctccttgaagtttccctggtgttcaaagtaa
aggagtttgcaccagacgcacctctgttcactggtccggcgtattaaaacacgatacattgttattagtacattt
attaagcgctagattctgtgcgttgttgatttacagacaattgttgtacgtattttaataattcattaaatttat

aatctttagggtggtatgttagagcgaaaatcaaatgattttcagcgtctttatatctgaatttaaatattaaat
cctcaatagatttgtaaaataggtttcgattagtttcaaacaagggttgtttttccgaaccgatggctggactat
ctaatggattttcgctcaacgccacaaaacttgccaaatcttgtagcagcaatctagctttgtcgatattcgttt
gtgttttgttttgtaataaaggttcgacgtcgttcaaaatattatgcgcttttgtatttctttcatcactgtcgt
tagtgtacaattgactcgacgtaaacacgttaaatagagcttggacatatttaacatcgggcgtgttagctttat
taggccgattatcgtcgtcgtcccaaccctcgtcgttagaagttgcttccgaagacgattttgccatagccacac
gacgcctattaattgtgtcggctaacacgtccgcgatcaaatttgtagttgagctttttggaattatttctgatt
gcgggcgtttttgggcgggtttcaatctaactgtgcccgattttaattcagacaacacgttagaaagcgatggtg
caggcggtggtaacatttcagacggcaaatctactaatggcggcggtggtggagctgatgataaatctaccatcg
gtggaggcgcaggcggggctggcggcggaggcggaggcggaggtggtggcggtgatgcagacggcggtttaggct
caaatgtctctttaggcaacacagtcggcacctcaactattgtactggtttcgggcgccgtttttggtttgaccg
gtctgagacgagtgcgatttttttcgtttctaatagcttccaacaattgttgtctgtcgtctaaaggtgcagcgg
gttgaggttccgtcggcattggtggagcgggcggcaattcagacatcgatggtggtggtggtggaggcgctg
gaatgttaggcacgggagaaggtggtggcggcggtgccgccggtataatttgttctggtttagtttgttcgcgca
cgattgtgggcaccggcgcaggcgccgctggctgcacaacggaaggtcgtctgcttcgaggcagcgcttggggtg
gtggcaattcaatattataattggaatacaaatcgtaaaaatctgctataagcattgtaatttcgctatcgttta
ccgtgccgatatttaacaaccgctcaatgtaagcaattgtattgtaaagagattgtctcaagctcggaacgctgc
gctcggtcgttcggctgcggcgagcggtatcagctcactcaaaggcggtaatacggttatccacagaatcagggg
ataacgcaggaaagaacatgtgagcaaaaggccagcaaaaggccaggaaccgtaaaaaggccgcgttgctggcgt
ttttccataggctccgcccccctgacgagcatcacaaaaatcgacgctcaagtcagaggtggcgaaacccgacag
gactataaagataccaggcgtttccccctggaagctccctcgtgcgctctcctgttccgaccctgccgcttaccg
gatacctgtccgcctttctcccttcgggaagcgtggcgctttctcaatgctcacgctgtaggtatctcagttcgg
tgtaggtcgttcgctccaagctgggctgtgtgcacgaaccccccgttcagcccgaccgctgcgccttatccggta
actatcgtcttgagtccaacccggtaagacacgacttatcgccactggcagcagccactggtaacaggattagca
gagcgaggtatgtaggcggtgctacagagttcttgaagtggtggcctaactacggctacactagaaggacagtat
ttggtatctgcgctctgctgaagccagttaccttcggaaaaagagttggtagctcttgatccggcaaacaaacca
ccgctggtagcggtggtttttttgtttgcaagcagcagattacgcgcagaaaaaaaggatctcaagaagatcctt
tgttaccaatgcttaatcagtgaggcacctatctcagcgatctgtctatttcgttcatccatagttgcctgactc
cccgtcgtgtagataactacgatacgggagggcttaccatctggccccagtgctgcaatgataccgcgagaccca
cgctcaccggctccagatttatcagcaataaaccagccagccggaagggccgagcgcagaagtggtcctgcaact
ttatccgcctccatccagtctattaattgttgccgggaagctagagtaagtagttcgccagttaatagtttgcgc
aacgttgttgccattgctacaggcatcgtggtgtcacgctcgtcgtttggtatggcttcattcagctccggttcc
caacgatcaaggcgagttacatgatcccccatgttgtgcaaaaaagcggttagctccttcggtcctccgatcgtt
gtcagaagtaagttggccgcagtgttatcactcatggttatggcagcactgcataattctcttactgtcatgcca
tccgtaagatgcttttctgtgactggtgagtactcaaccaagtcattctgagaatagtgtatgcggcgaccgagt
tgctcttgcccggcgtcaatacgggataataccgcgccacatagcagaactttaaaagtgctcatcattggaaaa
cgttcttcggggcgaaaactctcaaggatcttaccgctgttgagatccagttcgatgtaacccactcgtgcaccc
aactgatcttcagcatcttttactttcaccagcgtttctgggtgagcaaaaacaggaaggcaaaatgccgcaaaa
aagggaataagggcgacacggaaatgttgaatactcatactcttcctttttcaatattattgaagcatttatcag
ggttattgtctcatgtccgcgcgtttcctgcatcttttaatcaaatcccaagatgtgtataaaccaccaaactgc
caaaaaatgaaaactgtcgacaagctctgtccgtttgctggcaactgcaagggtctcaatcctatttgtaattat
tgaataataaaacaattataaatgtcaaatttgttttttattaacgatacaaaccaaacgcaacaagaacatttg
tagtattatctataattgaaaacgcgtagttataatcgctgaggtaatatttaaaatcattttcaaatgattcac
agttaatttgcgacaatataattttattttcacataaactagacgccttgtcgtcttcttcttcgtattccttct
ctttttcatttttctcttcataaaaattaacatagttattatcgtatccatatatgtatctatcgtatagagtaa
atttttttgttgtcataaatatatatgtctttttttaatggggtgtatagtaccgctgcgcatagtttttctgtaat
ttacaacagtgctattttctggtagttcttcggagtgtgttgctttaattattaaatttatataatcaatgaatt
tgggatcgtcggttttgtacaatatgttgccggcatagtacgcagcttcttctagttcaattacaccatttttta
gcagcaccggattaacataactttccaaaatgttgtacgaaccgttaaacaaaaacagttcacctcccttttcta
tactattgtctgcgagcagttgtttgttgttaaaaataacagccattgtaatgagacgcacaaactaatatcaca
aactggaaatgtctatcaatatatagttgctctagttattaatagtaatcaattacggggtcattagttcatagc
ccatatatggagttccgcgttacataacttacggtaaatggcccgcctggctgaccgcccaacgacccccgccca
ttgacgtcaataatgacgtatgttcccatagtaacgccaatagggactttccattgacgtcaatgggtggactat
ttacggtaaactgcccacttggcagtacatcaagtgtatcatatgccaagtacgccccctattgacgtcaatgac
ggtaaatggcccgcctggcattatgcccagtacatgaccttatgggactttcctacttggcagtacatctacgta
ttagtcatcgctattaccatgcatggtcgaggtgagccccacgttctgcttcactctccccatctcccccccctc
cccacccccaatttgtatttatttattttttaattattttgtgcagcgatgggggcgggggggggggggggcg
cgcgccaggcggggcggggcggggcgaggggcggggcggggcgaggcggagaggtgcggcggcagccaatcagag
cggcgcgctccgaaagtttccttttatggcgaggcggcggcggcggcggccctataaaaagcgaagcgcgcggcg

ggcgggagtcgctgcgacgctgccttcgccccgtgccccgctccgccgccgcctcgcgccgccccgcccggctct
gactgaccgcgttactcccacaggtgagcgggcgggacggcccttctccttcgggctgtaattagcgcttggttt
aatgacggcttgtttcttttctgtggctgcgtgaaagccttgaggggctccgggagggccctttgtgcgggggga
gcggctcggggctgtccgcgggggggacggctgccttcgggggggacggggcagggcggggttcggcttctggcgt
gtgaccggcggctctagagcctctgctaaccatgttcatgccttcttctttttcctacagctcctgggcaacgtg
ctggttattgtgctgtctcatcattttggcaaagaattggatcggaccgaaattaatacgactcactata

SEQ ID NO9 pTriEx-1-CHRND
ggggaattgtgagcggataacaattccccggagttaatccgggacctttaattcaacccaacacaatatattata
gttaaataagaattattatcaaatcatttgtatattaattaaaatactatactgtaaattacattttatttacaa
tcaaaggagatataccatggagggccagtgctgacactggggctgctggctgccctggcggtgtgtggcagctg
gggctgaacgaggaggagcggctgatccggcacctgtttcaagagaagggctacaacaaggagctccggcccgt
ggcacacaaagaggagagtgtggacgttgccctggccctcacactctccaacctcatctccctgaaagaagttga
ggagaccctcactaccaatgtgtggatagagcacggctggacagacaaccggctgaagtggaatgctgaagaatt
tggaaacatcagtgtcctgcgcctcccccggacatggtgtggctcccagagattgtgctggagaacaacaatga
cggctccttccagatctcctactcctgcaacgtgcttgtctaccactacggcttcgtgtactggctgccacctgc
catcttccgctcctcctgccccatctctgtcacctatttccccttcgactggcagaactgctccctcaagttcag
ttccctcaagtatacggccaaagagatcaccctgagcctgaaacaggatgccaaggagaaccgcacctaccccgt
ggagtggatcatcattgatcctgaaggcttcacagagaacggggagtgggagatagtccaccggccggccagggt
caacgtggaccccagagcccctctggacagccccagccgccaggacatcaccttctacctcatcatccgccgcaa
gcccctcttctacatcatcaacatcctggtgccctgcgtgctcatctccttcatggtcaacctggtcttctacct
accggctgacagtggtgagaagacatcagtggccatctcggtgctcctggctcagtctgtcttcctgctgctcat
ctccaagcgtctgcctgccacatccatggccatccccttatcggcaagttcctgctcttcggcatggtgctggt
caccatggttgtggtgatctgtgtcatcgtgctcaacatccacttccgaacacccagcacccatgtgctgtctga
gggggtcaagaagctcttcctggagaccctgccggagctcctgcacatgtcccgcccagcagaggatggacccag
ccctggggccctggtgcggaggagcagctccctgggatacatctccaaggccgaggagtacttcctgctcaagtc
ccgcagtgacctcatgttcgagaagcagtcagagcggcatgggctggccaggcgcctcaccactgcacgccggcc
cccagcaagctctgagcaggcccagcaggaactcttcaatgagctgaagccagctgtggatgggcaaacttcat
tgttaaccacatgaggaccagaacaattacaatgaggagaaagacagctggaaccgagtggcccgcacagtgga
ccgcctctgcctgtttgtggtgacgcctgtcatggtggtgggcacagcctggatcttcctgcagggcgtttacaa
ccagccaccaccccagccttttcctggggacccctactcctacaacgtgcaggacaagcgcttcatctaactcga
gcaccaccatcaccatcaccatcactaagtgattaacctcaggtgcaggctgcctatcagaaggtggtggctggt
gtggccaatgccctggctcacaaataccactgagatcgatctttttccctctgccaaaaattatggggacatcat
gaagccccttgagcatctgacttctggctaataaaggaaatttattttcattgcaatagtgtgttggaattttt
gtgtctctcactcggaaggacatatgggagggcaaatcatttaaaacatcagaatgagtatttggtttagagttt
ggcaacatatgcccatatgtaactagcataaccccttggggcctctaaacgggtcttgaggggttttttgctgaa
agcatgcggaggaaattctccttgaagtttccctggtgttcaaagtaaaggagtttgcaccagacgcacctctgt
tcactggtccggcgtattaaaacacgatacattgttattagtacatttattaagcgctagattctgtgcgttgtt
gatttacagacaattgttgtacgtattttaataattcattaaatttataatctttagggtggtatgttagagcga
aaatcaaatgattttcagcgtctttatatctgaatttaaatattaaatcctcaatagatttgtaaaataggtttc
gattagtttcaaacaagggttgtttttccgaaccgatggctggactatctaatggattttcgctcaacgccacaa
aacttgccaaatcttgtagcagcaatctagctttgtcgatattcgtttgtgttttgttttgtaataaaggttcga
cgtcgttcaaaatattatgcgcttttgtatttctttcatcactgtcgttagtgtacaattgactcgacgtaaaca
cgttaaatagagcttggacatatttaacatcgggcgtgttagctttattaggccgattatcgtcgtcgtcccaac
cctcgtcgttagaagttgcttccgaagacgattttgccatagccacacgacgcctattaattgtgtcggctaaca
cgtccgcgatcaaatttgtagttgagctttttggaattatttctgattgcgggcgtttttgggcgggtttcaatc
taactgtgcccgattttaattcagacaacacgttagaaagcgatggtgcaggcggtggtaacatttcagacggca
aatctactaatggcggcggtggtggagctgatgataaatctaccatcggtggaggcgcaggcggggctggcggcg
gaggcggaggcggaggtggtggcggtgatgcagacggcggtttaggctcaaatgtctctttaggcaacacagtcg
gcacctcaactattgtactggtttcgggcgccgtttttggtttgaccggtctgagacgagtgcgatttttttcgt
ttctaatagcttccaacaattgttgtctgtcgtctaaaggtgcagcgggttgaggttccgtcggcattggtggag
cgggcggcaattcagacatcgatggtggtggtggtggtggaggcgctggaatgttaggcacgggagaaggtggtg
gcggcggtgccgccggtataatttgttctggtttagtttgttcgcgcacgattgtgggcaccggcgcaggcgccg
ctggctgcacaacggaaggtcgtctgcttcgaggcagcgcttggggtggtggcaattcaatattataattggaat
acaaatcgtaaaaatctgctataagcattgtaatttcgctatcgtttaccgtgccgatatttaacaaccgctcaa
tgtaagcaattgtattgtaaagagattgtctcaagctcggaacgctgcgctcggtcgttcggctgcggcgagcgg
tatcagctcactcaaaggcggtaatacggttatccacagaatcaggggataacgcaggaaagaacatgtgagcaa
aaggccagcaaaaggccaggaaccgtaaaaaggccgcgttgctggcgtttttccataggctccgcccccctgacg
agcatcacaaaaatcgacgctcaagtcagaggtggcgaaacccgacaggactataaagataccaggcgtttcccc

ctggaagctccctcgtgcgctctcctgttccgaccctgccgcttaccggatacctgtccgcctttctcccttcgg
gaagcgtggcgctttctcaatgctcacgctgtaggtatctcagttcggtgtaggtcgttcgctccaagctgggct
gtgtgcacgaaccccccgttcagcccgaccgctgcgccttatccggtaactatcgtcttgagtccaacccggtaa
gacacgacttatcgccactggcagcagccactggtaacaggattagcagagcgaggtatgtaggcggtgctacag
agttcttgaagtggtggcctaactacggctacactagaaggacagtatttggtatctgcgctctgctgaagccag
ttaccttcggaaaaagagttggtagctcttgatccggcaaacaaaccaccgctggtagcggtggtttttttgttt
gcaagcagcagattacgcgcagaaaaaaaggatctcaagaagatcctttgttaccaatgcttaatcagtgaggca
cctatctcagcgatctgtctatttcgttcatccatagttgcctgactccccgtcgtgtagataactacgatacgg
gagggcttaccatctggccccagtgctgcaatgataccgcgagacccacgctcaccggctccagatttatcagca
ataaaccagccagccggaagggccgagcgcagaagtggtcctgcaactttatccgcctccatccagtctattaat
tgttgccgggaagctagagtaagtagttcgccagttaatagtttgcgcaacgttgttgccattgctacaggcatc
gtggtgtcacgctcgtcgtttggtatggcttcattcagctccggttcccaacgatcaaggcgagttacatgatcc
cccatgttgtgcaaaaaagcggttagctccttcggtcctccgatcgttgtcagaagtaagttggccgcagtgtta
tcactcatggttatggcagcactgcataattctcttactgtcatgccatccgtaagatgcttttctgtgactggt
gagtactcaaccaagtcattctgagaatagtgtatgcggcgaccgagttgctcttgcccggcgtcaatacgggat
aataccgcgccacatagcagaactttaaaagtgctcatcattggaaaacgttcttcggggcgaaaactctcaagg
atcttaccgctgttgagatccagttcgatgtaacccactcgtgcacccaactgatcttcagcatcttttactttc
accagcgtttctgggtgagcaaaaacaggaaggcaaaatgccgcaaaaaagggaataagggcgacacggaaatgt
tgaatactcatactcttcctttttcaatattattgaagcatttatcagggttattgtctcatgtccgcgtttc
ctgcatcttttaatcaaatcccaagatgtgtataaaccaccaaactgccaaaaaatgaaaactgtcgacaagctc
tgtccgtttgctggcaactgcaagggtctcaatcctatttgtaattattgaataataaaacaattataaatgtca
aatttgtttttattaacgatacaaaccaaacgcaacaagaacatttgtagtattatctataattgaaaacgcgt
agttataatcgctgaggtaatatttaaaatcattttcaaatgattcacagttaatttgcgacaatataattttat
tttcacataaactagacgccttgtcgtcttcttcttcgtattccttctctttttcattttctcttcataaaaat
taacatagttattatcgtatccatatatgtatctatcgtatagagtaaattttttgttgtcataaatatatatgt
ctttttttaatggggtgtatagtaccgctgcgcatagtttttctgtaatttacaacagtgctattttctggtagtt
cttcggagtgtgttgctttaattattaaatttatataatcaatgaatttgggatcgtcggttttgtacaatatgt
tgccggcatagtacgcagcttcttctagttcaattacaccatttttttagcagcaccggattaacataactttcca
aaatgttgtacgaaccgttaaacaaaaacagttcacctcccttttctatactattgtctgcgagcagttgtttgt
tgttaaaaataacagccattgtaatgagacgcacaaactaatatcacaaactggaaatgtctatcaatatatagt
tgctctagttattaatagtaatcaattacggggtcattagttcatagcccatatatggagttccgcgttacataa
cttacggtaaatggcccgcctggctgaccgcccaacgacccccgcccattgacgtcaataatgacgtatgttccc
atagtaacgccaatagggactttccattgacgtcaatgggtggactatttacggtaaactgcccacttggcagta
catcaagtgtatcatatgccaagtacgccccctattgacgtcaatgacggtaaatggcccgcctggcattatgcc
cagtacatgaccttatgggactttcctacttggcagtacatctacgtattagtcatcgctattaccatgcatggt
cgaggtgagccccacgttctgcttcactctccccatctcccccccctccccacccccaattttgtatttattat
tttttaattattttgtgcagcgatgggggcgggggggggggggggcgcgcgccaggcggggcggggcggggcga
ggggcggggcggggcgaggcggagaggtgcggcggcagccaatcagagcggcgcgctccgaaagtttccttttat
ggcgaggcggcggcggcggcggccctataaaaagcgaagcgcgcggcgggcgggagtcgctgcgacgctgccttc
gccccgtgccccgctccgccgccgcctcgcgccgcccgccccggctctgactgaccgcgttactcccacaggtga
gcgggcgggacggcccttctccttcgggctgtaattagcgcttggtttaatgacggcttgtttcttttctgtggc
tgcgtgaaagccttgaggggctccgggagggccctttgtgcggggggagcggctcggggctgtccgcggggggac
ggctgccttcgggggggacggggcagggcggggttcggcttctggcgtgtgaccggcggctctagagcctctgct
aaccatgttcatgccttcttctttttcctacagctcctgggcaacgtgctggttattgtgctgtctcatcatttt
ggcaaagaattggatcggaccgaaattaatacgactcactata

SEQ ID NO10 pTriEx-1-CHRNE
ggggaattgtgagcggataacaattccccggagttaatccggacctttaattcaacccaacacaatatattata
gttaaataagaattattatcaaatcatttgtatattaattaaaatactatactgtaaattacattttatttacaa
tcaaaggagatataccatggcaagggctccgcttggggtcctgctcctcttggggcttctcggcaggggtgtggg
gaagaacgaggaactgcgtctttatcaccatctcttcaacaactatgacccaggaagccggccagtgcgggagcc
tgaggatactgtcaccatcagcctcaaggtcaccctgacgaatctcatctcactgaatgaaaaagaggagactct
caccactagcgtctggattggaatcgattggcaggattaccgactcaactacagcaaggacgactttgggggtat
agaaaccctgcgagtcccttcagaactcgtgtggctgccagagattgtgctggaaaacaatattgatggccagtt
cggagtggcctacgacgccaacgtgctcgtctacgagggcggctccgtgacgtggctgcctccggccatctaccg
cagcgtctgcgcagtggaggtcacctacttccccttcgattggcagaactgttcgcttattttccgctctcagac
gtacaatgccgaagaggtggagttcacttttgccgtagacaacgacggcaagaccatcaacaagatcgacatcga
cacagaggcctatactgagaacggcgagtggccatcgacttctgcccggggtgatccgccgccaccacggtgg
cgccaccgacggcccaggggagactgacgtcatctactcgctcatcatccgccggaagccgctcttctacgtcat

taacatcatcgtgccctgtgtgctcatctcgggcctggtgctgctcgcctacttcctgccggcgcaggccggcgg
ccagaaatgcacggtctccatcaacgtcctgctcgcccagaccgtcttcttgttcctcattgcccagaaaatccc
agagacttctctgagcgtgccgctcctgggcaggttccttatttcgtcatggtggtcgccacgctcattgtcat
gaattgcgtcatcgtgctcaacgtgtcccagcggacgcccaccacccacgccatgtccccgcggctgcgccacgt
tctcctggagctgctgccgcgcctcctgggctccccgccgccgcccgaggcccccgggccgcctcgcccccaag
gcgggcgtcgtcggtgggcttattgctccgcgcggaggagctgatactgaaaaagccacggagcgagctcgtgtt
tgagggcagaggcaccggcaggggacctggacggctgccttctgccagagcctgggcgccgccgcccccgaggt
ccgctgctgtgtggatgccgtgaacttcgtggccgagagcacgagagatcaggaggccaccggcgaggaagtgtc
cgactgggtgcgcatggggaatgcccttgacaacatctgcttctgggccgctctggtgctcttcagcgtgggctc
cagcctcatcttcctcggggcctacttcaaccgagtgcctgatctcccctacgcgccgtgtatccagccttaact
cgagcaccaccatcaccatcaccatcactaagtgattaacctcaggtgcaggctgcctatcagaaggtggtggct
ggtgtggccaatgccctggctcacaaataccactgagatcgatctttttccctctgccaaaaattatggggacat
catgaagccccttgagcatctgacttctggctaataaaggaaatttattttcattgcaatagtgtgttggaattt
tttgtgtctctcactcggaaggacatatgggagggcaaatcatttaaaacatcagaatgagtatttggtttagag
tttggcaacatatgcccatatgtaactagcataaccccttggggcctctaaacgggtcttgaggggttttttgct
gaaagcatgcggaggaaattctccttgaagtttccctggtgttcaaagtaaaggagtttgcaccagacgcacctc
tgttcactggtccggcgtattaaaacacgatacattgttattagtacatttattaagcgctagattctgtgcgtt
gttgatttacagacaattgttgtacgtattttaataattcattaaatttataatctttagggtggtatgttagag
cgaaaatcaaatgattttcagcgtctttatatctgaatttaaatattaaatcctcaatagatttgtaaaataggt
ttcgattagtttcaaacaaggttgttttccgaaccgatggctggactatctaatggattttcgctcaacgcca
caaaacttgccaaatcttgtagcagcaatctagctttgtcgatattcgtttgtgttttgttttgtaataaaggtt
cgacgtcgttcaaaatattatgcgcttttgtatttctttcatcactgtcgttagtgtacaattgactcgacgtaa
acacgttaaatagagcttggacatatttaacatcggcgtgttagctttattaggccgattatcgtcgtcgtccc
aaccctcgtcgttagaagttgcttccgaagacgattttgccatagccacacgacgcctattaattgtgtcggcta
acacgtccgcgatcaaatttgtagttgagctttttggaattatttctgattgcgggcgtttttgggcgggtttca
atctaactgtgcccgattttaattcagacaacacgttagaaagcgatggtgcaggcggtggtaacatttcagacg
gcaaatctactaatggcggcggtggtggagctgatgataaatctaccatcggtggaggcgcaggcggggctggcg
gcggaggcggaggcggaggtggtggcggtgatgcagacggcggtttaggctcaaatgtctctttaggcaacacag
tcggcacctcaactattgtactggtttcgggcgccgtttttggtttgaccggtctgagacgagtgcgattttttt
cgtttctaatagcttccaacaattgttgtctgtcgtctaaaggtgcagcgggttgaggttccgtcggcattggtg
gagcgggcggcaattcagacatcgatggtggtggtggtggaggcgctggaatgttaggcacgggagaaggtg
gtggcggcggtgccgccggtataatttgttctggtttagtttgttcgcgcacgattgtgggcaccggcgcaggcg
ccgctggctgcacaacggaaggtcgtctgcttcgaggcagcgcttggggtggtggcaattcaatattataattgg
aatacaaatcgtaaaaatctgctataagcattgtaatttcgctatcgtttaccgtgccgatatttaacaaccgct
caatgtaagcaattgtattgtaaagagattgtctcaagctcggaacgctgcgctcggtcgttcggctgcggcgag
cggtatcagctcactcaaaggcggtaatacggttatccacagaatcaggggataacgcaggaaagaacatgtgag
caaaaggccagcaaaaggccaggaaccgtaaaaaggccgcgttgctggcgtttttccataggctccgcccccctg
acgagcatcacaaaaatcgacgctcaagtcagaggtggcgaaacccgacaggactataaagataccaggcgtttc
cccctggaagctccctcgtgcgctctcctgttccgaccctgccgcttaccggatacctgtccgcctttctccctt
cgggaagcgtggcgctttctcaatgctcacgctgtaggtatctcagttcggtgtaggtcgttcgctccaagctgg
gctgtgtgcacgaaccccccgttcagcccgaccgctgcgccttatccggtaactatcgtcttgagtccaacccgg
taagacacgacttatcgccactggcagcagccactggtaacaggattagcagagcgaggtatgtaggcggtgcta
cagagttcttgaagtggtggcctaactacggctacactagaaggacagtatttggtatctgcgctctgctgaagc
cagttaccttcggaaaaagagttggtagctcttgatccggcaaacaaaccaccgctggtagcggtggtttttttg
tttgcaagcagcagattacgcgcagaaaaaaaggatctcaagaagatcctttgttaccaatgcttaatcagtgag
gcacctatctcagcgatctgtctatttcgttcatccatagttgcctgactccccgtcgtgtagataactacgata
cgggagggcttaccatctggccccagtgctgcaatgataccgcgagacccacgctcaccggctccagatttatca
gcaataaaccagccagccggaagggccgagcgcagaagtggtcctgcaactttatccgcctccatccagtctatt
aattgttgccgggaagctagagtaagtagttcgccagttaatagtttgcgcaacgttgttgccattgctacaggc
atcgtggtgtcacgctcgtcgtttggtatggcttcattcagctccggttcccaacgatcaaggcgagttacatga
tcccccatgttgtgcaaaaaagcggttagctccttcggtcctccgatcgttgtcagaagtaagttggccgcagtg
ttatcactcatggttatggcagcactgcataattctcttactgtcatgccatccgtaagatgcttttctgtgact
ggtgagtactcaaccaagtcattctgagaatagtgtatgcggcgaccgagttgctcttgcccggcgtcaatacgg
gataataccgcgccacatagcagaactttaaaagtgctcatcattggaaaacgttcttcggggcgaaaactctca
aggatcttaccgctgttgagatccagttcgatgtaacccactcgtgcacccaactgatcttcagcatcttttact
ttcaccagcgtttctgggtgagcaaaaacaggaaggcaaaatgccgcaaaaaagggaataagggcgacacggaaa
tgttgaatactcatactcttcctttttcaatattattgaagcatttatcagggttattgtctcatgtccgcgcgt
ttcctgcatcttttaatcaaatcccaagatgtgtataaaccaccaaactgccaaaaaatgaaaactgtcgacaag
ctctgtccgtttgctggcaactgcaagggtctcaatcctatttgtaattattgaataataaaacaattataaatg

tcaaatttgtttttattaacgatacaaaccaaacgcaacaagaacatttgtagtattatctataattgaaaacg
cgtagttataatcgctgaggtaatatttaaaatcattttcaaatgattcacagttaatttgcgacaatataattt
tattttcacataaactagacgccttgtcgtcttcttcttcgtattccttctctttttcatttttctcttcataaa
aattaacatagttattatcgtatccatatatgtatctatcgtatagagtaaattttttgttgtcataaatatata
tgtctttttaatggggtgtatagtaccgctgcgcatagtttttctgtaatttacaacagtgctattttctggta
gttcttcggagtgtgttgctttaattattaaatttatataatcaatgaatttgggatcgtcggttttgtacaata
tgttgccggcatagtacgcagcttcttctagttcaattacaccatttttttagcagcaccggattaacataacttt
ccaaaatgttgtacgaaccgttaaacaaaaacagttcacctcccttttctatactattgtctgcgagcagttgtt
tgttgttaaaaataacagccattgtaatgagacgcacaaactaatatcacaaactggaaatgtctatcaatatat
agttgctctagttattaatagtaatcaattacggggtcattagttcatagcccatatatggagttccgcgttaca
taacttacggtaaatggcccgcctggctgaccgcccaacgacccccgcccattgacgtcaataatgacgtatgtt
cccatagtaacgccaatagggactttccattgacgtcaatgggtggactatttacggtaaactgcccacttggca
gtacatcaagtgtatcatatgccaagtacgccccctattgacgtcaatgacggtaaatggcccgcctggcattat
gcccagtacatgaccttatgggactttcctacttggcagtacatctacgtattagtcatcgctattaccatgcat
ggtcgaggtgagccccacgttctgcttcactctccccatctcccccccctccccacccccaattttgtatttatt
tattttttaattattttgtgcagcgatgggggcgggggggggggggggcgcgcgccaggcggggcggggcgggg
cgaggggcggggcggggcgaggcggagaggtgcggcggcagccaatcagagcggcgcgctccgaaagtttcctttt
tatggcgaggcggcggcggcggcggccctataaaaagcgaagcgcgcggcgggcgggagtcgctgcgacgctgcc
ttcgccccgtgccccgctccgccgccgcctcgcgccgcccgccccggctctgactgaccgcgttactcccacagg
tgagcgggcgggacggcccttctccttcgggctgtaattagcgcttggtttaatgacggcttgtttcttttctgt
ggctgcgtgaaagccttgaggggctccgggagggccctttgtgcggggggagcggctcggggctgtccgcgggg
gacggctgccttcgggggggacggggcagggcggggttcggcttctggcgtgtgaccggcggctctagagcctct
gctaaccatgttcatgccttcttctttttcctacagctcctgggcaacgtgctggttattgtgctgtctcatcat
tttggcaaagaattggatcggaccgaaattaatacgactcactata

SEQ ID NO11 pTriEx-1-CHRNG
ggggaattgtgagcggataacaattccccggagttaatccgggacctttaattcaacccaacacaatatattata
gttaaataagaattattatcaaatcatttgtatattaattaaaatactatactgtaaattacattttatttacaa
tcaaaggagatataccatgcatggggccaggggccgctgctcctcctgctgctgctggctgtctgcctggggc
ccagggccggaaccaggaggagcgcctgctcgcagacctgatgcaaaactacgaccccaacctgcggcccgcgga
acgagactcggatgtggtcaatgtcagcctgaagctaaccctcaccaacctcatctccctgaacgagcgagagga
agccctcaccaccaatgtctggatagagatgcagtggtgcgactatcgcctgcgctgggatccgcgagactacga
aggcctgtgggtgctgagggtgccgtccaccatggtgtggcggccggatatcgtgctggagaataacgtggacgg
tgtcttcgaggtggccctctactgcaatgtgctcgtgtcccctgacggctgtatctactggctgccgcctgccat
cttccgttccgcctgctctatctcagtcacctacttccccttcgactggcagaactgctcccttatcttccagtc
ccagacttacagcaccaatgagattgatctgcagctgagtcaggaagatggccagaccatcgagtggattttcat
tgaccctgaggccttcacagagaatggggagtgggccatccagcaccgaccagccaagatgctcctggacccagc
ggcgccagcccaggaagcaggccaccagaaggtggtgttctacctgctcatccagcgcaagcccctcttctacgt
catcaacatcatcgcccccctgtgtgctcatctcctctgtcgccatcctcatccacttccttcctgccaaggctgg
gggccagaagtgtaccgtcgccatcaacgtgctcctggcccagactgtcttcctcttccttgtggccaagaaggt
gcctgaaacctcccaggcggtgccactcatcagcaagtacctgaccttcctcctggtggtgaccatcctcattgt
cgtgaatgctgtggttgtgctcaatgtctccttgcggtctccacacacacactccatggcccgaggggtccgcaa
ggtgttcctgaggctcttgccccagctgctgaggatgcacgttcgcccgctggccccggcagctgtgcaggacac
ccagtcccggctacagaatggctcctcgggatggtcgatcacaactggggaggaggtggccctctgcctgcctcg
cagtgaactcctcttccagcagtggcagcggcaaggctggtggcggcagcgctggagaagctagagaaaggccc
ggagttagggctgagccagttctgtggcagcctgaagcaggctgccccagccatccaggcctgtgtggaagcctg
caacctcattgcctgtgcccggcaccagcagagtcactttgacaatggaatgaggagtggttcctggtgggccg
agtgctggaccgtgtctgcttcctggccatgctctcgctcttcatctgtggcacagctggcatcttcctcatggc
ccactacaaccgggtgccggccctgccattccctggagatccacgcccctacctgccctcaccagactgactcga
gcaccaccatcaccatcaccatcactaagtgattaacctcaggtgcaggctgcctatcagaaggtggtggctggt
gtggccaatgccctggctcacaaataccactgagatcgatctttttccctctgccaaaaattatggggacatcat
gaagccccttgagcatctgacttctggctaataaaggaaatttattttcattgcaatagtgtgttggaattttt
gtgtctctcactcggaaggacatatgggagggcaaatcatttaaaacatcagaatgagtatttggtttagagttt
ggcaacatatgcccatatgtaactagcataaccccttggggcctctaaacgggtcttgaggggttttttgctgaa
agcatgcggaggaaattctccttgaagtttccctggtgttcaaagtaaaggagtttgcaccagacgcacctctgt
tcactggtccggcgtattaaaacacgatacattgttattagtacatttattaagcgctagattctgtgcgttgtt
gatttacagacaattgttgtacgtattttaataattcattaaatttataatctttagggtggtatgttagagcga
aaatcaaatgatttcagcgtctttatatctgaatttaaatattaaatcctcaatagatttgtaaaataggtttc
gattagtttcaaacaagggttgtttttccgaaccgatggctggactatctaatggattttcgctcaacgccacaa

aacttgccaaatcttgtagcagcaatctagctttgtcgatattcgtttgtgttttgttttgtaataaaggttcga
cgtcgttcaaaatattatgcgcttttgtatttctttcatcactgtcgttagtgtacaattgactcgacgtaaaca
cgttaaatagagcttggacatatttaacatcgggcgtgttagctttattaggccgattatcgtcgtcgtcccaac
cctcgtcgttagaagttgcttccgaagacgatttgccatagccacacgacgcctattaattgtgtcggctaaca
cgtccgcgatcaaatttgtagttgagctttttggaattatttctgattgcgggcgtttttgggcgggtttcaatc
taactgtgcccgattttaattcagacaacacgttagaaagcgatggtgcaggcggtggtaacatttcagacggca
aatctactaatggcggcggtggtggagctgatgataaatctaccatcggtggaggcgcaggcggggctggcggcg
gaggcggaggcggaggtggtggcggtgatgcagacggcggtttaggctcaaatgtctctttaggcaacacagtcg
gcacctcaactattgtactggtttcgggcgccgttttggtttgaccggtctgagacgagtgcgatttttttcgt
ttctaatagcttccaacaattgttgtctgtcgtctaaaggtgcagcgggttgaggttccgtcggcattggtggag
cgggcggcaattcagacatcgatggtggtggtggtggtggaggcgctggaatgttaggcacgggagaaggtggtg
gcggcggtgccgccggtataatttgttctggtttagtttgttcgcgcacgattgtgggcaccggcgcaggcgccg
ctggctgcacaacggaaggtcgtctgcttcgaggcagcgcttggggtggtggcaattcaatattataattggaat
acaaatcgtaaaaatctgctataagcattgtaatttcgctatcgtttaccgtgccgatatttaacaaccgctcaa
tgtaagcaattgtattgtaaagagattgtctcaagctcggaacgctgcgctcggtcgttcggctgcggcgagcgg
tatcagctcactcaaaggcggtaatacggttatccacagaatcaggggataacgcaggaaagaacatgtgagcaa
aaggccagcaaaaggccaggaaccgtaaaaaggccgcgttgctggcgtttttccataggctccgcccccctgacg
agcatcacaaaaatcgacgctcaagtcagaggtggcgaaacccgacaggactataaagataccaggcgtttcccc
ctggaagctccctcgtgcgctctcctgttccgaccctgccgcttaccggatacctgtccgcctttctcccttcgg
gaagcgtggcgctttctcaatgctcacgctgtaggtatctcagttcggtgtaggtcgttcgctccaagctgggct
gtgtgcacgaaccccccgttcagcccgaccgctgcgccttatccggtaactatcgtcttgagtccaacccggtaa
gacacgacttatcgccactggcagcagccactggtaacaggattagcagagcgaggtatgtaggcggtgctacag
agttcttgaagtggtggcctaactacggctacactagaaggacagtatttggtatctgcgctctgctgaagccag
ttaccttcggaaaaagagttggtagctcttgatccggcaaacaaaccaccgctggtagcggtggtttttttgttt
gcaagcagcagattacgcgcagaaaaaaaggatctcaagaagatcctttgttaccaatgcttaatcagtgaggca
cctatctcagcgatctgtctatttcgttcatccatagttgcctgactccccgtcgtgtagataactacgatacgg
gagggcttaccatctggccccagtgctgcaatgataccgcgagacccacgctcaccggctccagatttatcagca
ataaaccagccagccggaagggccgagcgcagaagtggtcctgcaactttatccgcctccatccagtctattaat
tgttgccgggaagctagagtaagtagttcgccagttaatagtttgcgcaacgttgttgccattgctacaggcatc
gtggtgtcacgctcgtcgtttggtatggcttcattcagctccggttcccaacgatcaaggcgagttacatgatcc
cccatgttgtgcaaaaaagcggttagctccttcggtcctccgatcgttgtcagaagtaagttggccgcagtgtta
tcactcatggttatggcagcactgcataattctcttactgtcatgccatccgtaagatgcttttctgtgactggt
gagtactcaaccaagtcattctgagaatagtgtatgcggcgaccgagttgctcttgcccggcgtcaatacgggat
aataccgcgccacatagcagaactttaaaagtgctcatcattggaaaacgttcttcggggcgaaaactctcaagg
atcttaccgctgttgagatccagttcgatgtaacccactcgtgcacccaactgatcttcagcatcttttactttc
accagcgtttctgggtgagcaaaaacaggaaggcaaaatgccgcaaaaaagggaataagggcgacacggaaatgt
tgaatactcatactcttcctttttcaatattattgaagcatttatcagggttattgtctcatgtccgcgcgtttc
ctgcatcttttaatcaaatcccaagatgtgtataaaccaccaaactgccaaaaaatgaaaactgtcgacaagctc
tgtccgtttgctggcaactgcaagggtctcaatcctatttgtaattattgaataataaaacaattataaatgtca
aatttgtttttattaacgatacaaaccaaacgcaacaagaacatttgtagtattatctataattgaaaacgcgt
agttataatcgctgaggtaatatttaaaatcattttcaaatgattcacagttaatttgcgacaatataatttat
tttcacataaactagacgccttgtcgtcttcttcttcgtattccttctctttttcatttttctcttcataaaaat
taacatagttattatcgtatccatatatgtatctatcgtatagagtaaatttttgttgtcataaatatatatgt
ctttttttaatggggtgtatagtaccgctgcgcatagtttttctgtaatttacaacagtgctattttctggtagtt
cttcggagtgtgttgctttaattattaaatttatataatcaatgaatttgggatcgtcggttttgtacaatatgt
tgccggcatagtacgcagcttcttctagttcaattacaccatttttttagcagcaccggattaacataactttcca
aaatgttgtacgaaccgttaaacaaaaacagttcacctcccttttctatactattgtctgcgagcagttgtttgt
tgttaaaaataacagccattgtaatgagacgcacaaactaatatcacaaactggaaatgtctatcaatatatagt
tgctctagttattaatagtaatcaattacggggtcattagttcatagcccatatatggagttccgcgttacataa
cttacggtaaatggcccgcctggctgaccgcccaacgacccccgcccattgacgtcaataatgacgtatgttccc
atagtaacgccaatagggactttccattgacgtcaatgggtggactatttacggtaaactgcccacttggcagta
catcaagtgtatcatatgccaagtacgccccctattgacgtcaatgacggtaaatggcccgcctggcattatgcc
cagtacatgaccttatgggactttcctacttggcagtacatctacgtattagtcatcgctattaccatgcatggt
cgaggtgagccccacgttctgcttcactctccccatctcccccccctccccacccccaattttgtatttatttat
ttttttaattattttgtgcagcgatgggggcgggggggggggggggcgcgcgccaggcggggcggggcggggcga
ggggcggggcggggcgaggcggagaggtgcggcggcagccaatcagagcggcgcgctccgaaagtttccttttat
ggcgaggcggcggcggcggcggccctataaaaagcgaagcgcgcggcgggcgggagtcgctgcgacgctgccttc
gccccgtgccccgctccgccgccgcctcgcgccgcccgccccggctctgactgaccgcgttactcccacaggtga
gcgggcgggacggcccttctccttcgggctgtaattagcgcttggtttaatgacggcttgtttcttttctgtggc

tgcgtgaaagccttgaggggctccgggagggccctttgtgcggggggagcggctcggggctgtccgcggggggac
ggctgccttcgggggggacggggcagggcggggttcggcttctggcgtgtgaccggcggctctagagcctctgct
aaccatgttcatgccttcttctttttcctacagctcctgggcaacgtgctggttattgtgctgtctcatcatttt
ggcaaagaattggatcggaccgaaattaatacgactcactata

SEQ ID NO12 pTriEx-1-RAPSyn
ggggaattgtgagcggataacaattccccggagttaatccgggacctttaattcaacccaacacaatatattata
gttaaataagaattattatcaaatcatttgtatattaattaaaatactatactgtaaattacattttatttacaa
tcaaaggagatataccatggggcaggaccagaccaagcagcagatcgagaaggggctccagctgtaccagtccaa
ccagacagagaaggcattgcaggtgtggacaaaggtgctggagaagagctcggacctcatgggcgcttccgcgt
gctgggctgcctggtcacagcccactcggagatgggccgctacaaggagatgctgaagttcgctgtggtccagat
cgacacggcccgggagctggaggatgccgacttcctcctggagagctacctgaacctggcacgcagcaacgagaa
gctgtgcgagtttcacaagaccatctcctactgcaagacctgccttgggctgcctggtaccagggcaggtgccca
gctcggaggccaggtcagcctgagcatgggcaatgccttcctgggcctcagcgtcttccagaaggccctggagag
cttcgagaaggccctgcgctatgcccacaacaatgatgacgccatgctcgagtgccgcgtgtgctgcagcctggg
cagcttctatgcccaggtcaaggactacgagaaagccctgttcttcccctgcaaggcggcagagcttgtcaacaa
ctatggcaaaggctggagcctgaagtaccgggccatgagccagtaccacatggccgtggcctatcgcctgctggg
ccgcctgggcagtgccatggagtgttgtgaggagtctatgaagatcgcgctgcagcacggggaccggccactgca
ggcgctctgcctgctctgcttcgctgacatccaccggagccgtggggacctggagacagccttccccaggtacga
ctccgccatgagcatcatgaccgagatcggaaaccgcctggggcaggtgcaggcgctgctgggtgtggccaagtg
ctgggtggccaggaaggcgctggacaaggctctggatgccatcgagagagcccaggatctggccgaggaggtggg
gaacaagctgagccagctcaagctgcactgtctgagcgagagcatttaccgcagcaaaggcctgcagcgggaact
gcgggcgcacgttgtgaggttccacgagtgcgtggaggagacggagctctactgcggcctgtgcggcgagtccat
aggcgagaagaacagccggctgcaggccctaccttgctcccacatcttccacctcaggtgcctgcagaacaacgg
gacccggagctgtcccaactgccgccgctcatccatgaagcctggctttgtataactcgagcaccaccatcacca
tcaccatcactaagtgattaacctcaggtgcaggctgcctatcagaaggtggtggctggtgtggccaatgccctg
gctcacaaataccactgagatcgatctttttccctctgccaaaaattatggggacatcatgaagccccttgagca
tctgacttctggctaataaaggaaatttattttcattgcaatagtgtgttggaattttttgtgtctctcactcgg
aaggacatatgggagggcaaatcatttaaaacatcagaatgagtatttggtttagagtttggcaacatatgccca
tatgtaactagcataaccccttggggcctctaaacgggtcttgaggggttttttgctgaaagcatgcggaggaaa
ttctccttgaagtttccctggtgttcaaagtaaaggagtttgcaccagacgcacctctgttcactggtccggcgt
attaaaacacgatacattgttattagtacatttattaagcgctagattctgtgcgttgttgatttacagacaatt
gttgtacgtattttaataattcattaaatttataatctttagggtggtatgttagagcgaaaatcaaatgatttt
cagcgtctttatatctgaatttaaatattaaatcctcaatagatttgtaaaataggtttcgattagtttcaaaca
agggttgtttttccgaaccgatggctggactatctaatggattttcgctcaacgccacaaaacttgccaaatctt
gtagcagcaatctagctttgtcgatattcgtttgtgttttgttttgtaataaaggttcgacgtcgttcaaaatat
tatgcgcttttgtatttctttcatcactgtcgttagtgtacaattgactcgacgtaaacacgttaaatagagctt
ggacatatttaacatcgggcgtgttagctttattaggccgattatcgtcgtcgtcccaaccctcgtcgttagaag
ttgcttccgaagacgattttgccatagccacacgacgcctattaattgtgtcggctaacacgtccgcgatcaaat
ttgtagttgagctttttggaattatttctgattgcgggcgtttttgggcgggtttcaatctaactgtgcccgatt
ttaattcagacaacacgttagaaagcgatggtgcaggcggtggtaacatttcagacggcaaatctactaatggcg
gcggtggtggagctgatgataaatctaccatcggtggaggcgcaggcggggctggcggcggaggcggaggcggag
gtggtggcggtgatgcagacggcggtttaggctcaaatgtctctttaggcaacacagtcggcacctcaactattg
tactggtttcgggcgccgtttttggtttgaccggtctgagacgagtgcgatttttttcgtttctaatagcttcca
acaattgttgtctgtcgtctaaaggtgcagcgggttgaggttccgtcggcattggtggagcgggcggcaattcag
acatcgatggtggtggtggtggtggaggcgctggaatgttaggcacgggagaaggtggtggcggcggtgccgccg
gtataatttgttctggtttagtttgttcgcgcacgattgtgggcaccggcgcaggcgccgctggctgcacaacgg
aaggtcgtctgcttcgaggcagcgcttggggtggtggcaattcaatattataattggaatacaaatcgtaaaaat
ctgctataagcattgtaatttcgctatcgtttaccgtgccgatatttaacaaccgctcaatgtaagcaattgtat
tgtaaagagattgtctcaagctcggaacgctgcgctcggtcgttcggctgcggcgagcggtatcagctcactcaa
aggcggtaatacggttatccacagaatcaggggataacgcaggaaagaacatgtgagcaaaaggccagcaaaagg
ccaggaaccgtaaaaaggccgcgttgctggcgtttttccataggctccgcccccctgacgagcatcacaaaaatc
gacgctcaagtcagaggtggcgaaacccgacaggactataaagataccaggcgtttccccctggaagctccctcg
tgcgctctcctgttccgaccctgccgcttaccggatacctgtccgcctttctcccttcgggaagcgtggcgcttt
ctcaatgctcacgctgtaggtatctcagttcggtgtaggtcgttcgctccaagctgggctgtgtgcacgaacccc
ccgttcagcccgaccgctgcgccttatccggtaactatcgtcttgagtccaacccggtaagacacgacttatcgc
cactggcagcagccactggtaacaggattagcagagcgaggtatgtaggcggtgctacagagttcttgaagtggt
ggcctaactacggctacactagaaggacagtatttggtatctgcgctctgctgaagccagttaccttcggaaaaa
gagttggtagctcttgatccggcaaacaaaccaccgctggtagcggtggtttttttgtttgcaagcagcagatta

cgcgcagaaaaaaaggatctcaagaagatcctttgttaccaatgcttaatcagtgaggcacctatctcagcgatc
tgtctatttcgttcatccatagttgcctgactccccgtcgtgtagataactacgatacgggagggcttaccatct
ggccccagtgctgcaatgataccgcgagacccacgctcaccggctccagatttatcagcaataaaccagccagcc
ggaagggccgagcgcagaagtggtcctgcaactttatccgcctccatccagtctattaattgttgccgggaagct
agagtaagtagttcgccagttaatagtttgcgcaacgttgttgccattgctacaggcatcgtggtgtcacgctcg
tcgtttggtatggcttcattcagctccggttcccaacgatcaaggcgagttacatgatcccccatgttgtgcaaa
aaagcggttagctccttcggtcctccgatcgttgtcagaagtaagttggccgcagtgttatcactcatggttatg
gcagcactgcataattctcttactgtcatgccatccgtaagatgcttttctgtgactggtgagtactcaaccaag
tcattctgagaatagtgtatgcggcgaccgagttgctcttgcccggcgtcaatacgggataataccgcgccacat
agcagaactttaaaagtgctcatcattggaaaacgttcttcggggcgaaaactctcaaggatcttaccgctgttg
agatccagttcgatgtaacccactcgtgcacccaactgatcttcagcatcttttactttcaccagcgtttctggg
tgagcaaaaacaggaaggcaaaatgccgcaaaaaagggaataagggcgacacggaaatgttgaatactcatactc
ttcctttttcaatattattgaagcatttatcagggttattgtctcatgtccgcgcgtttcctgcatcttttaatc
aaatcccaagatgtgtataaaccaccaaactgccaaaaaatgaaaactgtcgacaagctctgtccgtttgctggc
aactgcaagggtctcaatcctatttgtaattattgaataataaaacaattataaatgtcaaatttgttttttatt
aacgatacaaaccaaacgcaacaagaacatttgtagtattatctataattgaaaacgcgtagttataatcgctga
ggtaatatttaaaatcattttcaaatgattcacagttaatttgcgacaatataattttattttcacataaactag
acgccttgtcgtcttcttcttcgtattccttctctttttcatttttctcttcataaaaattaacatagttattat
cgtatccatatatgtatctatcgtatagagtaaattttttgttgtcataaatatatatgtcttttttaatggggt
gtatagtaccgctgcgcatagtttttctgtaatttacaacagtgctattttctggtagttcttcggagtgtgttg
ctttaattattaaatttatataatcaatgaatttgggatcgtcggttttgtacaatatgttgccggcatagtacg
cagcttcttctagttcaattacaccatttttagcagcaccggattaacataactttccaaaatgttgtacgaac
cgttaaacaaaaacagttcacctcccttttctatactattgtctgcgagcagttgtttgttgttaaaaataacag
ccattgtaatgagacgcacaaactaatatcacaaactggaaatgtctatcaatatatagttgctctagttattaa
tagtaatcaattacggggtcattagttcatagcccatatatggagttccgcgttacataacttacggtaaatggc
ccgcctggctgaccgcccaacgacccccgcccattgacgtcaataatgacgtatgttcccatagtaacgccaata
gggactttccattgacgtcaatgggtggactatttacggtaaactgcccacttggcagtacatcaagtgtatcat
atgccaagtacgccccctattgacgtcaatgacggtaaatggcccgcctggcattatgcccagtacatgacctta
tgggactttcctacttggcagtacatctacgtattagtcatcgctattaccatgcatggtcgaggtgagccccac
gttctgcttcactctccccatctcccccccctccccacccccaattttgtatttatttattttttaattattttg
tgcagcgatgggggcgggggggggggggggcgcgcgccaggcggggcggggcggggcgaggggcggggcggggc
gaggcggagaggtgcggcggcagccaatcagagcggcgcgctccgaaagtttccttttatggcgaggcggcggcg
gcggcggccctataaaaagcgaagcgcgcggcgggcgggagtcgctgcgacgctgccttcgccccgtgccccgct
ccgccgccgcctcgcgccgcccgccccggctctgactgaccgcgttactcccacaggtgagcgggcgggacggcc
cttctccttcgggctgtaattagcgcttggtttaatgacggcttgtttcttttctgtggctgcgtgaaagccttg
aggggctccgggagggccctttgtgcggggggagcggctcggggctgtccgcggggggacggctgccttcggggg
ggacggggcagggcggggttcggcttctggcgtgtgaccggcggctctagagcctctgctaaccatgttcatgcc
ttcttctttttcctacagctcctgggcaacgtgctggttattgtgctgtctcatcattttggcaaagaattggat
cggaccgaaattaatacgactcactata

SEQ ID NO13 >MuSK
MRELVNIPLVHILTLVAFSGTEKLPKAPVITTPLETVDALVEEVATFMCAVESYPQPEISWTRNKILIKLFDTRY
SIRENGQLLTILSVEDSDDGIYCCTANNGVGGAVESCGALQVKMKPKITRPPINVKIIEGLKAVLPCTTMGNPKP
SVSWIKGDSPLRENSRIAVLESGSLRIHNVQKEDAGQYRCVAKNSLGTAYSKVVKLEVEVFARILRAPESHNVTF
GSFVTLHCTATGIPVPTITWIENGNAVSSGSIQESVKDRVIDSRLQLFITKPGLYTCIATNKHGEKFSTAKAAAT
ISIAEWSKPQKDNKGYCAQYRGEVCNAVLAKDALVFLNTSYADPEEAQELLVHTAWNELKVVSPVCRPAAEALLC
NHIFQECSPGVVPTPIPICREYCLAVKELFCAKEWLVMEEKTHRGLYRSEMHLLSVPECSKLPSMHWDPTACARL
PHLAFPPMTSSKPSVDIPNLPSSSSSSFSVSPTYSMTVIISIMSSFAIFVLLTITTLYCCRRRKQWKNKKRESAA
VTLTTLPSELLLDRLHPNPMYQRMPLLLNPKLLSLEYPRNNIEYVRDIGEGAFGRVFQARAPGLLPYEPFTMVAV
KMLKEEASADMQADFQREAALMAEFDNPNIVKLLGVCAVGKPMCLLFEYMAYGDLNEFLRSMSPHTVCSLSHSDL
SMRAQVSSPGPPPLSCAEQLCIARQVAAGMAYLSERKFVHRDLATRNCLVGENMVVKIADFGLSRNIYSADYYKA
NENDAIPIRWMPPESIFYNRYTTESDVWAYGVVLWEIFSYGLQPYYGMAHEEVIYYVRDGNILSCPENCPVELYN
LMRLCWSKLPADRPSFTSIHRILERMCERAEGTVSV

SEQ ID NO14 >pTriEx-1-MuSK
GGGGAATTGTGAGCGGATAACAATTCCCCGGAGTTAATCCGGACCTTTAATTCAACCCAACACAATATATTATA
GTTAAATAAGAATTATTATCAAATCATTTGTATATTAATTAAAATACTATACTGTAAATTACATTTTATTTACAA
TCAAAGGAGATATACCATGAGAGAGCTCGTCAACATTCCACTGGTACATATTCTTACTCTGGTTGCCTTCAGCGG
AACTGAGAAACTTCCAAAAGCTCCTGTCATCACCACTCCTCTTGAAACAGTGGATGCCTTAGTTGAAGAAGTGGC

TACTTTCATGTGTGCAGTGGAATCCTACCCCCAGCCTGAGATTTCCTGGACTAGAAATAAAATTCTCATTAAACT
CTTTGACACCCGGTACAGCATCCGGGAGAATGGGCAGCTCCTCACCATCCTGAGTGTGGAAGACAGTGATGATGG
CATTTACTGCTGCACGGCCAACAATGGTGTGGGAGGAGCTGTGGAGAGTTGTGGAGCCCTGCAAGTGAAGATGAA
ACCTAAAATAACTCGTCCTCCCATAAATGTGAAAATAATAGAGGGATTAAAAGCAGTCCTACCATGTACTACAAT
GGGTAATCCCAAACCATCAGTGTCTTGGATAAAGGGAGACAGCCCACTCAGGGAAAATTCCCGAATTGCAGTTCT
TGAATCTGGGAGCTTGAGGATTCATAACGTACAAAGGAAGATGCAGGACAGTATCGATGTGTGGCAAAAAACAG
CCTCGGGACAGCATATTCCAAAGTGGTGAAGCTGGAAGTTGAGGTTTTTGCCAGGATCCTGCGGGCTCCTGAATC
CCACAATGTCACCTTTGGCTCCTTTGTGACCCTGCACTGTACAGCAACAGGCATTCCTGTCCCCACCATCACCTG
GATTGAAAACGGAAATGCTGTTTCTTCTGGGTCCATTCAAGAGAGTGTGAAAGACCGAGTGATTGACTCAAGACT
GCAGCTGTTTATCACCAAGCCAGGACTCTACACATGCATAGCTACCAATAAGCATGGGGAGAAGTTCAGTACTGC
CAAGGCTGCAGCCACCATCAGCATAGCAGAATGGAGTAAACCACAGAAAGATAACAAAGGCTACTGCGCCCAGTA
CAGAGGGGAGGTGTGTAATGCAGTCCTGGCAAAAGATGCTCTTGTTTTTCTCAACACCTCCTATGCGGACCCTGA
GGAGGCCCAAGAGCTACTGGTCCACACGGCCTGGAATGAACTGAAAGTAGTGAGCCCAGTCTGCCGGCCAGCTGC
TGAGGCTTTGTTGTGTAACCACATCTTCCAGGAGTGCAGTCCTGGAGTAGTGCCTACTCCTATTCCCATTTGCAG
AGAGTACTGCTTGGCAGTAAAGGAGCTCTTCTGCGCAAAAGAATGGCTGGTAATGGAAGAGAAGACCCACAGAGG
ACTCTACAGATCCGAGATGCATTTGCTGTCCGTGCCAGAATGCAGCAAGCTTCCCAGCATGCATTGGGACCCCAC
GGCCTGTGCCAGACTGCCACATCTAGCATTCCCACCAATGACGTCCTCAAAGCCAAGTGTGGACATTCCAAACCT
GCCTTCCTCCTCCTCTTCTTCCTTCTCTGTCTCACCTACATACTCCATGACTGTAATAATCTCCATCATGTCCAG
CTTTGCAATATTTGTGCTTCTTACCATAACTACTCTCTATTGCTGCCGAAGAAGAAAACAATGGAAAAATAAGAA
AAGAGAATCAGCAGCAGTAACCCTCACCACACTGCCTTCTGAGCTCTTACTAGATAGACTTCATCCCAACCCCAT
GTACCAGAGGATGCCGCTCCTTCTGAACCCCAAATTGCTCAGCCTGGAGTATCCAAGGAATAACATTGAATATGT
GAGAGACATCGGAGAGGGAGCGTTTGGAAGGGTGTTTCAAGCAAGGGCACCAGGCTTACTTCCCTATGAACCTTT
CACTATGGTGGCAGTAAAGATGCTCAAAGAAGAAGCCTCGGCAGATATGCAAGCGGACTTTCAGAGGGAGGCAGC
CCTCATGGCAGAATTTGACAACCCTAACATTGTGAAGCTATTAGGAGTGTGTGCTGTCGGGAAGCCAATGTGCCT
GCTCTTTGAATACATGGCCTATGGTGACCTCAATGAGTTCCTCCGCAGCATGTCCCCTCACACCGTGTGCAGCCT
CAGTCACAGTGACTTGTCTATGAGGGCTCAGGTCTCCAGCCCTGGGCCCCCACCCCTCTCCTGTGCTGAGCAGCT
TTGCATTGCCAGGCAGGTGGCAGCTGGCATGGCTTACCTCTCAGAACGTAAGTTTGTTCACCGAGATTTAGCCAC
CAGGAACTGCCTGGTGGGCGAGAACATGGTGGTGAAAATTGCCGACTTTGGCCTCTCCAGGAACATCTACTCAGC
AGACTACTACAAAGCTAATGAAAACGACGCTATCCCTATCCGTTGGATGCCACCAGAGTCCATTTTTTATAACCG
CTACACTACAGAGTCTGATGTGTGGGCCTATGGCGTGGTCCTCTGGGAGATCTTCTCCTATGGCCTGCAGCCCTA
CTATGGGATGGCCCATGAGGAGGTCATTTACTACGTGCGAGATGGCAACATCCTCTCCTGCCCTGAGAACTGCCC
CGTGGAGCTGTACAATCTCATGCGTCTATGTTGGAGCAAGCTGCCTGCAGACAGACCCAGTTTCACCAGTATTCA
CCGAATTCTGGAACGCATGTGTGAGAGGGCAGAGGGAACTGTGAGTGTCTAACTCGAGCACCACCATCACCATCA
CCATCACTAAGTGATTAACCTCAGGTGCAGGCTGCCTATCAGAAGGTGGTGGCTGGTGTGGCCAATGCCCTGGCT
CACAAATACCACTGAGATCGATCTTTTTCCCTCTGCCAAAAATTATGGGGACATCATGAAGCCCCTTGAGCATCT
GACTTCTGGCTAATAAAGGAAATTTATTTTCATTGCAATAGTGTGTTGGAATTTTTTGTGTCTCTCACTCGGAAG
GACATATGGGAGGGCAAATCATTTAAAACATCAGAATGAGTATTTGGTTTAGAGTTTGGCAACATATGCCCATAT
GTAACTAGCATAACCCCTTGGGGCCTCTAAACGGGTCTTGAGGGGTTTTTTGCTGAAAGCATGCGGAGGAAATTC
TCCTTGAAGTTTCCCTGGTGTTCAAAGTAAAGGAGTTTGCACCAGACGCACCTCTGTTCACTGGTCCGGCGTATT
AAAACACGATACATTGTTATTAGTACATTTATTAAGCGCTAGATTCTGTGCGTTGTTGATTTACAGACAATTGTT
GTACGTATTTTAATAATTCATTAAATTTATAATCTTTAGGGTGGTATGTTAGAGCGAAAATCAAATGATTTTCAG
CGTCTTTATATCTGAATTTAAATATTAAATCCTCAATAGATTTGTAAAATAGGTTTCGATTAGTTTCAAACAAGG
GTTGTTTTTCCGAACCGATGGCTGGACTATCTAATGGATTTTCGCTCAACGCCACAAAACTTGCCAAATCTTGTA
GCAGCAATCTAGCTTTGTCGATATTCGTTTGTGTTTTGTTTTGTAATAAAGGTTCGACGTCGTTCAAAATATTAT
GCGCTTTTGTATTTCTTTCATCACTGTCGTTAGTGTACAATTGACTCGACGTAAACACGTTAAATAGAGCTTGGA
CATATTTAACATCGGGCGTGTTAGCTTTATTAGGCCGATTATCGTCGTCGTCCCAACCCTCGTCGTTAGAAGTTG
CTTCCGAAGACGATTTTGCCATAGCCACACGACGCCTATTAATTGTGTCGGCTAACACGTCCGCGATCAAATTTG
TAGTTGAGCTTTTTGGAATTATTTCTGATTGCGGGCGTTTTTGGGCGGGTTTCAATCTAACTGTGCCCGATTTTA
ATTCAGACAACACGTTAGAAAGCGATGGTGCAGGCGGTGGTAACATTTCAGACGGCAAATCTACTAATGGCGGCG
GTGGTGGAGCTGATGATAAATCTACCATCGGTGGAGGCGCAGGCGGGGCTGGCGGCGGAGGCGGAGGCGGAGGTG
GTGGCGGTGATGCAGACGGCGGTTTAGGCTCAAATGTCTCTTTAGGCAACACAGTCGGCACCTCAACTATTGTAC
TGGTTTCGGGCGCCGTTTTTGGTTTGACCGGTCTGAGACGAGTGCGATTTTTTTCGTTTCTAATAGCTTCCAACA
ATTGTTGTCTGTCGTCTAAAGGTGCAGCGGGTTGAGGTTCCGTCGGCATTGGTGGAGCGGGCGGCAATTCAGACA
TCGATGGTGGTGGTGGTGGTGGAGGCGCTGGAATGTTAGGCACGGGAGAAGGTGGTGGCGGCGGTGCCGCCGGTA
TAATTTGTTCTGGTTTAGTTTGTTCGCGCACGATTGTGGGCACCGGCGCAGGCGCCGCTGGCTGCACAACGGAAG
GTCGTCTGCTTCGAGGCAGCGCTTGGGGTGGTGGCAATTCAATATTATAATTGGAATACAAATCGTAAAAATCTG
CTATAAGCATTGTAATTTCGCTATCGTTTACCGTGCCGATATTTAACAACCGCTCAATGTAAGCAATTGTATTGT
AAAGAGATTGTCTCAAGCTCGGAACGCTGCGCTCGGTCGTTCGGCTGCGGCGAGCGGTATCAGCTCACTCAAAGG
CGGTAATACGGTTATCCACAGAATCAGGGGATAACGCAGGAAAGAACATGTGAGCAAAAGGCCAGCAAAAGGCCA

GGAACCGTAAAAAGGCCGCGTTGCTGGCGTTTTTCCATAGGCTCCGCCCCCCTGACGAGCATCACAAAAATCGAC
GCTCAAGTCAGAGGTGGCGAAACCCGACAGGACTATAAAGATACCAGGCGTTTCCCCCTGGAAGCTCCCTCGTGC
GCTCTCCTGTTCCGACCCTGCCGCTTACCGGATACCTGTCCGCCTTTCTCCCTTCGGGAAGCGTGGCGCTTTCTC
AATGCTCACGCTGTAGGTATCTCAGTTCGGTGTAGGTCGTTCGCTCCAAGCTGGGCTGTGTGCACGAACCCCCCG
TTCAGCCCGACCGCTGCGCCTTATCCGGTAACTATCGTCTTGAGTCCAACCCGGTAAGACACGACTTATCGCCAC
TGGCAGCAGCCACTGGTAACAGGATTAGCAGAGCGAGGTATGTAGGCGGTGCTACAGAGTTCTTGAAGTGGTGGC
CTAACTACGGCTACACTAGAAGGACAGTATTTGGTATCTGCGCTCTGCTGAAGCCAGTTACCTTCGGAAAAAGAG
TTGGTAGCTCTTGATCCGGCAAACAAACCACCGCTGGTAGCGGTGGTTTTTTTGTTTGCAAGCAGCAGATTACGC
GCAGAAAAAAAGGATCTCAAGAAGATCCTTTGTTACCAATGCTTAATCAGTGAGGCACCTATCTCAGCGATCTGT
CTATTTCGTTCATCCATAGTTGCCTGACTCCCCGTCGTGTAGATAACTACGATACGGGAGGGCTTACCATCTGGC
CCCAGTGCTGCAATGATACCGCGAGACCCACGCTCACCGGCTCCAGATTTATCAGCAATAAACCAGCCAGCCGGA
AGGGCCGAGCGCAGAAGTGGTCCTGCAACTTTATCCGCCTCCATCCAGTCTATTAATTGTTGCCGGGAAGCTAGA
GTAAGTAGTTCGCCAGTTAATAGTTTGCGCAACGTTGTTGCCATTGCTACAGGCATCGTGGTGTCACGCTCGTCG
TTTGGTATGGCTTCATTCAGCTCCGGTTCCCAACGATCAAGGCGAGTTACATGATCCCCCATGTTGTGCAAAAAA
GCGGTTAGCTCCTTCGGTCCTCCGATCGTTGTCAGAAGTAAGTTGGCCGCAGTGTTATCACTCATGGTTATGGCA
GCACTGCATAATTCTCTTACTGTCATGCCATCCGTAAGATGCTTTTCTGTGACTGGTGAGTACTCAACCAAGTCA
TTCTGAGAATAGTGTATGCGGCGACCGAGTTGCTCTTGCCCGGCGTCAATACGGGATAATACCGCGCCACATAGC
AGAACTTTAAAAGTGCTCATCATTGGAAAACGTTCTTCGGGGCGAAAACTCTCAAGGATCTTACCGCTGTTGAGA
TCCAGTTCGATGTAACCCACTCGTGCACCCAACTGATCTTCAGCATCTTTTACTTTCACCAGCGTTTCTGGGTGA
GCAAAAACAGGAAGGCAAAATGCCGCAAAAAAGGGAATAAGGGCGACACGGAAATGTTGAATACTCATACTCTTC
CTTTTTCAATATTATTGAAGCATTTATCAGGGTTATTGTCTCATGTCCGCGCGTTTCCTGCATCTTTTAATCAAA
TCCCAAGATGTGTATAAACCACCAAACTGCCAAAAAATGAAAACTGTCGACAAGCTCTGTCCGTTTGCTGGCAAC
TGCAAGGGTCTCAATCCTATTTGTAATTATTGAATAATAAAACAATTATAAATGTCAAATTTGTTTTTTATTAAC
GATACAAACCAAACGCAACAAGAACATTTGTAGTATTATCTATAATTGAAAACGCGTAGTTATAATCGCTGAGGT
AATATTTAAAATCATTTTCAAATGATTCACAGTTAATTTGCGACAATATAATTTTATTTTCACATAAACTAGACG
CCTTGTCGTCTTCTTCTTCGTATTCCTTCTCTTTTTCATTTTTCTCTTCATAAAAATTAACATAGTTATTATCGT
ATCCATATATGTATCTATCGTATAGAGTAAATTTTTTGTTGTCATAAATATATATGTCTTTTTTAATGGGGTGTA
TAGTACCGCTGCGCATAGTTTTTCTGTAATTTACAACAGTGCTATTTTCTGGTAGTTCTTCGGAGTGTGTTGCTT
TAATTATTAAATTTATATAATCAATGAATTTGGGATCGTCGGTTTTGTACAATATGTTGCCGGCATAGTACGCAG
CTTCTTCTAGTTCAATTACACCATTTTTTAGCAGCACCGGATTAACATAACTTTCCAAAATGTTGTACGAACCGT
TAAACAAAAACAGTTCACCTCCCTTTTCTATACTATTGTCTGCGAGCAGTTGTTTGTTGTTAAAAATAACAGCCA
TTGTAATGAGACGCACAAACTAATATCACAAACTGGAAATGTCTATCAATATATAGTTGCTCTAGTTATTAATAG
TAATCAATTACGGGGTCATTAGTTCATAGCCCATATATGGAGTTCCGCGTTACATAACTTACGGTAAATGGCCCG
CCTGGCTGACCGCCCAACGACCCCCGCCCATTGACGTCAATAATGACGTATGTTCCCATAGTAACGCCAATAGGG
ACTTTCCATTGACGTCAATGGGTGGACTATTTACGGTAAACTGCCCACTTGGCAGTACATCAAGTGTATCATATG
CCAAGTACGCCCCCTATTGACGTCAATGACGGTAAATGGCCCGCCTGGCATTATGCCCAGTACATGACCTTATGG
GACTTTCCTACTTGGCAGTACATCTACGTATTAGTCATCGCTATTACCATGCATGGTCGAGGTGAGCCCCACGTT
CTGCTTCACTCTCCCCATCTCCCCCCCCTCCCCACCCCCAATTTTGTATTTATTTATTTTTTAATTATTTTGTGC
AGCGATGGGGGCGGGGGGGGGGGGGGGGCGCGCGCCAGGCGGGGCGGGGCGGGGCGAGGGGCGGGGCGGGGCGAG
GCGGAGAGGTGCGGCGGCAGCCAATCAGAGCGGCGCGCTCCGAAAGTTTCCTTTTATGGCGAGGCGGCGGCGGCG
GCGGCCCTATAAAAAGCGAAGCGCGCGGCGGGCGGGAGTCGCTGCGACGCTGCCTTCGCCCCGTGCCCCGCTCCG
CCGCCGCCTCGCGCCGCCCGCCCCGGCTCTGACTGACCGCGTTACTCCCACAGGTGAGCGGGCGGGACGGCCCTT
CTCCTTCGGGCTGTAATTAGCGCTTGGTTTAATGACGGCTTGTTTCTTTTCTGTGGCTGCGTGAAAGCCTTGAGG
GGCTCCGGGAGGGCCCTTTGTGCGGGGGGAGCGGCTCGGGGCTGTCCGCGGGGGGACGGCTGCCTTCGGGGGGGA
CGGGGCAGGGCGGGGTTCGGCTTCTGGCGTGTGACCGGCGGCTCTAGAGCCTCTGCTAACCATGTTCATGCCTTC
TTCTTTTTCCTACAGCTCCTGGGCAACGTGCTGGTTATTGTGCTGTCTCATCATTTTGGCAAAGAATTGGATCGG
ACCGAAATTAATACGACTCACTATA

### Example: Enhancing the sensitivity of an indirect immunofluorescent test for the direct detection of an autoantibody to ACh receptor

60 samples comprising an autoantibody to ACh receptor as shown by RIA (EUROIMMUN Medizinische Labordiagnostika AG, RA 1435-1, carried out according manufacturer's instructions) were subjected to immunofluorescence analysis.

A biochip mosaik comprising a panel with fixed HEK293 cells expressing the ACh receptor comprising the epsilon subunit and a panel coated with fixed HEK293 cells expressing the ACh receptor comprising the gamma subunit was prepared (**Fig. 1**). Cells were made by transfecting HEK293 cells cultured according to standard protocols with plasmids pTriEx-1-CHRNA1 (SEQ ID NO7, alpha subunit), pTriEx-1-CHRNB1 (SEQ ID NO8, beta subunit), pTriEx-1-CHRND (SEQ ID NO9, delta subunit), pTriEx-1-RAPSyn (SEQ ID NO12) and either pTriEx-1-CHRNE (SEQ ID NO 10, epsilon subunit) or pTriEx-1-CHRNG (SEQ ID NO 11, gamma subunit)). The protocol provided by Invitrogen for the T-Rex™ System (Catalog number K1020-01, from 2011) was used for stable transfection. However, ExGen500 (Catalog number 12783652, Thermofisher) was used rather than Lipofectamin. according to the manufacturer's instructions followed by fixing the coated cells by immersion in formalin and followed by washing. Equal amounts of all vectors were used.

The Titerplane technology, described with suitable reagents in the manual of FA_112d-1_A_DE_C13 was subsequently used with minor modifications. Essentially human serum samples were diluted 1:10 in SAMPLE BUFFER and then 30 µl of diluted sample was placed on the biochip and incubated for 30 minutes. Unbound IgG was washed off using PBS 0.2% Tween. Any bound IgG was detected by incubating the substrate with 25-30 µl CONJUGATE_1 (biotinylated goat anti-human-IgG, EUROIMMUN), washing it with PBS-Tween and incubating the substrate with 25 µl CONJUGATE_2 (fluorescein labelled extravidin, EUROIMMUN). An example is shown in **Fig. 2****.** Samples were then analyzed using a EUROStar Bluelight microscope (EUROIMMUN Medizinische Labordiagnostika AG, Lübeck) according to the manufacturer's instructions.

### Results:

55/60 samples could be shown to contain an autoantibody to AChR. 3/60 were identified as positive only when the ACh receptor comprising the epsilon subunit was used as an antigen.
2/60 could be identified as positive only when the ACh receptor comprising the gamma subunit was used.

### Conclusions:

Some samples comprising autoantibodies to ACh receptor comprise autoantibodies only that are monospecific to the epsilon or gamma subunit in the assembled receptor.

Some samples could even be shown to be positive only when the ACh receptor comprising the fetal subunit was used. This is in complete contrast to George *et al.* (George, S., Noack, M., Vanek, M., Rentzsch, J., Röber, N., Conrad, K., Roggenbuck, D., and Küpper, J. H. (2015) Expression of nicotinic acetylcholine receptor subunits in HEp-2 cells for immunodetection of autoantibodies specificities in sera from Myasthenia gravis patients, Clin. Hamorheology and Microcirculation DOI 10.3233/CH-15199).

A certain number of samples positive in the RIA could not be identified as such. However, it should also be borne in mind that the RIA has been reported to occasionally produce false-positive results (Maddison, P., Sadalage, G., Ambrose, P. A., Jacob, S., and Vincent, A. (2019) False-positive acetylcholine receptor antibody results in patients without myasthenia gravis, J. Neuroimm. 332, 69-72).

Therefore, the combination of both receptors should be used in immunofluorescence analyses, at least when the presence or absence of the autoantibodies rather than a colocalization is detected.

## Claims

1. A diagnostically useful carrier comprising a eukaryotic cell overexpressing an adult ACh receptor or a variant thereof and a eukaryotic cell overexpressing a fetal ACh receptor or a variant thereof, wherein the cell is fixed for immunofluorescence.

2. The carrier according to claim 1, wherein any eukaryotic cell overexpressing an autoantigen is pre-fixed.

3. The diagnostically useful carrier according to any of claims 1 to 2, wherein the carrier further comprises a eukaryotic cell overexpressing Musk or a variant thereof.

4. The diagnostically useful carrier according to any of claims 1 to 3, wherein the cell overexpressing an adult ACh receptor or a variant thereof and the cell overexpressing a fetal ACh receptor or a variant thereof permanently express adult and fetal ACh receptor, respectively.

5. The diagnostically useful carrier according to any of claims 1 to 4, wherein the carrier further comprises a control, preferably a mock-transfected cell.

6. The diagnostically useful carrier according to any of claims 1 to 5, wherein the cell is selected from the group comprising HEK293, HeLa, CHO and Jurkat cells and derivatives thereof.

7. The diagnostically useful carrier according to any of claims 1 to 6,
wherein at least one eukaryotic cell, preferably all eukaryotic cells overexpressing an autoantigen from the group comprising adult ACh receptor, fetal ACh receptor and Musk or a variant thereof comprises or comprise one or more vectors, wherein the nucleic acid encoding the autoantigen is under the control of an promotor inducible by addition of an inducer,
and wherein for each cell an additional spatially separate eukaryotic cell is present which comprises the same vector lacking said nucleic acid or comprises the same vector, but does not express the autoantigen, preferably because of the absence of the inducer.

8. A method for the detection of an autoantibody to ACh receptor, optionally in addition to detecting an autoantibody to Musk, comprising the steps:
a) contacting the sample with the carrier according to any of claims 1 to 7 under conditions allowing formation of a complex comprising an autoantibody from the group comprising an autoantibody to ACh receptor, preferably adult and/or fetal ACh receptor, and Musk and an autoantigen from the group comprising ACh receptor and Musk,
b) contacting any complex formed in step b) with a fluorescent ligand to the autoantibody,
c) removing any fluorescent ligand not bound to an autoantibody, and
d) detecting the presence or absence of the fluorescent ligand.

9. A kit comprising the carrier according to any of claims 1 to 7 and a ligand to an autoantibody to ACh receptor, optionally also to Musk, wherein the ligand is a fluorescent ligand, wherein any eukaryotic cell overexpressing an autoantigen is preferably pre-fixed.

10. A use of the combination of a eukaryotic cell overexpressing an adult ACh receptor or a variant thereof and a eukaryotic cell overexpressing a fetal ACh receptor or a variant thereof for increasing the sensitivity of an immunofluorescence assay for the detection of an autoantibody to ACh receptor.

11. The use according to claim 10, wherein the presence or absence of a fluorescent ligand binding to the autoantibody indicates whether or not the autoantibody is present in a sample.

12. The carrier, method or use according to any of claims 1 to 11, wherein the eukaryotic cell expressing an adult ACh receptor or a variant thereof and the eukaryotic cell overexpressing a fetal ACh receptor or a variant thereof and optionally the eukaryotic cell expressing Musk or a variant thereof are spatially separated.

13. The carrier, method or use according to any of claims 1 to 12, wherein the eukaryotic cell overexpressing an adult ACh receptor or a variant thereof and a eukaryotic cell overexpressing a fetal ACh receptor or a variant thereof and optionally the eukaryotic cell expressing Musk or a variant thereof are colocalized.

14. The carrier, method or use according to any of claims 1 to 13, wherein the sample comprises antibodies from a patient, preferably blood, more preferably selected from the group comprising serum, liquor, plasma and whole blood.

15. The carrier, method or use according to any of claims 1 to 14, wherein the carrier method or use is for the diagnosis of myasthenia gravis, LEMS and OMD.


SEQUENCE LISTING

<110>  EUROIMMUN Medizinische Labordiagnostika AG

<120>  Method for the detection of an autoantibody to acetylcholine
       receptor

<130>  19PP166EP1

<160>  14

<170>  PatentIn version 3.5

<210>  1
<211>  457
<212>  PRT
<213>  Homo sapiens

<400>  1

<210>  2
<211>  501
<212>  PRT
<213>  Homo sapiens

<400>  2

<210>  3
<211>  517
<212>  PRT
<213>  Homo sapiens

<400>  3

<210> 4
<211> 493
<212> PRT
<213> Homo sapiens

<400> 4

<210>  5
<211>  517
<212>  PRT
<213>  Homo sapiens

<400>  5

<210>  6
<211>  412
<212>  PRT
<213>  Homo sapiens

<400>  6

<210>  7
<211>  6988
<212>  DNA
<213>  Artificial Sequence

<220>
<223>  pTriEx-1-CHRNA1

<400>  7
ggggaattgt gagcggataa caattccccg gagttaatcc gggaccttta attcaaccca      60

acacaatata ttatagttaa ataagaatta ttatcaaatc atttgtatat taattaaaat     120

actatactgt aaattacatt ttatttacaa tcaaaggaga tataccatgg agccctggcc     180

tctcctcctg ctctttagcc tttgctcagc tggcctcgtc ctgggctccg aacatgagac     240

ccgtctggtg gcaaagctat ttaaagacta cagcagcgtg gtgcggccag tggaagacca     300

ccgccaggtc gtggaggtca ccgtgggcct gcagctgata cagctcatca atgtggatga     360

agtaaatcag atcgtgacaa ccaatgtgcg tctgaaacag caatgggtgg attacaacct     420

aaaatggaat ccagatgact atggcggtgt gaaaaaaatt cacattcctt cagaaaagat     480

ctggcgccca gaccttgttc tctataacaa tgcagatggt gactttgcta ttgtcaagtt     540

caccaaagtg ctcctgcagt acactggcca catcacgtgg acacctccag ccatctttaa     600

aagctactgt gagatcatcg tcacccactt tccctttgat gaacagaact gcagcatgaa 660

gctgggcacc tggacctacg acggctctgt cgtggccatc aacccggaaa gcgaccagcc 720

agacctgagc aacttcatgg agagcgggga gtgggtgatc aaggagtccc ggggctggaa 780

gcactccgtg acctattcct gctgccccga caccccctac ctggacatca cctaccactt 840

cgtcatgcag cgcctgcccc tctacttcat cgtcaacgtc atcatcccct gcctgctctt 900

ctccttctta actggcctgg tattctacct gcccacagac tcaggggaga agatgactct 960

gagcatctct gtcttactgt ctttgactgt gttccttctg gtcatcgtgg agctgatccc 1020

ctccacgtcc agtgctgtgc ccttgattgg aaaatacatg ctgttcacca tggtgttcgt 1080

cattgcctcc atcatcatca ctgtcatcgt catcaacaca caccaccgct cacccagcac 1140

ccatgtcatg cccaactggg tgcggaaggt ttttatcgac actatcccaa atatcatgtt 1200

tttctccaca atgaaaagac catccagaga aaagcaagac aaaaagattt ttacagaaga 1260

cattgatatc tctgacattt ctggaaagcc agggcctcca cccatgggct tccactctcc 1320

cctgatcaaa caccccgagg tgaaaagtgc catcgagggc atcaagtaca tcgcagagac 1380

catgaagtca gaccaggagt ctaacaatgc ggcggcagag tggaagtacg ttgcaatggt 1440

gatggaccac atactcctcg gagtcttcat gcttgtttgc atcatcggaa ccctagccgt 1500

gtttgcaggt cgactcattg aattaaatca gcaaggatga ctcgagcacc accatcacca 1560

tcaccatcac taagtgatta acctcaggtg caggctgcct atcagaaggt ggtggctggt 1620

gtggccaatg ccctggctca caaataccac tgagatcgat ctttttccct ctgccaaaaa 1680

ttatggggac atcatgaagc cccttgagca tctgacttct ggctaataaa ggaaatttat 1740

tttcattgca atagtgtgtt ggaatttttt gtgtctctca ctcggaagga catatgggag 1800

ggcaaatcat ttaaaacatc agaatgagta tttggtttag agtttggcaa catatgccca 1860

tatgtaacta gcataacccc ttggggcctc taaacgggtc ttgaggggtt ttttgctgaa 1920

agcatgcgga ggaaattctc cttgaagttt ccctggtgtt caaagtaaag gagtttgcac 1980

cagacgcacc tctgttcact ggtccggcgt attaaaacac gatacattgt tattagtaca 2040

tttattaagc gctagattct gtgcgttgtt gatttacaga caattgttgt acgtatttta 2100

ataattcatt aaatttataa tctttagggt ggtatgttag agcgaaaatc aaatgatttt 2160

cagcgtcttt atatctgaat ttaaatatta aatcctcaat agatttgtaa aataggtttc 2220

gattagtttc aaacaagggt tgtttttccg aaccgatggc tggactatct aatggatttt 2280

cgctcaacgc cacaaaactt gccaaatctt gtagcagcaa tctagctttg tcgatattcg 2340

tttgtgtttt gttttgtaat aaaggttcga cgtcgttcaa aatattatgc gcttttgtat 2400

ttctttcatc actgtcgtta gtgtacaatt gactcgacgt aaacacgtta aatagagctt 2460

ggacatattt aacatcgggc gtgttagctt tattaggccg attatcgtcg tcgtcccaac 2520

cctcgtcgtt agaagttgct tccgaagacg attttgccat agccacacga cgcctattaa 2580

ttgtgtcggc taacacgtcc gcgatcaaat ttgtagttga gctttttgga attatttctg 2640

attgcgggcg tttttgggcg ggtttcaatc taactgtgcc cgattttaat tcagacaaca 2700

cgttagaaag cgatggtgca ggcggtggta acatttcaga cggcaaatct actaatggcg 2760

gcggtggtgg agctgatgat aaatctacca tcggtggagg cgcaggcggg gctggcggcg 2820

gaggcggagg cggaggtggt ggcggtgatg cagacggcgg tttaggctca aatgtctctt 2880

taggcaacac agtcggcacc tcaactattg tactggtttc gggcgccgtt tttggtttga 2940

ccggtctgag acgagtgcga tttttttcgt ttctaatagc ttccaacaat tgttgtctgt 3000

cgtctaaagg tgcagcgggt tgaggttccg tcggcattgg tggagcgggc ggcaattcag 3060

acatcgatgg tggtggtggt ggtggaggcg ctggaatgtt aggcacggga gaaggtggtg 3120

gcggcggtgc cgccggtata atttgttctg gtttagtttg ttcgcgcacg attgtgggca 3180

ccggcgcagg cgccgctggc tgcacaacgg aaggtcgtct gcttcgaggc agcgcttggg 3240

gtggtggcaa ttcaatatta taattggaat acaaatcgta aaaatctgct ataagcattg 3300

taatttcgct atcgtttacc gtgccgatat ttaacaaccg ctcaatgtaa gcaattgtat 3360

tgtaaagaga ttgtctcaag ctcggaacgc tgcgctcggt cgttcggctg cggcgagcgg 3420

tatcagctca ctcaaaggcg gtaatacggt tatccacaga atcaggggat aacgcaggaa 3480

agaacatgtg agcaaaaggc cagcaaaagg ccaggaaccg taaaaaggcc gcgttgctgg 3540

cgtttttcca taggctccgc ccccctgacg agcatcacaa aaatcgacgc tcaagtcaga 3600

ggtggcgaaa cccgacagga ctataaagat accaggcgtt tccccctgga agctccctcg 3660

tgcgctctcc tgttccgacc ctgccgctta ccggatacct gtccgccttt ctcccttcgg 3720

gaagcgtggc gctttctcaa tgctcacgct gtaggtatct cagttcggtg taggtcgttc 3780

gctccaagct gggctgtgtg cacgaacccc ccgttcagcc cgaccgctgc gccttatccg 3840

gtaactatcg tcttgagtcc aacccggtaa gacacgactt atcgccactg gcagcagcca 3900

ctggtaacag gattagcaga gcgaggtatg taggcggtgc tacagagttc ttgaagtggt 3960

ggcctaacta cggctacact agaaggacag tatttggtat ctgcgctctg ctgaagccag 4020

ttaccttcgg aaaaagagtt ggtagctctt gatccggcaa acaaaccacc gctggtagcg 4080

gtggtttttt tgtttgcaag cagcagatta cgcgcagaaa aaaaggatct caagaagatc 4140

ctttgttacc aatgcttaat cagtgaggca cctatctcag cgatctgtct atttcgttca 4200

tccatagttg cctgactccc cgtcgtgtag ataactacga tacgggaggg cttaccatct 4260

ggccccagtg ctgcaatgat accgcgagac ccacgctcac cggctccaga tttatcagca 4320

ataaaccagc cagccggaag ggccgagcgc agaagtggtc ctgcaacttt atccgcctcc 4380

atccagtcta ttaattgttg ccgggaagct agagtaagta gttcgccagt taatagtttg 4440

cgcaacgttg ttgccattgc tacaggcatc gtggtgtcac gctcgtcgtt tggtatggct 4500

tcattcagct ccggttccca acgatcaagg cgagttacat gatcccccat gttgtgcaaa 4560

aaagcggtta gctccttcgg tcctccgatc gttgtcagaa gtaagttggc cgcagtgtta 4620

tcactcatgg ttatggcagc actgcataat tctcttactg tcatgccatc cgtaagatgc 4680

ttttctgtga ctggtgagta ctcaaccaag tcattctgag aatagtgtat gcggcgaccg 4740

agttgctctt gcccggcgtc aatacgggat aataccgcgc cacatagcag aactttaaaa 4800

gtgctcatca ttggaaaacg ttcttcgggg cgaaaactct caaggatctt accgctgttg 4860

agatccagtt cgatgtaacc cactcgtgca cccaactgat cttcagcatc ttttactttc 4920

accagcgttt ctgggtgagc aaaaacagga aggcaaaatg ccgcaaaaaa gggaataagg 4980

gcgacacgga aatgttgaat actcatactc ttcctttttc aatattattg aagcatttat 5040

cagggttatt gtctcatgtc cgcgcgtttc ctgcatcttt taatcaaatc ccaagatgtg 5100

tataaaccac caaactgcca aaaaatgaaa actgtcgaca agctctgtcc gtttgctggc 5160

aactgcaagg gtctcaatcc tatttgtaat tattgaataa taaaacaatt ataaatgtca 5220

aatttgtttt ttattaacga tacaaaccaa acgcaacaag aacatttgta gtattatcta 5280

taattgaaaa cgcgtagtta taatcgctga ggtaatattt aaaatcattt tcaaatgatt 5340

cacagttaat ttgcgacaat ataattttat tttcacataa actagacgcc ttgtcgtctt 5400

cttcttcgta ttccttctct tttcattttt tctcttcata aaaattaaca tagttattat 5460

cgtatccata tatgtatcta tcgtatagag taaatttttt gttgtcataa atatatatgt 5520

cttttttaat ggggtgtata gtaccgctgc gcatagtttt tctgtaattt acaacagtgc 5580

tattttctgg tagttcttcg gagtgtgttg ctttaattat taaatttata taatcaatga 5640

atttgggatc gtcggttttg tacaatatgt tgccggcata gtacgcagct tcttctagtt 5700

caattacacc attttttagc agcaccggat taacataact ttccaaaatg ttgtacgaac 5760

cgttaaacaa aaacagttca cctccctttt ctatactatt gtctgcgagc agttgtttgt 5820

tgttaaaaat aacagccatt gtaatgagac gcacaaacta atatcacaaa ctggaaatgt 5880

ctatcaatat atagttgctc tagttattaa tagtaatcaa ttacggggtc attagttcat 5940

agcccatata tggagttccg cgttacataa cttacggtaa atggcccgcc tggctgaccg 6000

cccaacgacc cccgcccatt gacgtcaata atgacgtatg ttcccatagt aacgccaata 6060

gggactttcc attgacgtca atgggtggac tatttacggt aaactgccca cttggcagta 6120

catcaagtgt atcatatgcc aagtacgccc cctattgacg tcaatgacgg taaatggccc 6180

gcctggcatt atgcccagta catgacctta tgggactttc ctacttggca gtacatctac 6240

gtattagtca tcgctattac catgcatggt cgaggtgagc cccacgttct gcttcactct 6300

ccccatctcc cccccctccc cacccccaat tttgtattta tttatttttt aattattttg 6360

tgcagcgatg ggggcggggg gggggggggg gcgcgcgcca ggcggggcgg ggcggggcga 6420

ggggcggggc ggggcgaggc ggagaggtgc ggcggcagcc aatcagagcg gcgcgctccg 6480

aaagtttcct tttatggcga ggcggcggcg gcggcggccc tataaaaagc gaagcgcgcg 6540

gcgggcggga gtcgctgcga cgctgccttc gccccgtgcc ccgctccgcc gccgcctcgc 6600

gccgcccgcc ccggctctga ctgaccgcgt tactcccaca ggtgagcggg cgggacggcc 6660

cttctccttc gggctgtaat tagcgcttgg tttaatgacg gcttgtttct tttctgtggc 6720

tgcgtgaaag ccttgagggg ctccgggagg gccctttgtg cggggggagc ggctcggggc 6780

tgtccgcggg gggacggctg ccttcggggg ggacggggca gggcggggtt cggcttctgg 6840

cgtgtgaccg gcggctctag agcctctgct aaccatgttc atgccttctt cttttcccta 6900

cagctcctgg gcaacgtgct ggttattgtg ctgtctcatc attttggcaa agaattggat 6960

cggaccgaaa ttaatacgac tcactata 6988

<210> 8
<211> 7120
<212> DNA
<213> Artificial Sequence

<220>
<223> pTriEx-1-CHRNB1

<400> 8
ggggaattgt gagcggataa caattccccg gagttaatcc gggaccttta attcaaccca 60

acacaatata ttatagttaa ataagaatta ttatcaaatc atttgtatat taattaaaat 120

actatactgt aaattacatt ttatttacaa tcaaaggaga tataccatga ccccaggggc 180

tctgctgatg ctgctggggg cgctgggggc gccgctcgcc ccaggcgtcc gcggctcgga 240

ggcggagggt cgactccggg agaaactttt ctctggctat gatagctccg tgcggccagc 300

gcgggaggtg ggagaccgtg tcagggtcag cgttggtctc atcctggcgc aactcatcag 360

cctgaacgag aaggatgaag agatgagcac aaaggtgtac ttagacctgg agtggactga 420

ctacaggctg agctgggacc ctgcggagca cgacggcatc gattcgctcc gcatcacggc 480

ggaatccgtg tggctccctg acgtggtgct actgaacaac aatgatggga attttgacgt 540

ggctctggac attagcgtcg tggtgtcctc cgacggctcc gtgcgttggc aacccccggg 600

catctatcgc agcagctgca gcatccaggt cacctacttc cccttcgact ggcagaattg 660

cactatggtg ttcagctcct acagctacga cagctcggag gtcagcctgc agacaggcct 720

gggtcctgac gggcaaggc atcaggaaat ccacattcat gaaggacttt tcattgagaa 780

tggccagtgg gagattatcc acaagccctc tcggctaatc cagcctccag gcgatcctag 840

gggagggagg gaaggacagc gccaggaagt catcttctac ctcatcatcc gccgcaagcc 900

tctcttctac ctggtcaacg tcattgcccc atgcatcctc atcactcttc tggccatctt 960

cgtcttctac ctgccaccag atgcaggaga gaagatgggg ctctcaatct ttgccctgct 1020

gacccttact gtgttcctgc tgctgctggc tgacaaagta cctgagacct cactatcagt 1080

acccattatt atcaagtacc tcatgtttac catggtcctc gtcaccttct cagtcatcct 1140

tagtgtcgtg gttctcaacc tgcaccaccg ctcaccccac acccaccaaa tgcccctttg 1200

ggtccgtcag atcttcattc acaaacttcc gctgtacctg cgtctaaaaa ggcccaaacc 1260

cgagagagac ctgatgccgg agccccctca ctgttcttct ccaggaagtg gctggggtcg 1320

gggaacagat gaatatttca tccggaagcc gccaagtgat tttctcttcc ccaaacccaa 1380

taggttccag cctgaactgt ctgcccctga tctgcggcga tttatcgatg gtccaaaccg 1440

ggctgtggcc ctgcttccgg agctacggga ggtcgtctcc tctatcagct acatcgctcg 1500

acagctgcag gaacaggagg accacgatgc gctgaaggag gactggcagt ttgtggccat 1560

ggtagtggac cgcctcttcc tgtggacttt catcatcttc accagcgttg ggaccctagt 1620

catcttcctg gacgccacgt accacttgcc ccctccagac ccctttcctt aactcgagca 1680

ccaccatcac catcaccatc actaagtgat taacctcagg tgcaggctgc ctatcagaag 1740

gtggtggctg gtgtggccaa tgccctggct cacaaatacc actgagatcg atctttttcc 1800

ctctgccaaa aattatgggg acatcatgaa gccccttgag catctgactt ctggctaata 1860

aaggaaattt attttcattg caatagtgtg ttggaatttt ttgtgtctct cactcggaag 1920

gacatatggg agggcaaatc atttaaaaca tcagaatgag tatttggttt agagtttggc 1980

aacatatgcc catatgtaac tagcataacc ccttggggcc tctaaacggg tcttgagggg 2040

ttttttgctg aaagcatgcg gaggaaattc tccttgaagt ttccctggtg ttcaaagtaa 2100

aggagtttgc accagacgca cctctgttca ctggtccggc gtattaaaac acgatacatt 2160

gttattagta catttattaa gcgctagatt ctgtgcgttg ttgatttaca gacaattgtt 2220

gtacgtattt taataattca ttaaatttat aatctttagg gtggtatgtt agagcgaaaa 2280

tcaaatgatt ttcagcgtct ttatatctga atttaaatat taaatcctca atagatttgt 2340

aaaataggtt tcgattagtt tcaaacaagg gttgtttttc cgaaccgatg gctggactat 2400

ctaatggatt ttcgctcaac gccacaaaac ttgccaaatc ttgtagcagc aatctagctt 2460

tgtcgatatt cgtttgtgtt ttgttttgta ataaaggttc gacgtcgttc aaaatattat 2520

gcgcttttgt atttctttca tcactgtcgt tagtgtacaa ttgactcgac gtaaacacgt 2580

taaatagagc ttggacatat ttaacatcgg gcgtgttagc tttattaggc cgattatcgt 2640

cgtcgtccca accctcgtcg ttagaagttg cttccgaaga cgattttgcc atagccacac 2700

gacgcctatt aattgtgtcg gctaacacgt ccgcgatcaa atttgtagtt gagctttttg 2760

gaattatttc tgattgcggg cgtttttggg cgggtttcaa tctaactgtg cccgatttta 2820

attcagacaa cacgttagaa agcgatggtg caggcggtgg taacatttca gacggcaaat 2880

ctactaatgg cggcggtggt ggagctgatg ataaatctac catcggtgga ggcgcaggcg 2940

gggctggcgg cggaggcgga ggcggaggtg gtggcggtga tgcagacggc ggtttaggct 3000

caaatgtctc tttaggcaac acagtcggca cctcaactat tgtactggtt tcgggcgccg 3060

tttttggttt gaccggtctg agacgagtgc gatttttttc gtttctaata gcttccaaca 3120

attgttgtct gtcgtctaaa ggtgcagcgg gttgaggttc cgtcggcatt ggtggagcgg 3180

gcggcaattc agacatcgat ggtggtggtg gtggtggagg cgctggaatg ttaggcacgg 3240

gagaaggtgg tggcggcggt gccgccggta taatttgttc tggtttagtt tgttcgcgca 3300

cgattgtggg caccggcgca ggcgccgctg gctgcacaac ggaaggtcgt ctgcttcgag 3360

gcagcgcttg gggtggtggc aattcaatat tataattgga atacaaatcg taaaaatctg 3420

ctataagcat tgtaatttcg ctatcgttta ccgtgccgat atttaacaac cgctcaatgt 3480

aagcaattgt attgtaaaga gattgtctca agctcggaac gctgcgctcg gtcgttcggc 3540

tgcggcgagc ggtatcagct cactcaaagg cggtaatacg gttatccaca gaatcagggg 3600

ataacgcagg aaagaacatg tgagcaaaag gccagcaaaa ggccaggaac cgtaaaaagg 3660

ccgcgttgct ggcgtttttc cataggctcc gcccccctga cgagcatcac aaaaatcgac 3720

gctcaagtca gaggtggcga aacccgacag gactataaag ataccaggcg tttccccctg 3780

gaagctccct cgtgcgctct cctgttccga ccctgccgct taccggatac ctgtccgcct 3840

ttctcccttc gggaagcgtg gcgctttctc aatgctcacg ctgtaggtat ctcagttcgg 3900

tgtaggtcgt tcgctccaag ctgggctgtg tgcacgaacc ccccgttcag cccgaccgct 3960

gcgccttatc cggtaactat cgtcttgagt ccaacccggt aagacacgac ttatcgccac 4020

tggcagcagc cactggtaac aggattagca gagcgaggta tgtaggcggt gctacagagt 4080

tcttgaagtg gtggcctaac tacggctaca ctagaaggac agtatttggt atctgcgctc 4140

tgctgaagcc agttaccttc ggaaaaagag ttggtagctc ttgatccggc aaacaaacca 4200

ccgctggtag cggtggtttt tttgtttgca agcagcagat tacgcgcaga aaaaaaggat 4260

ctcaagaaga tcctttgtta ccaatgctta atcagtgagg cacctatctc agcgatctgt 4320

ctatttcgtt catccatagt tgcctgactc cccgtcgtgt agataactac gatacgggag 4380

ggcttaccat ctggccccag tgctgcaatg ataccgcgag acccacgctc accggctcca 4440

gatttatcag caataaacca gccagccgga agggccgagc gcagaagtgg tcctgcaact 4500

ttatccgcct ccatccagtc tattaattgt tgccgggaag ctagagtaag tagttcgcca 4560

gttaatagtt tgcgcaacgt tgttgccatt gctacaggca tcgtggtgtc acgctcgtcg 4620

tttggtatgg cttcattcag ctccggttcc caacgatcaa ggcgagttac atgatccccc 4680

atgttgtgca aaaaagcggt tagctccttc ggtcctccga tcgttgtcag aagtaagttg 4740

gccgcagtgt tatcactcat ggttatggca gcactgcata attctcttac tgtcatgcca 4800

tccgtaagat gcttttctgt gactggtgag tactcaacca agtcattctg agaatagtgt 4860

atgcggcgac cgagttgctc ttgcccggcg tcaatacggg ataataccgc gccacatagc 4920

agaactttaa aagtgctcat cattggaaaa cgttcttcgg ggcgaaaact ctcaaggatc 4980

ttaccgctgt tgagatccag ttcgatgtaa cccactcgtg cacccaactg atcttcagca 5040

tcttttactt tcaccagcgt ttctgggtga gcaaaaacag gaaggcaaaa tgccgcaaaa 5100

aagggaataa gggcgacacg gaaatgttga atactcatac tcttcctttt tcaatattat 5160

tgaagcattt atcagggtta ttgtctcatg tccgcgcgtt tcctgcatct tttaatcaaa 5220

tcccaagatg tgtataaacc accaaactgc caaaaaatga aaactgtcga caagctctgt 5280

ccgtttgctg gcaactgcaa gggtctcaat cctatttgta attattgaat aataaaacaa 5340

ttataaatgt caaatttgtt ttttattaac gatacaaacc aaacgcaaca agaacatttg 5400

tagtattatc tataattgaa aacgcgtagt tataatcgct gaggtaatat ttaaaatcat 5460

tttcaaatga ttcacagtta atttgcgaca atataatttt attttcacat aaactagacg 5520

ccttgtcgtc ttcttcttcg tattccttct ctttttcatt tttctcttca taaaaattaa 5580

catagttatt atcgtatcca tatatgtatc tatcgtatag agtaaatttt ttgttgtcat 5640

aaatatatat gtctttttta atggggtgta tagtaccgct gcgcatagtt tttctgtaat 5700

ttacaacagt gctattttct ggtagttctt cggagtgtgt tgctttaatt attaaattta 5760

tataatcaat gaatttggga tcgtcggttt tgtacaatat gttgccggca tagtacgcag 5820

cttcttctag ttcaattaca ccatttttta gcagcaccgg attaacataa ctttccaaaa 5880

tgttgtacga accgttaaac aaaaacagtt cacctccctt ttctatacta ttgtctgcga 5940

gcagttgttt gttgttaaaa ataacagcca ttgtaatgag acgcacaaac taatatcaca 6000

aactggaaat gtctatcaat atatagttgc tctagttatt aatagtaatc aattacgggg 6060

tcattagttc atagcccata tatggagttc cgcgttacat aacttacggt aaatggcccg 6120

cctggctgac cgcccaacga cccccgccca ttgacgtcaa taatgacgta tgttcccata 6180

gtaacgccaa tagggacttt ccattgacgt caatgggtgg actatttacg gtaaactgcc 6240

cacttggcag tacatcaagt gtatcatatg ccaagtacgc cccctattga cgtcaatgac 6300

ggtaaatggc ccgcctggca ttatgcccag tacatgacct tatgggactt tcctacttgg 6360

cagtacatct acgtattagt catcgctatt accatgcatg gtcgaggtga gccccacgtt 6420

ctgcttcact ctccccatct cccccccctc cccaccccca attttgtatt tatttatttt 6480

ttaattattt tgtgcagcga tgggggcggg gggggggggg gggcgcgcgc caggcggggc 6540

ggggcggggc gaggggcggg gcggggcgag gcggagaggt gcggcggcag ccaatcagag 6600

cggcgcgctc cgaaagtttc cttttatggc gaggcggcgg cggcggcggc cctataaaaa 6660

gcgaagcgcg cggcgggcgg gagtcgctgc gacgctgcct tcgccccgtg ccccgctccg 6720

ccgccgcctc gcgccgcccg ccccggctct gactgaccgc gttactccca caggtgagcg 6780

ggcgggacgg cccttctcct tcgggctgta attagcgctt ggtttaatga cggcttgttt 6840

cttttctgtg gctgcgtgaa agccttgagg ggctccggga gggccctttg tgcgggggga 6900

gcggctcggg gctgtccgcg gggggacggc tgccttcggg ggggacgggg cagggcgggg 6960

ttcggcttct ggcgtgtgac cggcggctct agagcctctg ctaaccatgt tcatgccttc 7020

ttctttttcc tacagctcct gggcaacgtg ctggttattg tgctgtctca tcattttggc 7080

aaagaattgg atcggaccga aattaatacg actcactata 7120

<210> 9
<211> 7168
<212> DNA
<213> Artificial Sequence

<220>
<223> pTriEx-1-CHRND

<400> 9
ggggaattgt gagcggataa caattccccg gagttaatcc gggaccttta attcaaccca 60

acacaatata ttatagttaa ataagaatta ttatcaaatc atttgtatat taattaaaat 120

actatactgt aaattacatt ttatttacaa tcaaaggaga tataccatgg aggggccagt 180

gctgacactg gggctgctgg ctgccctggc ggtgtgtggc agctgggggc tgaacgagga 240

ggagcggctg atccggcacc tgtttcaaga gaagggctac aacaaggagc tccggcccgt 300

ggcacacaaa gaggagagtg tggacgttgc cctggccctc acactctcca acctcatctc 360

cctgaaagaa gttgaggaga ccctcactac caatgtgtgg atagagcacg gctggacaga 420

caaccggctg aagtggaatg ctgaagaatt tggaaacatc agtgtcctgc gcctcccccc 480

ggacatggtg tggctcccag agattgtgct ggagaacaac aatgacggct ccttccagat 540

ctcctactcc tgcaacgtgc ttgtctacca ctacggcttc gtgtactggc tgccacctgc 600

catcttccgc tcctcctgcc ccatctctgt cacctatttc cccttcgact ggcagaactg 660

ctccctcaag ttcagttccc tcaagtatac ggccaaagag atcaccctga gcctgaaaca 720

ggatgccaag gagaaccgca cctaccccgt ggagtggatc atcattgatc ctgaaggctt 780

cacagagaac ggggagtggg agatagtcca ccggccggcc agggtcaacg tggaccccag 840

agcccctctg gacagcccca gccgccagga catcaccttc tacctcatca tccgccgcaa 900

gcccctcttc tacatcatca acatcctggt gccctgcgtg ctcatctcct tcatggtcaa 960

cctggtcttc tacctaccgg ctgacagtgg tgagaagaca tcagtggcca tctcggtgct 1020

cctggctcag tctgtcttcc tgctgctcat ctccaagcgt ctgcctgcca catccatggc 1080

catccccctt atcggcaagt tcctgctctt cggcatggtg ctggtcacca tggttgtggt 1140

gatctgtgtc atcgtgctca acatccactt ccgaacaccc agcacccatg tgctgtctga 1200

gggggtcaag aagctcttcc tggagaccct gccggagctc ctgcacatgt cccgcccagc 1260

agaggatgga cccagccctg gggccctggt gcggaggagc agctccctgg gatacatctc 1320

caaggccgag gagtacttcc tgctcaagtc ccgcagtgac ctcatgttcg agaagcagtc 1380

agagcggcat gggctggcca ggcgcctcac cactgcacgc cggcccccag caagctctga 1440

gcaggcccag caggaactct tcaatgagct gaagccagct gtggatgggg caaacttcat 1500

tgttaaccac atgagggacc agaacaatta caatgaggag aaagacagct ggaaccgagt 1560

ggcccgcaca gtggaccgcc tctgcctgtt tgtggtgacg cctgtcatgg tggtgggcac 1620

agcctggatc ttcctgcagg gcgtttacaa ccagccacca ccccagcctt ttcctgggga 1680

cccctactcc tacaacgtgc aggacaagcg cttcatctaa ctcgagcacc accatcacca 1740

tcaccatcac taagtgatta acctcaggtg caggctgcct atcagaaggt ggtggctggt 1800

gtggccaatg ccctggctca caaataccac tgagatcgat ctttttccct ctgccaaaaa 1860

ttatggggac atcatgaagc cccttgagca tctgacttct ggctaataaa ggaaatttat 1920

tttcattgca atagtgtgtt ggaatttttt gtgtctctca ctcggaagga catatgggag 1980

ggcaaatcat ttaaaacatc agaatgagta tttggtttag agtttggcaa catatgccca 2040

tatgtaacta gcataacccc ttggggcctc taaacgggtc ttgaggggtt ttttgctgaa 2100

agcatgcgga ggaaattctc cttgaagttt ccctggtgtt caaagtaaag gagtttgcac 2160

cagacgcacc tctgttcact ggtccggcgt attaaaacac gatacattgt tattagtaca 2220

tttattaagc gctagattct gtgcgttgtt gatttacaga caattgttgt acgtatttta 2280

ataattcatt aaatttataa tctttagggt ggtatgttag agcgaaaatc aaatgatttt 2340

cagcgtcttt atatctgaat ttaaatatta aatcctcaat agatttgtaa aataggtttc 2400

gattagtttc aaacaagggt tgtttttccg aaccgatggc tggactatct aatggatttt 2460

cgctcaacgc cacaaaactt gccaaatctt gtagcagcaa tctagctttg tcgatattcg 2520

tttgtgtttt gttttgtaat aaaggttcga cgtcgttcaa aatattatgc gcttttgtat 2580

ttctttcatc actgtcgtta gtgtacaatt gactcgacgt aaacacgtta aatagagctt 2640

ggacatattt aacatcgggc gtgttagctt tattaggccg attatcgtcg tcgtcccaac 2700

cctcgtcgtt agaagttgct tccgaagacg attttgccat agccacacga cgcctattaa 2760

ttgtgtcggc taacacgtcc gcgatcaaat ttgtagttga gctttttgga attatttctg 2820

attgcgggcg tttttgggcg ggtttcaatc taactgtgcc cgattttaat tcagacaaca 2880

cgttagaaag cgatggtgca ggcggtggta acatttcaga cggcaaatct actaatggcg 2940

gcggtggtgg agctgatgat aaatctacca tcggtggagg cgcaggcggg gctggcggcg 3000

gaggcggagg cggaggtggt ggcggtgatg cagacggcgg tttaggctca aatgtctctt 3060

taggcaacac agtcggcacc tcaactattg tactggtttc gggcgccgtt tttggtttga 3120

ccggtctgag acgagtgcga tttttttcgt ttctaatagc ttccaacaat tgttgtctgt 3180

cgtctaaagg tgcagcgggt tgaggttccg tcggcattgg tggagcgggc ggcaattcag 3240

acatcgatgg tggtggtggt ggtggaggcg ctggaatgtt aggcacggga gaaggtggtg 3300

gcggcggtgc cgccggtata atttgttctg gtttagtttg ttcgcgcacg attgtgggca 3360

ccggcgcagg cgccgctggc tgcacaacgg aaggtcgtct gcttcgaggc agcgcttggg 3420

gtggtggcaa ttcaatatta taattggaat acaaatcgta aaaatctgct ataagcattg 3480

taatttcgct atcgtttacc gtgccgatat ttaacaaccg ctcaatgtaa gcaattgtat 3540

tgtaaagaga ttgtctcaag ctcggaacgc tgcgctcggt cgttcggctg cggcgagcgg 3600

tatcagctca ctcaaaggcg gtaatacggt tatccacaga atcaggggat aacgcaggaa 3660

agaacatgtg agcaaaaggc cagcaaaagg ccaggaaccg taaaaaggcc gcgttgctgg 3720

cgtttttcca taggctccgc ccccctgacg agcatcacaa aaatcgacgc tcaagtcaga 3780

ggtggcgaaa cccgacagga ctataaagat accaggcgtt tccccctgga agctccctcg 3840

tgcgctctcc tgttccgacc ctgccgctta ccggatacct gtccgccttt ctcccttcgg 3900

gaagcgtggc gctttctcaa tgctcacgct gtaggtatct cagttcggtg taggtcgttc 3960

gctccaagct gggctgtgtg cacgaacccc ccgttcagcc cgaccgctgc gccttatccg 4020

gtaactatcg tcttgagtcc aacccggtaa gacacgactt atcgccactg gcagcagcca 4080

ctggtaacag gattagcaga gcgaggtatg taggcggtgc tacagagttc ttgaagtggt 4140

ggcctaacta cggctacact agaaggacag tatttggtat ctgcgctctg ctgaagccag 4200

ttaccttcgg aaaaagagtt ggtagctctt gatccggcaa acaaaccacc gctggtagcg 4260

gtggtttttt tgtttgcaag cagcagatta cgcgcagaaa aaaaggatct caagaagatc 4320

ctttgttacc aatgcttaat cagtgaggca cctatctcag cgatctgtct atttcgttca 4380

tccatagttg cctgactccc cgtcgtgtag ataactacga tacgggaggg cttaccatct 4440

ggccccagtg ctgcaatgat accgcgagac ccacgctcac cggctccaga tttatcagca 4500

ataaaccagc cagccggaag ggccgagcgc agaagtggtc ctgcaacttt atccgcctcc 4560

atccagtcta ttaattgttg ccgggaagct agagtaagta gttcgccagt taatagtttg 4620

cgcaacgttg ttgccattgc tacaggcatc gtggtgtcac gctcgtcgtt tggtatggct 4680

tcattcagct ccggttccca acgatcaagg cgagttacat gatcccccat gttgtgcaaa 4740

aaagcggtta gctccttcgg tcctccgatc gttgtcagaa gtaagttggc cgcagtgtta 4800

tcactcatgg ttatggcagc actgcataat tctcttactg tcatgccatc cgtaagatgc 4860

ttttctgtga ctggtgagta ctcaaccaag tcattctgag aatagtgtat gcggcgaccg 4920

agttgctctt gcccggcgtc aatacgggat aataccgcgc cacatagcag aactttaaaa 4980

gtgctcatca ttggaaaacg ttcttcgggg cgaaaactct caaggatctt accgctgttg 5040

agatccagtt cgatgtaacc cactcgtgca cccaactgat cttcagcatc ttttactttc 5100

accagcgttt ctgggtgagc aaaaacagga aggcaaaatg ccgcaaaaaa gggaataagg 5160

gcgacacgga aatgttgaat actcatactc ttcctttttc aatattattg aagcatttat 5220

cagggttatt gtctcatgtc cgcgcgtttc ctgcatcttt taatcaaatc ccaagatgtg 5280

tataaaccac caaactgcca aaaaatgaaa actgtcgaca agctctgtcc gtttgctggc 5340

aactgcaagg gtctcaatcc tatttgtaat tattgaataa taaaacaatt ataaatgtca 5400

aatttgtttt ttattaacga tacaaaccaa acgcaacaag aacatttgta gtattatcta 5460

taattgaaaa cgcgtagtta taatcgctga ggtaatattt aaaatcattt tcaaatgatt 5520

cacagttaat ttgcgacaat ataattttat tttcacataa actagacgcc ttgtcgtctt 5580

cttcttcgta ttccttctct ttttcatttt tctcttcata aaaattaaca tagttattat 5640

cgtatccata tatgtatcta tcgtatagag taaatttttt gttgtcataa atatatatgt 5700

cttttttaat ggggtgtata gtaccgctgc gcatagtttt tctgtaattt acaacagtgc 5760

tattttctgg tagttcttcg gagtgtgttg ctttaattat taaatttata taatcaatga 5820

atttgggatc gtcggttttg tacaatatgt tgccggcata gtacgcagct tcttctagtt 5880

caattacacc attttttagc agcaccggat taacataact ttccaaaatg ttgtacgaac 5940

cgttaaacaa aaacagttca cctccctttt ctatactatt gtctgcgagc agttgtttgt 6000

tgttaaaaat aacagccatt gtaatgagac gcacaaacta atatcacaaa ctggaaatgt 6060

ctatcaatat atagttgctc tagttattaa tagtaatcaa ttacggggtc attagttcat 6120

agcccatata tggagttccg cgttacataa cttacggtaa atggcccgcc tggctgaccg 6180

cccaacgacc cccgcccatt gacgtcaata atgacgtatg ttcccatagt aacgccaata 6240

gggactttcc attgacgtca atgggtggac tatttacggt aaactgccca cttggcagta 6300

catcaagtgt atcatatgcc aagtacgccc cctattgacg tcaatgacgg taaatggccc 6360

gcctggcatt atgcccagta catgacctta tgggactttc ctacttggca gtacatctac 6420

gtattagtca tcgctattac catgcatggt cgaggtgagc cccacgttct gcttcactct 6480

ccccatctcc cccccctccc cacccccaat tttgtattta tttatttttt aattattttg 6540

tgcagcgatg ggggcggggg gggggggggg gcgcgcgcca ggcggggcgg ggcggggcga 6600

ggggcggggc ggggcgaggc ggagaggtgc ggcggcagcc aatcagagcg gcgcgctccg 6660

aaagtttcct tttatggcga ggcggcggcg gcggcggccc tataaaaagc gaagcgcgcg 6720

gcgggcggga gtcgctgcga cgctgccttc gccccgtgcc ccgctccgcc gccgcctcgc 6780

gccgcccgcc ccggctctga ctgaccgcgt tactcccaca ggtgagcggg cgggacggcc 6840

cttctccttc gggctgtaat tagcgcttgg tttaatgacg gcttgtttct tttctgtggc 6900

tgcgtgaaag ccttgagggg ctccgggagg gccctttgtg cggggggagc ggctcggggc 6960

tgtccgcggg gggacggctg ccttcggggg ggacggggca gggcggggtt cggcttctgg 7020

cgtgtgaccg gcggctctag agcctctgct aaccatgttc atgccttctt ctttttccta 7080

cagctcctgg gcaacgtgct ggttattgtg ctgtctcatc attttggcaa agaattggat 7140

cggaccgaaa ttaatacgac tcactata 7168

<210> 10
<211> 7096
<212> DNA
<213> Artificial Sequence

<220>
<223> pTriEx-1-CHRNE

<400> 10
ggggaattgt gagcggataa caattccccg gagttaatcc gggaccttta attcaaccca 60

acacaatata ttatagttaa ataagaatta ttatcaaatc atttgtatat taattaaaat 120

actatactgt aaattacatt ttatttacaa tcaaaggaga tataccatgg caaggctccg 180

gcttggggtc ctgctcctct tggggcttct cggcaggggt gtggggaaga acgaggaact 240

gcgtctttat caccatctct tcaacaacta tgacccagga agccggccag tgcgggagcc 300

tgaggatact gtcaccatca gcctcaaggt caccctgacg aatctcatct cactgaatga 360

aaaagaggag actctcacca ctagcgtctg gattggaatc gattggcagg attaccgact 420

caactacagc aaggacgact ttgggggtat agaaaccctg cgagtccctt cagaactcgt 480

gtggctgcca gagattgtgc tggaaaacaa tattgatggc cagttcggag tggcctacga 540

cgccaacgtg ctcgtctacg agggcggctc cgtgacgtgg ctgcctccgg ccatctaccg 600

cagcgtctgc gcagtggagg tcacctactt ccccttcgat tggcagaact gttcgcttat 660

tttccgctct cagacgtaca atgccgaaga ggtggagttc acttttgccg tagacaacga 720

cggcaagacc atcaacaaga tcgacatcga cacagaggcc tatactgaga acggcgagtg 780

ggccatcgac ttctgcccgg gggtgatccg ccgccaccac ggtggcgcca ccgacggccc 840

aggggagact gacgtcatct actcgctcat catccgccgg aagccgctct tctacgtcat 900

taacatcatc gtgccctgtg tgctcatctc gggcctggtg ctgctcgcct acttcctgcc 960

ggcgcaggcc ggcggccaga aatgcacggt ctccatcaac gtcctgctcg cccagaccgt 1020

cttcttgttc ctcattgccc agaaaatccc agagacttct ctgagcgtgc cgctcctggg 1080

caggttcctt attttcgtca tggtggtcgc cacgctcatt gtcatgaatt gcgtcatcgt 1140

gctcaacgtg tcccagcgga cgcccaccac ccacgccatg tccccgcggc tgcgccacgt 1200

tctcctggag ctgctgccgc gcctcctggg ctccccgccg ccgcccgagg ccccccgggc 1260

cgcctcgccc ccaaggcggg cgtcgtcggt gggcttattg ctccgcgcgg aggagctgat 1320

actgaaaaag ccacggagcg agctcgtgtt tgaggggcag aggcaccggc aggggacctg 1380

gacggctgcc ttctgccaga gcctgggcgc cgccgccccc gaggtccgct gctgtgtgga 1440

tgccgtgaac ttcgtggccg agagcacgag agatcaggag gccaccggcg aggaagtgtc 1500

cgactgggtg cgcatgggga atgcccttga caacatctgc ttctgggccg ctctggtgct 1560

cttcagcgtg ggctccagcc tcatcttcct cggggcctac ttcaaccgag tgcctgatct 1620

cccctacgcg ccgtgtatcc agccttaact cgagcaccac catcaccatc accatcacta 1680

agtgattaac ctcaggtgca ggctgcctat cagaaggtgg tggctggtgt ggccaatgcc 1740

ctggctcaca aataccactg agatcgatct ttttccctct gccaaaaatt atggggacat 1800

catgaagccc cttgagcatc tgacttctgg ctaataaagg aaatttattt tcattgcaat 1860

agtgtgttgg aattttttgt gtctctcact cggaaggaca tatgggaggg caaatcattt 1920

aaaacatcag aatgagtatt tggtttagag tttggcaaca tatgcccata tgtaactagc 1980

ataacccctt ggggcctcta aacgggtctt gaggggtttt ttgctgaaag catgcggagg 2040

aaattctcct tgaagtttcc ctggtgttca aagtaaagga gtttgcacca gacgcacctc 2100

tgttcactgg tccggcgtat taaaacacga tacattgtta ttagtacatt tattaagcgc 2160

tagattctgt gcgttgttga tttacagaca attgttgtac gtattttaat aattcattaa 2220

atttataatc tttagggtgg tatgttagag cgaaaatcaa atgattttca gcgtctttat 2280

atctgaattt aaatattaaa tcctcaatag atttgtaaaa taggtttcga ttagtttcaa 2340

acaagggttg tttttccgaa ccgatggctg gactatctaa tggattttcg ctcaacgcca 2400

caaaacttgc caaatcttgt agcagcaatc tagctttgtc gatattcgtt tgtgttttgt 2460

tttgtaataa aggttcgacg tcgttcaaaa tattatgcgc ttttgtattt ctttcatcac 2520

tgtcgttagt gtacaattga ctcgacgtaa acacgttaaa tagagcttgg acatatttaa 2580

catcgggcgt gttagcttta ttaggccgat tatcgtcgtc gtcccaaccc tcgtcgttag 2640

aagttgcttc cgaagacgat tttgccatag ccacacgacg cctattaatt gtgtcggcta 2700

acacgtccgc gatcaaattt gtagttgagc tttttggaat tatttctgat tgcgggcgtt 2760

tttgggcggg tttcaatcta actgtgcccg attttaattc agacaacacg ttagaaagcg 2820

atggtgcagg cggtggtaac atttcagacg gcaaatctac taatggcggc ggtggtggag 2880

ctgatgataa atctaccatc ggtggaggcg caggcggggc tggcggcgga ggcggaggcg 2940

gaggtggtgg cggtgatgca gacggcggtt taggctcaaa tgtctcttta ggcaacacag 3000

tcggcacctc aactattgta ctggtttcgg gcgccgtttt tggtttgacc ggtctgagac 3060

gagtgcgatt tttttcgttt ctaatagctt ccaacaattg ttgtctgtcg tctaaaggtg 3120

cagcgggttg aggttccgtc ggcattggtg gagcgggcgg caattcagac atcgatggtg 3180

gtggtggtgg tggaggcgct ggaatgttag gcacgggaga aggtggtggc ggcggtgccg 3240

ccggtataat ttgttctggt ttagtttgtt cgcgcacgat tgtgggcacc ggcgcaggcg 3300

ccgctggctg cacaacggaa ggtcgtctgc ttcgaggcag cgcttggggt ggtggcaatt 3360

caatattata attggaatac aaatcgtaaa aatctgctat aagcattgta atttcgctat 3420

cgtttaccgt gccgatattt aacaaccgct caatgtaagc aattgtattg taaagagatt 3480

gtctcaagct cggaacgctg cgctcggtcg ttcggctgcg gcgagcggta tcagctcact 3540

caaaggcggt aatacggtta tccacagaat caggggataa cgcaggaaag aacatgtgag 3600

caaaaggcca gcaaaaggcc aggaaccgta aaaaggccgc gttgctggcg tttttccata 3660

ggctccgccc ccctgacgag catcacaaaa atcgacgctc aagtcagagg tggcgaaacc 3720

cgacaggact ataaagatac caggcgtttc cccctggaag ctccctcgtg cgctctcctg 3780

ttccgaccct gccgcttacc ggatacctgt ccgcctttct cccttcggga agcgtggcgc 3840

tttctcaatg ctcacgctgt aggtatctca gttcggtgta ggtcgttcgc tccaagctgg 3900

gctgtgtgca cgaacccccc gttcagcccg accgctgcgc cttatccggt aactatcgtc 3960

ttgagtccaa cccggtaaga cacgacttat cgccactggc agcagccact ggtaacagga 4020

ttagcagagc gaggtatgta ggcggtgcta cagagttctt gaagtggtgg cctaactacg 4080

gctacactag aaggacagta tttggtatct gcgctctgct gaagccagtt accttcggaa 4140

aaagagttgg tagctcttga tccggcaaac aaaccaccgc tggtagcggt ggtttttttg 4200

tttgcaagca gcagattacg cgcagaaaaa aaggatctca agaagatcct ttgttaccaa 4260

tgcttaatca gtgaggcacc tatctcagcg atctgtctat ttcgttcatc catagttgcc 4320

tgactccccg tcgtgtagat aactacgata cgggagggct taccatctgg ccccagtgct 4380

gcaatgatac cgcgagaccc acgctcaccg gctccagatt tatcagcaat aaaccagcca 4440

gccggaaggg ccgagcgcag aagtggtcct gcaactttat ccgcctccat ccagtctatt 4500

aattgttgcc gggaagctag agtaagtagt tcgccagtta atagtttgcg caacgttgtt 4560

gccattgcta caggcatcgt ggtgtcacgc tcgtcgtttg gtatggcttc attcagctcc 4620

ggttcccaac gatcaaggcg agttacatga tcccccatgt tgtgcaaaaa agcggttagc 4680

tccttcggtc ctccgatcgt tgtcagaagt aagttggccg cagtgttatc actcatggtt 4740

atggcagcac tgcataattc tcttactgtc atgccatccg taagatgctt ttctgtgact 4800

ggtgagtact caaccaagtc attctgagaa tagtgtatgc ggcgaccgag ttgctcttgc 4860

ccggcgtcaa tacgggataa taccgcgcca catagcagaa ctttaaaagt gctcatcatt 4920

ggaaaacgtt cttcggggcg aaaactctca aggatcttac cgctgttgag atccagttcg 4980

atgtaaccca ctcgtgcacc caactgatct tcagcatctt ttactttcac cagcgtttct 5040

gggtgagcaa aaacaggaag gcaaaatgcc gcaaaaaagg gaataagggc gacacggaaa 5100

tgttgaatac tcatactctt cctttttcaa tattattgaa gcatttatca gggttattgt 5160

ctcatgtccg cgcgtttcct gcatctttta atcaaatccc aagatgtgta taaaccacca 5220

aactgccaaa aaatgaaaac tgtcgacaag ctctgtccgt ttgctggcaa ctgcaagggt 5280

ctcaatccta tttgtaatta ttgaataata aaacaattat aaatgtcaaa tttgtttttt 5340

attaacgata caaaccaaac gcaacaagaa catttgtagt attatctata attgaaaacg 5400

cgtagttata atcgctgagg taatatttaa aatcattttc aaatgattca cagttaattt 5460

gcgacaatat aattttattt tcacataaac tagacgcctt gtcgtcttct tcttcgtatt 5520

ccttctcttt ttcatttttc tcttcataaa aattaacata gttattatcg tatccatata 5580

tgtatctatc gtatagagta aattttttgt tgtcataaat atatatgtct tttttaatgg 5640

ggtgtatagt accgctgcgc atagtttttc tgtaatttac aacagtgcta ttttctggta 5700

gttcttcgga gtgtgttgct ttaattatta aatttatata atcaatgaat ttgggatcgt 5760

cggttttgta caatatgttg ccggcatagt acgcagcttc ttctagttca attacaccat 5820

tttttagcag caccggatta acataacttt ccaaaatgtt gtacgaaccg ttaaacaaaa 5880

acagttcacc tcccttttct atactattgt ctgcgagcag ttgtttgttg ttaaaaataa 5940

cagccattgt aatgagacgc acaaactaat atcacaaact ggaaatgtct atcaatatat 6000

agttgctcta gttattaata gtaatcaatt acggggtcat tagttcatag cccatatatg 6060

gagttccgcg ttacataact tacggtaaat ggcccgcctg gctgaccgcc caacgacccc 6120

cgcccattga cgtcaataat gacgtatgtt cccatagtaa cgccaatagg gactttccat 6180

tgacgtcaat gggtggacta tttacggtaa actgcccact tggcagtaca tcaagtgtat 6240

catatgccaa gtacgccccc tattgacgtc aatgacggta aatggcccgc ctggcattat 6300

gcccagtaca tgaccttatg ggactttcct acttggcagt acatctacgt attagtcatc 6360

gctattacca tgcatggtcg aggtgagccc cacgttctgc ttcactctcc ccatctcccc 6420

cccctcccca cccccaattt tgtatttatt tattttttaa ttattttgtg cagcgatggg 6480

ggcggggggg gggggggggc gcgcgccagg cggggcgggg cggggcgagg ggcggggcgg 6540

ggcgaggcgg agaggtgcgg cggcagccaa tcagagcggc gcgctccgaa agtttccttt 6600

tatggcgagg cggcggcggc ggcggcccta taaaaagcga agcgcgcggc gggcgggagt 6660

cgctgcgacg ctgccttcgc cccgtgcccc gctccgccgc cgcctcgcgc cgcccgcccc 6720

ggctctgact gaccgcgtta ctcccacagg tgagcgggcg ggacggccct tctccttcgg 6780

gctgtaatta gcgcttggtt taatgacggc ttgtttcttt tctgtggctg cgtgaaagcc 6840

ttgaggggct ccgggagggc cctttgtgcg gggggagcgg ctcggggctg tccgcggggg 6900

gacggctgcc ttcggggggg acggggcagg gcggggttcg gcttctggcg tgtgaccggc 6960

ggctctagag cctctgctaa ccatgttcat gccttcttct ttttcctaca gctcctgggc 7020

aacgtgctgg ttattgtgct gtctcatcat tttggcaaag aattggatcg gaccgaaatt 7080

aatacgactc actata 7096

<210> 11
<211> 7168
<212> DNA
<213> Artificial Sequence

<220>
<223> pTriEx-1-CHRNG

<400> 11
ggggaattgt gagcggataa caattccccg gagttaatcc gggaccttta attcaaccca 60

acacaatata ttatagttaa ataagaatta ttatcaaatc atttgtatat taattaaaat 120

actatactgt aaattacatt ttatttacaa tcaaaggaga tataccatgc atgggggcca 180

ggggccgctg ctcctcctgc tgctgctggc tgtctgcctg ggggcccagg gccggaacca 240

ggaggagcgc ctgctcgcag acctgatgca aaactacgac cccaacctgc ggcccgcgga 300

acgagactcg gatgtggtca atgtcagcct gaagctaacc ctcaccaacc tcatctccct 360

gaacgagcga gaggaagccc tcaccaccaa tgtctggata gagatgcagt ggtgcgacta 420

tcgcctgcgc tgggatccgc gagactacga aggcctgtgg gtgctgaggg tgccgtccac 480

catggtgtgg cggccggata tcgtgctgga gaataacgtg gacggtgtct tcgaggtggc 540

cctctactgc aatgtgctcg tgtcccctga cggctgtatc tactggctgc cgcctgccat 600

cttccgttcc gcctgctcta tctcagtcac ctacttcccc ttcgactggc agaactgctc 660

ccttatcttc cagtcccaga cttacagcac caatgagatt gatctgcagc tgagtcagga 720

agatggccag accatcgagt ggattttcat tgaccctgag gccttcacag agaatgggga 780

gtgggccatc cagcaccgac cagccaagat gctcctggac ccagcggcgc cagcccagga 840

agcaggccac cagaaggtgg tgttctacct gctcatccag cgcaagcccc tcttctacgt 900

catcaacatc atcgccccct gtgtgctcat ctcctctgtc gccatcctca tccacttcct 960

tcctgccaag gctgggggcc agaagtgtac cgtcgccatc aacgtgctcc tggcccagac 1020

tgtcttcctc ttccttgtgg ccaagaaggt gcctgaaacc tcccaggcgg tgccactcat 1080

cagcaagtac ctgaccttcc tcctggtggt gaccatcctc attgtcgtga atgctgtggt 1140

tgtgctcaat gtctccttgc ggtctccaca cacacactcc atggcccgag gggtccgcaa 1200

ggtgttcctg aggctcttgc cccagctgct gaggatgcac gttcgcccgc tggccccggc 1260

agctgtgcag gacacccagt cccggctaca gaatggctcc tcgggatggt cgatcacaac 1320

tggggaggag gtggccctct gcctgcctcg cagtgaactc ctcttccagc agtggcagcg 1380

gcaagggctg gtggcggcag cgctggagaa gctagagaaa ggcccggagt tagggctgag 1440

ccagttctgt ggcagcctga agcaggctgc cccagccatc caggcctgtg tggaagcctg 1500

caacctcatt gcctgtgccc ggcaccagca gagtcacttt gacaatggga atgaggagtg 1560

gttcctggtg ggccgagtgc tggaccgtgt ctgcttcctg gccatgctct cgctcttcat 1620

ctgtggcaca gctggcatct tcctcatggc ccactacaac cgggtgccgg ccctgccatt 1680

ccctggagat ccacgcccct acctgccctc accagactga ctcgagcacc accatcacca 1740

tcaccatcac taagtgatta acctcaggtg caggctgcct atcagaaggt ggtggctggt 1800

gtggccaatg ccctggctca caaataccac tgagatcgat ctttttccct ctgccaaaaa 1860

ttatggggac atcatgaagc cccttgagca tctgacttct ggctaataaa ggaaatttat 1920

tttcattgca atagtgtgtt ggaatttttt gtgtctctca ctcggaagga catatgggag 1980

ggcaaatcat ttaaaacatc agaatgagta tttggtttag agtttggcaa catatgccca 2040

tatgtaacta gcataacccc ttggggcctc taaacgggtc ttgaggggtt ttttgctgaa 2100

agcatgcgga ggaaattctc cttgaagttt ccctggtgtt caaagtaaag gagtttgcac 2160

cagacgcacc tctgttcact ggtccggcgt attaaaacac gatacattgt tattagtaca 2220

tttattaagc gctagattct gtgcgttgtt gatttacaga caattgttgt acgtatttta 2280

ataattcatt aaatttataa tctttagggt ggtatgttag agcgaaaatc aaatgatttt 2340

cagcgtcttt atatctgaat ttaaatatta aatcctcaat agatttgtaa aataggtttc 2400

gattagtttc aaacaagggt tgtttttccg aaccgatggc tggactatct aatggatttt 2460

cgctcaacgc cacaaaactt gccaaatctt gtagcagcaa tctagctttg tcgatattcg 2520

tttgtgtttt gttttgtaat aaaggttcga cgtcgttcaa aatattatgc gcttttgtat 2580

ttctttcatc actgtcgtta gtgtacaatt gactcgacgt aaacacgtta aatagagctt 2640

ggacatattt aacatcgggc gtgttagctt tattaggccg attatcgtcg tcgtcccaac 2700

cctcgtcgtt agaagttgct tccgaagacg attttgccat agccacacga cgcctattaa 2760

ttgtgtcggc taacacgtcc gcgatcaaat ttgtagttga gctttttgga attatttctg 2820

attgcgggcg tttttgggcg ggtttcaatc taactgtgcc cgattttaat tcagacaaca 2880

cgttagaaag cgatggtgca ggcggtggta acatttcaga cggcaaatct actaatggcg 2940

gcggtggtgg agctgatgat aaatctacca tcggtggagg cgcaggcggg gctggcggcg 3000

gaggcggagg cggaggtggt ggcggtgatg cagacggcgg tttaggctca aatgtctctt 3060

taggcaacac agtcggcacc tcaactattg tactggtttc gggcgccgtt tttggtttga 3120

ccggtctgag acgagtgcga tttttttcgt ttctaatagc ttccaacaat tgttgtctgt 3180

cgtctaaagg tgcagcgggt tgaggttccg tcggcattgg tggagcgggc ggcaattcag 3240

acatcgatgg tggtggtggt ggtggaggcg ctggaatgtt aggcacggga gaaggtggtg 3300

gcggcggtgc cgccggtata atttgttctg gtttagtttg ttcgcgcacg attgtgggca 3360

ccggcgcagg cgccgctggc tgcacaacgg aaggtcgtct gcttcgaggc agcgcttggg 3420

gtggtggcaa ttcaatatta taattggaat acaaatcgta aaaatctgct ataagcattg 3480

taatttcgct atcgtttacc gtgccgatat ttaacaaccg ctcaatgtaa gcaattgtat 3540

tgtaaagaga ttgtctcaag ctcggaacgc tgcgctcggt cgttcggctg cggcgagcgg 3600

tatcagctca ctcaaaggcg gtaatacggt tatccacaga atcaggggat aacgcaggaa 3660

agaacatgtg agcaaaaggc cagcaaaagg ccaggaaccg taaaaaggcc gcgttgctgg 3720

cgtttttcca taggctccgc ccccctgacg agcatcacaa aaatcgacgc tcaagtcaga 3780

ggtggcgaaa cccgacagga ctataaagat accaggcgtt tccccctgga agctccctcg 3840

tgcgctctcc tgttccgacc ctgccgctta ccggatacct gtccgccttt ctcccttcgg 3900

gaagcgtggc gctttctcaa tgctcacgct gtaggtatct cagttcggtg taggtcgttc 3960

gctccaagct gggctgtgtg cacgaacccc ccgttcagcc cgaccgctgc gccttatccg 4020

gtaactatcg tcttgagtcc aacccggtaa gacacgactt atcgccactg gcagcagcca 4080

ctggtaacag gattagcaga gcgaggtatg taggcggtgc tacagagttc ttgaagtggt 4140

ggcctaacta cggctacact agaaggacag tatttggtat ctgcgctctg ctgaagccag 4200

ttaccttcgg aaaaagagtt ggtagctctt gatccggcaa acaaaccacc gctggtagcg 4260

gtggtttttt tgtttgcaag cagcagatta cgcgcagaaa aaaaggatct caagaagatc 4320

ctttgttacc aatgcttaat cagtgaggca cctatctcag cgatctgtct atttcgttca 4380

tccatagttg cctgactccc cgtcgtgtag ataactacga tacgggaggg cttaccatct 4440

ggccccagtg ctgcaatgat accgcgagac ccacgctcac cggctccaga tttatcagca 4500

ataaaccagc cagccggaag ggccgagcgc agaagtggtc ctgcaacttt atccgcctcc 4560

atccagtcta ttaattgttg ccgggaagct agagtaagta gttcgccagt taatagtttg 4620

cgcaacgttg ttgccattgc tacaggcatc gtggtgtcac gctcgtcgtt tggtatggct 4680

tcattcagct ccggttccca acgatcaagg cgagttacat gatcccccat gttgtgcaaa 4740

aaagcggtta gctccttcgg tcctccgatc gttgtcagaa gtaagttggc cgcagtgtta 4800

tcactcatgg ttatggcagc actgcataat tctcttactg tcatgccatc cgtaagatgc 4860

ttttctgtga ctggtgagta ctcaaccaag tcattctgag aatagtgtat gcggcgaccg 4920

agttgctctt gcccggcgtc aatacgggat aataccgcgc cacatagcag aactttaaaa 4980

gtgctcatca ttggaaaacg ttcttcgggg cgaaaactct caaggatctt accgctgttg 5040

agatccagtt cgatgtaacc cactcgtgca cccaactgat cttcagcatc ttttactttc 5100

accagcgttt ctgggtgagc aaaaacagga aggcaaaatg ccgcaaaaaa gggaataagg 5160

gcgacacgga aatgttgaat actcatactc ttcctttttc aatattattg aagcatttat 5220

cagggttatt gtctcatgtc cgcgcgtttc ctgcatcttt taatcaaatc ccaagatgtg 5280

tataaaccac caaactgcca aaaaatgaaa actgtcgaca agctctgtcc gtttgctggc 5340

aactgcaagg gtctcaatcc tatttgtaat tattgaataa taaaacaatt ataaatgtca 5400

aatttgtttt ttattaacga tacaaaccaa acgcaacaag aacatttgta gtattatcta 5460

taattgaaaa cgcgtagtta taatcgctga ggtaatattt aaaatcattt tcaaatgatt 5520

cacagttaat ttgcgacaat ataattttat tttcacataa actagacgcc ttgtcgtctt 5580

cttcttcgta ttccttctct tttcattttt tctcttcata aaaattaaca tagttattat 5640

cgtatccata tatgtatcta tcgtatagag taaatttttt gttgtcataa atatatatgt 5700

cttttttaat ggggtgtata gtaccgctgc gcatagtttt tctgtaattt acaacagtgc 5760

tattttctgg tagttcttcg gagtgtgttg ctttaattat taaatttata taatcaatga 5820

atttgggatc gtcggttttg tacaatatgt tgccggcata gtacgcagct tcttctagtt 5880

caattacacc attttttagc agcaccggat taacataact ttccaaaatg ttgtacgaac 5940

cgttaaacaa aaacagttca cctccctttt ctatactatt gtctgcgagc agttgtttgt 6000

tgttaaaaat aacagccatt gtaatgagac gcacaaacta atatcacaaa ctggaaatgt 6060

ctatcaatat atagttgctc tagttattaa tagtaatcaa ttacggggtc attagttcat 6120

agcccatata tggagttccg cgttacataa cttacggtaa atggcccgcc tggctgaccg 6180

cccaacgacc cccgcccatt gacgtcaata atgacgtatg ttcccatagt aacgccaata 6240

gggactttcc attgacgtca atgggtggac tatttacggt aaactgccca cttggcagta 6300

catcaagtgt atcatatgcc aagtacgccc cctattgacg tcaatgacgg taaatggccc 6360

gcctggcatt atgcccagta catgacctta tgggactttc ctacttggca gtacatctac 6420

gtattagtca tcgctattac catgcatggt cgaggtgagc cccacgttct gcttcactct 6480

ccccatctcc cccccctccc cacccccaat tttgtattta tttatttttt aattattttg 6540

tgcagcgatg ggggcggggg gggggggggg gcgcgcgcca ggcggggcgg ggcggggcga 6600

ggggcggggc ggggcgaggc ggagaggtgc ggcggcagcc aatcagagcg gcgcgctccg 6660

aaagtttcct tttatggcga ggcggcggcg gcggcggccc tataaaaagc gaagcgcgcg 6720

gcgggcggga gtcgctgcga cgctgccttc gccccgtgcc ccgctccgcc gccgcctcgc 6780

gccgcccgcc ccggctctga ctgaccgcgt tactcccaca ggtgagcggg cgggacggcc 6840

cttctccttc gggctgtaat tagcgcttgg tttaatgacg gcttgtttct tttctgtggc 6900

tgcgtgaaag ccttgagggg ctccgggagg gccctttgtg cggggggagc ggctcggggc 6960

tgtccgcggg gggacggctg ccttcggggg ggacggggca gggcggggtt cggcttctgg 7020

cgtgtgaccg gcggctctag agcctctgct aaccatgttc atgccttctt ctttttccta 7080

cagctcctgg gcaacgtgct ggttattgtg ctgtctcatc attttggcaa agaattggat 7140

cggaccgaaa ttaatacgac tcactata 7168

<210> 12
<211> 6853
<212> DNA
<213> Artificial Sequence

<220>
<223> pTriEx-1-RAPSyn

<400> 12
ggggaattgt gagcggataa caattccccg gagttaatcc gggaccttta attcaaccca 60

acacaatata ttatagttaa ataagaatta ttatcaaatc atttgtatat taattaaaat 120

actatactgt aaattacatt ttatttacaa tcaaaggaga tataccatgg ggcaggacca 180

gaccaagcag cagatcgaga aggggctcca gctgtaccag tccaaccaga cagagaaggc 240

attgcaggtg tggacaaagg tgctggagaa gagctcggac ctcatggggc gcttccgcgt 300

gctgggctgc ctggtcacag cccactcgga gatgggccgc tacaaggaga tgctgaagtt 360

cgctgtggtc cagatcgaca cggcccggga gctggaggat gccgacttcc tcctggagag 420

ctacctgaac ctggcacgca gcaacgagaa gctgtgcgag tttcacaaga ccatctccta 480

ctgcaagacc tgccttgggc tgcctggtac cagggcaggt gcccagctcg gaggccaggt 540

cagcctgagc atgggcaatg ccttcctggg cctcagcgtc ttccagaagg ccctggagag 600

cttcgagaag gccctgcgct atgcccacaa caatgatgac gccatgctcg agtgccgcgt 660

gtgctgcagc ctgggcagct tctatgccca ggtcaaggac tacgagaaag ccctgttctt 720

cccctgcaag gcggcagagc ttgtcaacaa ctatggcaaa ggctggagcc tgaagtaccg 780

ggccatgagc cagtaccaca tggccgtggc ctatcgcctg ctgggccgcc tgggcagtgc 840

catggagtgt tgtgaggagt ctatgaagat cgcgctgcag cacggggacc ggccactgca 900

ggcgctctgc ctgctctgct tcgctgacat ccaccggagc cgtggggacc tggagacagc 960

cttccccagg tacgactccg ccatgagcat catgaccgag atcggaaacc gcctggggca 1020

ggtgcaggcg ctgctgggtg tggccaagtg ctgggtggcc aggaaggcgc tggacaaggc 1080

tctggatgcc atcgagagag cccaggatct ggccgaggag gtggggaaca agctgagcca 1140

gctcaagctg cactgtctga gcgagagcat ttaccgcagc aaagggctgc agcgggaact 1200

gcgggcgcac gttgtgaggt tccacgagtg cgtggaggag acggagctct actgcggcct 1260

gtgcggcgag tccataggcg agaagaacag ccggctgcag gccctacctt gctcccacat 1320

cttccacctc aggtgcctgc agaacaacgg gacccggagc tgtcccaact gccgccgctc 1380

atccatgaag cctggctttg tataactcga gcaccaccat caccatcacc atcactaagt 1440

gattaacctc aggtgcaggc tgcctatcag aaggtggtgg ctggtgtggc caatgccctg 1500

gctcacaaat accactgaga tcgatctttt tccctctgcc aaaaattatg gggacatcat 1560

gaagcccctt gagcatctga cttctggcta ataaaggaaa tttattttca ttgcaatagt 1620

gtgttggaat tttttgtgtc tctcactcgg aaggacatat gggagggcaa atcatttaaa 1680

acatcagaat gagtatttgg tttagagttt ggcaacatat gcccatatgt aactagcata 1740

accccttggg gcctctaaac gggtcttgag gggttttttg ctgaaagcat gcggaggaaa 1800

ttctccttga agtttccctg gtgttcaaag taaaggagtt tgcaccagac gcacctctgt 1860

tcactggtcc ggcgtattaa aacacgatac attgttatta gtacatttat taagcgctag 1920

attctgtgcg ttgttgattt acagacaatt gttgtacgta ttttaataat tcattaaatt 1980

tataatcttt agggtggtat gttagagcga aaatcaaatg attttcagcg tctttatatc 2040

tgaatttaaa tattaaatcc tcaatagatt tgtaaaatag gtttcgatta gtttcaaaca 2100

agggttgttt ttccgaaccg atggctggac tatctaatgg attttcgctc aacgccacaa 2160

aacttgccaa atcttgtagc agcaatctag ctttgtcgat attcgtttgt gttttgtttt 2220

gtaataaagg ttcgacgtcg ttcaaaatat tatgcgcttt tgtatttctt tcatcactgt 2280

cgttagtgta caattgactc gacgtaaaca cgttaaatag agcttggaca tatttaacat 2340

cgggcgtgtt agctttatta ggccgattat cgtcgtcgtc ccaaccctcg tcgttagaag 2400

ttgcttccga agacgatttt gccatagcca cacgacgcct attaattgtg tcggctaaca 2460

cgtccgcgat caaatttgta gttgagcttt ttggaattat ttctgattgc gggcgttttt 2520

gggcgggttt caatctaact gtgcccgatt ttaattcaga caacacgtta gaaagcgatg 2580

gtgcaggcgg tggtaacatt tcagacggca aatctactaa tggcggcggt ggtggagctg 2640

atgataaatc taccatcggt ggaggcgcag gcggggctgg cggcggaggc ggaggcggag 2700

gtggtggcgg tgatgcagac ggcggtttag gctcaaatgt ctctttaggc aacacagtcg 2760

gcacctcaac tattgtactg gtttcgggcg ccgtttttgg tttgaccggt ctgagacgag 2820

tgcgattttt ttcgtttcta atagcttcca acaattgttg tctgtcgtct aaaggtgcag 2880

cgggttgagg ttccgtcggc attggtggag cgggcggcaa ttcagacatc gatggtggtg 2940

gtggtggtgg aggcgctgga atgttaggca cgggagaagg tggtggcggc ggtgccgccg 3000

gtataatttg ttctggttta gtttgttcgc gcacgattgt gggcaccggc gcaggcgccg 3060

ctggctgcac aacggaaggt cgtctgcttc gaggcagcgc ttggggtggt ggcaattcaa 3120

tattataatt ggaatacaaa tcgtaaaaat ctgctataag cattgtaatt tcgctatcgt 3180

ttaccgtgcc gatatttaac aaccgctcaa tgtaagcaat tgtattgtaa agagattgtc 3240

tcaagctcgg aacgctgcgc tcggtcgttc ggctgcggcg agcggtatca gctcactcaa 3300

aggcggtaat acggttatcc acagaatcag gggataacgc aggaaagaac atgtgagcaa 3360

aaggccagca aaaggccagg aaccgtaaaa aggccgcgtt gctggcgttt ttccataggc 3420

tccgcccccc tgacgagcat cacaaaaatc gacgctcaag tcagaggtgg cgaaacccga 3480

caggactata aagataccag gcgtttcccc ctggaagctc cctcgtgcgc tctcctgttc 3540

cgaccctgcc gcttaccgga tacctgtccg cctttctccc ttcgggaagc gtggcgcttt 3600

ctcaatgctc acgctgtagg tatctcagtt cggtgtaggt cgttcgctcc aagctgggct 3660

gtgtgcacga accccccgtt cagcccgacc gctgcgcctt atccggtaac tatcgtcttg 3720

agtccaaccc ggtaagacac gacttatcgc cactggcagc agccactggt aacaggatta 3780

gcagagcgag gtatgtaggc ggtgctacag agttcttgaa gtggtggcct aactacggct 3840

acactagaag gacagtattt ggtatctgcg ctctgctgaa gccagttacc ttcggaaaaa 3900

gagttggtag ctcttgatcc ggcaaacaaa ccaccgctgg tagcggtggt ttttttgttt 3960

gcaagcagca gattacgcgc agaaaaaaag gatctcaaga agatcctttg ttaccaatgc 4020

ttaatcagtg aggcacctat ctcagcgatc tgtctatttc gttcatccat agttgcctga 4080

ctccccgtcg tgtagataac tacgatacgg gagggcttac catctggccc cagtgctgca 4140

atgataccgc gagacccacg ctcaccggct ccagatttat cagcaataaa ccagccagcc 4200

ggaagggccg agcgcagaag tggtcctgca actttatccg cctccatcca gtctattaat 4260

tgttgccggg aagctagagt aagtagttcg ccagttaata gtttgcgcaa cgttgttgcc 4320

attgctacag gcatcgtggt gtcacgctcg tcgtttggta tggcttcatt cagctccggt 4380

tcccaacgat caaggcgagt tacatgatcc cccatgttgt gcaaaaaagc ggttagctcc 4440

ttcggtcctc cgatcgttgt cagaagtaag ttggccgcag tgttatcact catggttatg 4500

gcagcactgc ataattctct tactgtcatg ccatccgtaa gatgcttttc tgtgactggt 4560

gagtactcaa ccaagtcatt ctgagaatag tgtatgcggc gaccgagttg ctcttgcccg 4620

gcgtcaatac gggataatac cgcgccacat agcagaactt taaaagtgct catcattgga 4680

aaacgttctt cggggcgaaa actctcaagg atcttaccgc tgttgagatc cagttcgatg 4740

taacccactc gtgcacccaa ctgatcttca gcatctttta ctttcaccag cgtttctggg 4800

tgagcaaaaa caggaaggca aaatgccgca aaaaagggaa taagggcgac acggaaatgt 4860

tgaatactca tactcttcct ttttcaatat tattgaagca tttatcaggg ttattgtctc 4920

atgtccgcgc gtttcctgca tcttttaatc aaatcccaag atgtgtataa accaccaaac 4980

tgccaaaaaa tgaaaactgt cgacaagctc tgtccgtttg ctggcaactg caagggtctc 5040

aatcctattt gtaattattg aataataaaa caattataaa tgtcaaattt gttttttatt 5100

aacgatacaa accaaacgca acaagaacat ttgtagtatt atctataatt gaaaacgcgt 5160

agttataatc gctgaggtaa tatttaaaat cattttcaaa tgattcacag ttaatttgcg 5220

acaatataat tttattttca cataaactag acgccttgtc gtcttcttct tcgtattcct 5280

tctctttttc atttttctct tcataaaaat taacatagtt attatcgtat ccatatatgt 5340

atctatcgta tagagtaaat tttttgttgt cataaatata tatgtctttt ttaatggggt 5400

gtatagtacc gctgcgcata gtttttctgt aatttacaac agtgctattt tctggtagtt 5460

cttcggagtg tgttgcttta attattaaat ttatataatc aatgaatttg ggatcgtcgg 5520

ttttgtacaa tatgttgccg gcatagtacg cagcttcttc tagttcaatt acaccatttt 5580

ttagcagcac cggattaaca taactttcca aaatgttgta cgaaccgtta aacaaaaaca 5640

gttcacctcc cttttctata ctattgtctg cgagcagttg tttgttgtta aaaataacag 5700

ccattgtaat gagacgcaca aactaatatc acaaactgga aatgtctatc aatatatagt 5760

tgctctagtt attaatagta atcaattacg gggtcattag ttcatagccc atatatggag 5820

ttccgcgtta cataacttac ggtaaatggc ccgcctggct gaccgcccaa cgacccccgc 5880

ccattgacgt caataatgac gtatgttccc atagtaacgc caatagggac tttccattga 5940

cgtcaatggg tggactattt acggtaaact gcccacttgg cagtacatca agtgtatcat 6000

atgccaagta cgccccctat tgacgtcaat gacggtaaat ggcccgcctg gcattatgcc 6060

cagtacatga ccttatggga ctttcctact tggcagtaca tctacgtatt agtcatcgct 6120

attaccatgc atggtcgagg tgagccccac gttctgcttc actctcccca tctccccccc 6180

ctccccaccc ccaattttgt atttatttat tttttaatta ttttgtgcag cgatgggggc 6240

gggggggggg ggggggcgcg cgccaggcgg ggcggggcgg ggcgaggggc ggggcggggc 6300

gaggcggaga ggtgcggcgg cagccaatca gagcggcgcg ctccgaaagt ttccttttat 6360

ggcgaggcgg cggcggcggc ggccctataa aaagcgaagc gcgcggcggg cgggagtcgc 6420

tgcgacgctg ccttcgcccc gtgccccgct ccgccgccgc ctcgcgccgc ccgccccggc 6480

tctgactgac cgcgttactc ccacaggtga gcgggcggga cggcccttct ccttcgggct 6540

gtaattagcg cttggtttaa tgacggcttg tttcttttct gtggctgcgt gaaagccttg 6600

aggggctccg ggagggccct ttgtgcgggg ggagcggctc ggggctgtcc gcggggggac 6660

ggctgccttc gggggggacg gggcagggcg gggttcggct tctggcgtgt gaccggcggc 6720

tctagagcct ctgctaacca tgttcatgcc ttcttctttt tcctacagct cctgggcaac 6780

gtgctggtta ttgtgctgtc tcatcatttt ggcaaagaat tggatcggac cgaaattaat 6840

acgactcact ata                                                        6853

<210>  13
<211>  861
<212>  PRT
<213>  Homo sapiens

<400>  13
   Met Arg Glu Leu Val Asn Ile Pro Leu Val His Ile Leu Thr Leu Val
   1               5                   10                  15
   Ala Phe Ser Gly Thr Glu Lys Leu Pro Lys Ala Pro Val Ile Thr Thr
               20                  25                  30
   Pro Leu Glu Thr Val Asp Ala Leu Val Glu Glu Val Ala Thr Phe Met
           35                  40                  45
   Cys Ala Val Glu Ser Tyr Pro Gln Pro Glu Ile Ser Trp Thr Arg Asn
       50                  55                  60
   Lys Ile Leu Ile Lys Leu Phe Asp Thr Arg Tyr Ser Ile Arg Glu Asn
   65                  70                  75                  80
   Gly Gln Leu Leu Thr Ile Leu Ser Val Glu Asp Ser Asp Asp Gly Ile
                   85                  90                  95
   Tyr Cys Cys Thr Ala Asn Asn Gly Val Gly Gly Ala Val Glu Ser Cys
               100                 105                 110
   Gly Ala Leu Gln Val Lys Met Lys Pro Lys Ile Thr Arg Pro Pro Ile
           115                 120                 125
   Asn Val Lys Ile Ile Glu Gly Leu Lys Ala Val Leu Pro Cys Thr Thr
       130                 135                 140
   Met Gly Asn Pro Lys Pro Ser Val Ser Trp Ile Lys Gly Asp Ser Pro
   145                 150                 155                 160
   Leu Arg Glu Asn Ser Arg Ile Ala Val Leu Glu Ser Gly Ser Leu Arg
                   165                 170                 175
   Ile His Asn Val Gln Lys Glu Asp Ala Gly Gln Tyr Arg Cys Val Ala
               180                 185                 190
   Lys Asn Ser Leu Gly Thr Ala Tyr Ser Lys Val Val Lys Leu Glu Val
           195                 200                 205
   Glu Val Phe Ala Arg Ile Leu Arg Ala Pro Glu Ser His Asn Val Thr
   210                 215                 220
   Phe Gly Ser Phe Val Thr Leu His Cys Thr Ala Thr Gly Ile Pro Val
   225                 230                 235                 240
   Pro Thr Ile Thr Trp Ile Glu Asn Gly Asn Ala Val Ser Ser Gly Ser
                   245                 250                 255
   Ile Gln Glu Ser Val Lys Asp Arg Val Ile Asp Ser Arg Leu Gln Leu
               260                 265                 270
   Phe Ile Thr Lys Pro Gly Leu Tyr Thr Cys Ile Ala Thr Asn Lys His
           275                 280                 285
   Gly Glu Lys Phe Ser Thr Ala Lys Ala Ala Ala Thr Ile Ser Ile Ala
       290                 295                 300
   Glu Trp Ser Lys Pro Gln Lys Asp Asn Lys Gly Tyr Cys Ala Gln Tyr
   305                 310                 315                 320
   Arg Gly Glu Val Cys Asn Ala Val Leu Ala Lys Asp Ala Leu Val Phe
                   325                 330                 335
   Leu Asn Thr Ser Tyr Ala Asp Pro Glu Glu Ala Gln Glu Leu Leu Val
               340                 345                 350
   His Thr Ala Trp Asn Glu Leu Lys Val Val Ser Pro Val Cys Arg Pro
           355                 360                 365
   Ala Ala Glu Ala Leu Leu Cys Asn His Ile Phe Gln Glu Cys Ser Pro
       370                 375                 380
   Gly Val Val Pro Thr Pro Ile Pro Ile Cys Arg Glu Tyr Cys Leu Ala
   385                 390                 395                 400
   Val Lys Glu Leu Phe Cys Ala Lys Glu Trp Leu Val Met Glu Glu Lys
                   405                 410                 415
   Thr His Arg Gly Leu Tyr Arg Ser Glu Met His Leu Leu Ser Val Pro
               420                 425                 430
   Glu Cys Ser Lys Leu Pro Ser Met His Trp Asp Pro Thr Ala Cys Ala
           435                 440                 445
   Arg Leu Pro His Leu Ala Phe Pro Pro Met Thr Ser Ser Lys Pro Ser
       450                 455                 460
   Val Asp Ile Pro Asn Leu Pro Ser Ser Ser Ser Ser Ser Phe Ser Val
   465                 470                 475                 480
   Ser Pro Thr Tyr Ser Met Thr Val Ile Ile Ser Ile Met Ser Ser Phe
                   485                 490                 495
   Ala Ile Phe Val Leu Leu Thr Ile Thr Thr Leu Tyr Cys Cys Arg Arg
               500                 505                 510
   Arg Lys Gln Trp Lys Asn Lys Lys Arg Glu Ser Ala Ala Val Thr Leu
           515                 520                 525
   Thr Thr Leu Pro Ser Glu Leu Leu Leu Asp Arg Leu His Pro Asn Pro
       530                 535                 540
   Met Tyr Gln Arg Met Pro Leu Leu Leu Asn Pro Lys Leu Leu Ser Leu
   545                 550                 555                 560
   Glu Tyr Pro Arg Asn Asn Ile Glu Tyr Val Arg Asp Ile Gly Glu Gly
                   565                 570                 575
   Ala Phe Gly Arg Val Phe Gln Ala Arg Ala Pro Gly Leu Leu Pro Tyr
               580                 585                 590
   Glu Pro Phe Thr Met Val Ala Val Lys Met Leu Lys Glu Glu Ala Ser
           595                 600                 605
   Ala Asp Met Gln Ala Asp Phe Gln Arg Glu Ala Ala Leu Met Ala Glu
       610                 615                 620
   Phe Asp Asn Pro Asn Ile Val Lys Leu Leu Gly Val Cys Ala Val Gly
   625                 630                 635                 640
   Lys Pro Met Cys Leu Leu Phe Glu Tyr Met Ala Tyr Gly Asp Leu Asn
                   645                 650                 655
   Glu Phe Leu Arg Ser Met Ser Pro His Thr Val Cys Ser Leu Ser His
               660                 665                 670
   Ser Asp Leu Ser Met Arg Ala Gln Val Ser Ser Pro Gly Pro Pro Pro
           675                 680                 685
   Leu Ser Cys Ala Glu Gln Leu Cys Ile Ala Arg Gln Val Ala Ala Gly
       690                 695                 700
   Met Ala Tyr Leu Ser Glu Arg Lys Phe Val His Arg Asp Leu Ala Thr
   705                 710                 715                 720
   Arg Asn Cys Leu Val Gly Glu Asn Met Val Val Lys Ile Ala Asp Phe
                   725                 730                 735
   Gly Leu Ser Arg Asn Ile Tyr Ser Ala Asp Tyr Tyr Lys Ala Asn Glu
               740                 745                 750
   Asn Asp Ala Ile Pro Ile Arg Trp Met Pro Pro Glu Ser Ile Phe Tyr
           755                 760                 765
   Asn Arg Tyr Thr Thr Glu Ser Asp Val Trp Ala Tyr Gly Val Val Leu
       770                 775                 780
   Trp Glu Ile Phe Ser Tyr Gly Leu Gln Pro Tyr Tyr Gly Met Ala His
   785                 790                 795                 800
   Glu Glu Val Ile Tyr Tyr Val Arg Asp Gly Asn Ile Leu Ser Cys Pro
                   805                 810                 815
   Glu Asn Cys Pro Val Glu Leu Tyr Asn Leu Met Arg Leu Cys Trp Ser
               820                 825                 830
   Lys Leu Pro Ala Asp Arg Pro Ser Phe Thr Ser Ile His Arg Ile Leu
           835                 840                 845
   Glu Arg Met Cys Glu Arg Ala Glu Gly Thr Val Ser Val
       850                 855                 860

<210>  14
<211>  8200
<212>  DNA
<213>  Artificial Sequence

<220>
<223>  pTriEx-1-MuSK

<400>  14
ggggaattgt gagcggataa caattccccg gagttaatcc gggaccttta attcaaccca      60

acacaatata ttatagttaa ataagaatta ttatcaaatc atttgtatat taattaaaat     120

actatactgt aaattacatt ttatttacaa tcaaaggaga tataccatga gagagctcgt     180

caacattcca ctggtacata ttcttactct ggttgccttc agcggaactg agaaacttcc     240

aaaagctcct gtcatcacca ctcctcttga aacagtggat gccttagttg aagaagtggc     300

tactttcatg tgtgcagtgg aatcctaccc ccagcctgag atttcctgga ctagaaataa     360

aattctcatt aaactctttg acacccggta cagcatccgg gagaatgggc agctcctcac     420

catcctgagt gtggaagaca gtgatgatgg catttactgc tgcacggcca acaatggtgt     480

gggaggagct gtggagagtt gtggagccct gcaagtgaag atgaaaccta aaataactcg     540

tcctcccata aatgtgaaaa taatagaggg attaaaagca gtcctaccat gtactacaat 600

gggtaatccc aaaccatcag tgtcttggat aaagggagac agcccactca gggaaaattc 660

ccgaattgca gttcttgaat ctgggagctt gaggattcat aacgtacaaa aggaagatgc 720

aggacagtat cgatgtgtgg caaaaaacag cctcgggaca gcatattcca aagtggtgaa 780

gctggaagtt gaggtttttg ccaggatcct gcgggctcct gaatcccaca atgtcacctt 840

tggctccttt gtgaccctgc actgtacagc aacaggcatt cctgtcccca ccatcacctg 900

gattgaaaac ggaaatgctg tttcttctgg gtccattcaa gagagtgtga aagaccgagt 960

gattgactca agactgcagc tgtttatcac caagccagga ctctacacat gcatagctac 1020

caataagcat ggggagaagt tcagtactgc caaggctgca gccaccatca gcatagcaga 1080

atggagtaaa ccacagaaag ataacaaagg ctactgcgcc cagtacagag gggaggtgtg 1140

taatgcagtc ctggcaaaag atgctcttgt ttttctcaac acctcctatg cggaccctga 1200

ggaggcccaa gagctactgg tccacacggc ctggaatgaa ctgaaagtag tgagcccagt 1260

ctgccggcca gctgctgagg ctttgttgtg taaccacatc ttccaggagt gcagtcctgg 1320

agtagtgcct actcctattc ccatttgcag agagtactgc ttggcagtaa aggagctctt 1380

ctgcgcaaaa gaatggctgg taatggaaga gaagacccac agaggactct acagatccga 1440

gatgcatttg ctgtccgtgc cagaatgcag caagcttccc agcatgcatt gggaccccac 1500

ggcctgtgcc agactgccac atctagcatt cccaccaatg acgtcctcaa agccaagtgt 1560

ggacattcca aacctgcctt cctcctcctc ttcttccttc tctgtctcac ctacatactc 1620

catgactgta ataatctcca tcatgtccag ctttgcaata tttgtgcttc ttaccataac 1680

tactctctat tgctgccgaa gaagaaaaca atggaaaaat aagaaaagag aatcagcagc 1740

agtaaccctc accacactgc cttctgagct cttactagat agacttcatc ccaaccccat 1800

gtaccagagg atgccgctcc ttctgaaccc caaattgctc agcctggagt atccaaggaa 1860

taacattgaa tatgtgagag acatcggaga gggagcgttt ggaagggtgt ttcaagcaag 1920

ggcaccaggc ttacttccct atgaaccttt cactatggtg gcagtaaaga tgctcaaaga 1980

agaagcctcg gcagatatgc aagcggactt tcagagggag gcagccctca tggcagaatt 2040

tgacaaccct aacattgtga agctattagg agtgtgtgct gtcgggaagc caatgtgcct 2100

gctctttgaa tacatggcct atggtgacct caatgagttc ctccgcagca tgtcccctca 2160

caccgtgtgc agcctcagtc acagtgactt gtctatgagg gctcaggtct ccagccctgg 2220

gcccccaccc ctctcctgtg ctgagcagct ttgcattgcc aggcaggtgg cagctggcat 2280

ggcttacctc tcagaacgta agtttgttca ccgagattta gccaccagga actgcctggt 2340

gggcgagaac atggtggtga aaattgccga ctttggcctc tccaggaaca tctactcagc 2400

agactactac aaagctaatg aaaacgacgc tatccctatc cgttggatgc caccagagtc 2460

cattttttat aaccgctaca ctacagagtc tgatgtgtgg gcctatggcg tggtcctctg 2520

ggagatcttc tcctatggcc tgcagcccta ctatgggatg gcccatgagg aggtcattta 2580

ctacgtgcga gatggcaaca tcctctcctg ccctgagaac tgccccgtgg agctgtacaa 2640

tctcatgcgt ctatgttgga gcaagctgcc tgcagacaga cccagtttca ccagtattca 2700

ccgaattctg gaacgcatgt gtgagagggc agagggaact gtgagtgtct aactcgagca 2760

ccaccatcac catcaccatc actaagtgat taacctcagg tgcaggctgc ctatcagaag 2820

gtggtggctg gtgtggccaa tgccctggct cacaaatacc actgagatcg atctttttcc 2880

ctctgccaaa aattatgggg acatcatgaa gccccttgag catctgactt ctggctaata 2940

aaggaaattt attttcattg caatagtgtg ttggaatttt ttgtgtctct cactcggaag 3000

gacatatggg agggcaaatc atttaaaaca tcagaatgag tatttggttt agagtttggc 3060

aacatatgcc catatgtaac tagcataacc ccttggggcc tctaaacggg tcttgagggg 3120

ttttttgctg aaagcatgcg gaggaaattc tccttgaagt ttccctggtg ttcaaagtaa 3180

aggagtttgc accagacgca cctctgttca ctggtccggc gtattaaaac acgatacatt 3240

gttattagta catttattaa gcgctagatt ctgtgcgttg ttgatttaca gacaattgtt 3300

gtacgtattt taataattca ttaaatttat aatctttagg gtggtatgtt agagcgaaaa 3360

tcaaatgatt ttcagcgtct ttatatctga atttaaatat taaatcctca atagatttgt 3420

aaaataggtt tcgattagtt tcaaacaagg gttgtttttc cgaaccgatg gctggactat 3480

ctaatggatt ttcgctcaac gccacaaaac ttgccaaatc ttgtagcagc aatctagctt 3540

tgtcgatatt cgtttgtgtt ttgttttgta ataaaggttc gacgtcgttc aaaatattat 3600

gcgcttttgt atttctttca tcactgtcgt tagtgtacaa ttgactcgac gtaaacacgt 3660

taaatagagc ttggacatat ttaacatcgg gcgtgttagc tttattaggc cgattatcgt 3720

cgtcgtccca accctcgtcg ttagaagttg cttccgaaga cgattttgcc atagccacac 3780

gacgcctatt aattgtgtcg gctaacacgt ccgcgatcaa atttgtagtt gagctttttg 3840

gaattatttc tgattgcggg cgtttttggg cgggtttcaa tctaactgtg cccgatttta 3900

attcagacaa cacgttagaa agcgatggtg caggcggtgg taacatttca gacggcaaat 3960

ctactaatgg cggcggtggt ggagctgatg ataaatctac catcggtgga ggcgcaggcg 4020

gggctggcgg cggaggcgga ggcggaggtg gtggcggtga tgcagacggc ggtttaggct 4080

caaatgtctc tttaggcaac acagtcggca cctcaactat tgtactggtt tcgggcgccg 4140

tttttggttt gaccggtctg agacgagtgc gatttttttc gtttctaata gcttccaaca 4200

attgttgtct gtcgtctaaa ggtgcagcgg gttgaggttc cgtcggcatt ggtggagcgg 4260

gcggcaattc agacatcgat ggtggtggtg gtggtggagg cgctggaatg ttaggcacgg 4320

gagaaggtgg tggcggcggt gccgccggta taatttgttc tggtttagtt tgttcgcgca 4380

cgattgtggg caccggcgca ggcgccgctg gctgcacaac ggaaggtcgt ctgcttcgag 4440

gcagcgcttg gggtggtggc aattcaatat tataattgga atacaaatcg taaaaatctg 4500

ctataagcat tgtaatttcg ctatcgttta ccgtgccgat atttaacaac cgctcaatgt 4560

aagcaattgt attgtaaaga gattgtctca agctcggaac gctgcgctcg gtcgttcggc 4620

tgcggcgagc ggtatcagct cactcaaagg cggtaatacg gttatccaca gaatcagggg 4680

ataacgcagg aaagaacatg tgagcaaaag gccagcaaaa ggccaggaac cgtaaaaagg 4740

ccgcgttgct ggcgtttttc cataggctcc gcccccctga cgagcatcac aaaaatcgac 4800

gctcaagtca gaggtggcga aacccgacag gactataaag ataccaggcg tttccccctg 4860

gaagctccct cgtgcgctct cctgttccga ccctgccgct taccggatac ctgtccgcct 4920

ttctcccttc gggaagcgtg gcgctttctc aatgctcacg ctgtaggtat ctcagttcgg 4980

tgtaggtcgt tcgctccaag ctgggctgtg tgcacgaacc ccccgttcag cccgaccgct 5040

gcgccttatc cggtaactat cgtcttgagt ccaacccggt aagacacgac ttatcgccac 5100

tggcagcagc cactggtaac aggattagca gagcgaggta tgtaggcggt gctacagagt 5160

tcttgaagtg gtggcctaac tacggctaca ctagaaggac agtatttggt atctgcgctc 5220

tgctgaagcc agttaccttc ggaaaaagag ttggtagctc ttgatccggc aaacaaacca 5280

ccgctggtag cggtggtttt tttgtttgca agcagcagat tacgcgcaga aaaaaaggat 5340

ctcaagaaga tcctttgtta ccaatgctta atcagtgagg cacctatctc agcgatctgt 5400

ctatttcgtt catccatagt tgcctgactc cccgtcgtgt agataactac gatacgggag 5460

ggcttaccat ctggccccag tgctgcaatg ataccgcgag acccacgctc accggctcca 5520

gatttatcag caataaacca gccagccgga agggccgagc gcagaagtgg tcctgcaact 5580

ttatccgcct ccatccagtc tattaattgt tgccgggaag ctagagtaag tagttcgcca 5640

gttaatagtt tgcgcaacgt tgttgccatt gctacaggca tcgtggtgtc acgctcgtcg 5700

tttggtatgg cttcattcag ctccggttcc caacgatcaa ggcgagttac atgatccccc 5760

atgttgtgca aaaaagcggt tagctccttc ggtcctccga tcgttgtcag aagtaagttg 5820

gccgcagtgt tatcactcat ggttatggca gcactgcata attctcttac tgtcatgcca 5880

tccgtaagat gcttttctgt gactggtgag tactcaacca agtcattctg agaatagtgt 5940

atgcggcgac cgagttgctc ttgcccggcg tcaatacggg ataataccgc gccacatagc 6000

agaactttaa aagtgctcat cattggaaaa cgttcttcgg ggcgaaaact ctcaaggatc 6060

ttaccgctgt tgagatccag ttcgatgtaa cccactcgtg cacccaactg atcttcagca 6120

tcttttactt tcaccagcgt ttctgggtga gcaaaaacag gaaggcaaaa tgccgcaaaa 6180

aagggaataa gggcgacacg gaaatgttga atactcatac tcttcctttt tcaatattat 6240

tgaagcattt atcagggtta ttgtctcatg tccgcgcgtt tcctgcatct tttaatcaaa 6300

tcccaagatg tgtataaacc accaaactgc caaaaaatga aaactgtcga caagctctgt 6360

ccgtttgctg gcaactgcaa gggtctcaat cctatttgta attattgaat aataaaacaa 6420

ttataaatgt caaatttgtt ttttattaac gatacaaacc aaacgcaaca agaacatttg 6480

tagtattatc tataattgaa aacgcgtagt tataatcgct gaggtaatat ttaaaatcat 6540

tttcaaatga ttcacagtta atttgcgaca atataatttt attttcacat aaactagacg 6600

ccttgtcgtc ttcttcttcg tattccttct ctttttcatt tttctcttca taaaaattaa 6660

catagttatt atcgtatcca tatatgtatc tatcgtatag agtaaatttt ttgttgtcat 6720

aaatatatat gtctttttta atggggtgta tagtaccgct gcgcatagtt tttctgtaat 6780

ttacaacagt gctattttct ggtagttctt cggagtgtgt tgctttaatt attaaattta 6840

tataatcaat gaatttggga tcgtcggttt tgtacaatat gttgccggca tagtacgcag 6900

cttcttctag ttcaattaca ccatttttta gcagcaccgg attaacataa ctttccaaaa 6960

tgttgtacga accgttaaac aaaaacagtt cacctccctt ttctatacta ttgtctgcga 7020

gcagttgttt gttgttaaaa ataacagcca ttgtaatgag acgcacaaac taatatcaca 7080

aactggaaat gtctatcaat atatagttgc tctagttatt aatagtaatc aattacgggg 7140

tcattagttc atagcccata tatggagttc cgcgttacat aacttacggt aaatggcccg 7200

cctggctgac cgcccaacga cccccgccca ttgacgtcaa taatgacgta tgttcccata 7260

gtaacgccaa tagggacttt ccattgacgt caatgggtgg actatttacg gtaaactgcc 7320

cacttggcag tacatcaagt gtatcatatg ccaagtacgc cccctattga cgtcaatgac 7380

ggtaaatggc ccgcctggca ttatgcccag tacatgacct tatgggactt tcctacttgg 7440

cagtacatct acgtattagt catcgctatt accatgcatg gtcgaggtga gccccacgtt 7500

ctgcttcact ctccccatct cccccccctc cccaccccca attttgtatt tatttatttt 7560

ttaattattt tgtgcagcga tgggggcggg gggggggggg ggcgcgcgcc aggcggggcg 7620

gggcggggcg aggggcgggg cggggcgagg cggagaggtg cggcggcagc caatcagagc 7680

ggcgcgctcc gaaagtttcc ttttatggcg aggcggcggc ggcggcggcc ctataaaaag 7740

cgaagcgcgc ggcgggcggg agtcgctgcg acgctgcctt cgccccgtgc cccgctccgc 7800

cgccgcctcg cgccgcccgc cccggctctg actgaccgcg ttactcccac aggtgagcgg 7860

gcgggacggc ccttctcctc cgggctgtaa ttagcgcttg gtttaatgac ggcttgtttc 7920

ttttctgtgg ctgcgtgaaa gccttgaggg gctccgggag ggccctttgt gcggggggag 7980

cggctcgggg ctgtccgcgg ggggacggct gccttcgggg gggacggggc agggcggggt 8040

tcggcttctg gcgtgtgacc ggcggctcta gagcctctgc taaccatgtt catgccttct 8100

ttctttttcc tacagctcct gggcaacgtg ctggttattg tgctgtctca tcattttggc 8160

aaagaattgg atcggaccga aattaatacg actcactata 8200